# EUROPÄISCHE PATENTANMELDUNG

(11) **EP 4 353 078 A2**
(43) Veröffentlichungstag der Anmeldung: **17.04.2024**
(21) Anmeldenummer: 24159022.3
(22) Anmeldetag: 03.09.2014
(51) Int. Cl.: A01H 5/00

(54) **HELMINTHOSPORIUM TURCICUM-RESISTENTE PFLANZE**

(30) Priorität: 04.09.2013 DE 102013014637; 24.04.2014 DE 102014005823
(62) Teilanmeldung aus: 14761579.3
(71) Anmelder: KWS SAAT SE & Co. KGaA, 37574 Einbeck (DE); Universität Zürich, 8006 Zürich (CH)
(72) Erfinder: Ouzunova, Milena, 37075 Göttingen (DE); Scheuermann, Daniela, 37574 Einbeck (DE); Keller, Beat, 8006 Zürich (CH); Krattinger, Simon, 8906 Bonstetten (CH); Wicker, Thomas, 8536 Hüttwilen (CH); Herren, Gerhard, 8625 Gossau (CH); Bender, Severine, 35075 Gladenbach (DE); Kessel, Bettina, 37574 Einbeck (DE); Presterl, Thomas, 37574 Einbeck (DE); Knaak, Carsten, 37079 Göttingen (DE)
(74) Vertreter: Eisenführ Speiser

(57) **Zusammenfassung**

Durch die vorliegende Erfindung wird eine verbesserte *Helminthosporium turcicum*resistente Pflanze, insbesondere eine Maispflanze, welche ein Polynukleotid mit einem oder mehreren Resistenz-vermittelnden Genen beispielsweise auf einem trunkierten Chromosomfragment aus der Akzession Pepitilla umfasst, sowie eine Zelle, ein Gewebe, ein Teil, Korn und Samen davon, ein isoliertes Polynukleotid, das ein oder mehrere Resistenz-vermittelnden Genen gegen *Helminthosporium turcicum* umfasst, einen Vektor, eine transgene Pflanzenzelle und eine transgene Pflanze, enthaltend dieses Polynukleotid. Ferner sind von der Erfindung auch geeignete Marker und deren Verwendung zum Einbringen der Resistenz oder des Transgens in eine Pflanze sowie die Identifikation von verbesserten Maispflanzen, die ein trunkiertes Chromosomfragment aufweisen, miterfasst.

## Beschreibung

### Gebiet der Erfindung

Die vorliegende Erfindung betrifft das Gebiet der Modifikation von Pflanzen mittels molekularbiologischer Methoden und Markertechnologie und der Gentechnik. Sie betrifft eine neue *Helminthosporium turcicum*-resistente Pflanze, insbesondere eine Maispflanze, welche ein Polynukleotid mit einem oder mehreren Resistenz-vermittelnden Genen auf einem modifizierten Chromosomfragment aus der Akzession Pepitilla umfasst, sowie eine Zelle, ein Gewebe, ein Teil, Korn und Samen davon, ein isoliertes Polynukleotid, das ein oder mehrere Resistenz-vermittelnden Gene gegen *Helminthosporium turcicum* umfasst, einen Vektor, eine transgene Pflanzenzelle und eine transgene Pflanze, enthaltend dieses Polynukleotid. Ferner sind von der Erfindung auch geeignete molekulare Marker und deren Verwendung zum Einbringen des Resistenzlocus oder des Transgens in eine Pflanze sowie die Identifikation von verbesserten Maispflanzen, die ein modifiziertes Chromosomfragment aufweisen, miterfasst.

### Hintergrund der Erfindung

In Mais (*Zea mays* L.) findet man eine große Zahl von pilzlichen Pathogenen die Blatterkrankungen hervorrufen. Der Pilz, der unter tropischen aber auch den gemäßigten Klimabedingungen, wie sie in großen Teilen Europas und Nordamerikas, aber auch Afrika und Indien vorherrschen, den mit Abstand größten Schaden verursachen kann, ist unter *Helminthosporium turcicum* oder syn. auch *Exserohilum turcicum* (Pass.) Leonard et Suggs (Teleomorphe: *Setosphaeria turcica* (Luttrell) Leonard & Suggs) bekannt. *H. turcicum* ist der Verursacher der Blattfleckenkrankheit 'Northern Corn Leaf Blight' (NCLB), die in feuchten Jahren als Epidemie gegenüber anfälligen Maissorten hoch schädigend auftreten kann und dann beträchtlichen Ertragseinbußen von 30% und mehr über große Flächen zur Folge hat (Perkins & Pedersen, 1987; Raymundo & Hooker, 1981a; Ullstrup & Miles, 1957). Seit den 1970er Jahren wurde daher in genetischem Material nach natürlichen Resistenzen gesucht. Heute sind quantitative und qualitative Resistenzen bekannt. Während die oligo- oder polygenisch vererbte quantitative Resistenz unvollständig und rassenunspezifisch im Phänotyp erscheint und durch zusätzliche und partiell-dominante Gene beeinflusst wird, ist die qualitative Resistenz typischerweise rassenspezifisch und kann durch einzelne zumeist dominante Gene wie *Ht1, Ht2, Ht3, Htm1* oder *Htn1* vererbt werden (Lipps *et al.,* 1997; Welz & Geiger, 2000). Durch Rückkreuzungen gelang in zahlreichen häufig verwendeten Inzucht-Maislinien wie W22, A619, B37 oder B73 die Introgression der Ht-Gene, wo sie eine partielle Dominanz und eine Expression in Abhängigkeit vom jeweiligen genetischen Hintergrund zeigten (Welz, 1998).

Trotz dieser komplexen genetischen Architektur der NCLB-Resistenz im Mais war bislang vornehmlich die Verwendung des Gens *Ht1* in Mais zusammen mit einer partiellen quantitativen Resistenz ausreichend, um die Helminthosporiosis zu kontrollieren (Welz, 1998). Der Grund hierfür ist, dass die Rasse 0 von *H. turcicum* weltweit mit ca. 55% mit Abstand vorherrschend ist (Lipps *et al.,* 1997; Ferguson & Carson, 2007), während andere Rassen wie 2N und 23N dagegen nur sehr selten und häufig geographisch begrenzt auftreten (Moghaddam & Pataky, 1994; Jordan *et al.,* 1983; Lipps & Hite, 1982; Thakur *et al.,* 1989; Welz, 1998). Diese Rasse 0 ist gegenüber einer Maispflanze mit *Ht1* avirulent, sodass diese versehen mit einer geeigneten quantitativen Resistenz insgesamt eine ausreichende NCLB-Resistenz aufzeigt. Zahlreiche Studien berichten jedoch von einer zunehmenden Verbreitung der weniger häufigen Rassen (Jordan *et al.,* 1983; Welz, 1998; Pratt & Gordon, 2006). Gründe hierfür liegen in der Populationsdynamik eines Pathogens, welche durch neue Mutationen auf Avirulenzgenen und neue Kombinationen vorhandener Virulenzgene Veränderungen in der Pathogenvirulenz erlaubt. Dies kann letztendlich zum Auftauchen neuer, angepasster, teils aggressiverer pathogener Rassen führen. In Brasilien beispielsweise scheint die *H. turcicum*-Population schon deutlich diverser im Hinblick auf die Rassenzusammensetzung zu sein als zum Beispiel in Nordamerika. Gianasi *et al.* (1996) berichtet von *H. turcicum* Rassen, die bereits die Resistenz, welche durch das *Ht1-*Gen vermittelt wird, durchbrochen haben. Hinzu kommt dabei auch die Instabilität der Resistenzgene gegenüber bestimmten Umweltfaktoren wie Temperatur und Lichtintensität in einigen Klimazonen (Thakur *et al.,* 1989). Diese Entwicklung hat zur Folge, dass global gesehen die Verwendung der bisher weniger beachteten oder neuer *Ht*-Resistenzgene zur Herstellung kommerzieller Maispflanzen zunehmend an Bedeutung gewinnt, um das Ziel einer breiten und dauerhaften Resistenz gegenüber *H. turcicum* in Mais herbeizuführen. Erste Ansätze hierzu wurden bereits von Pataky *et al.* im Jahr 1998 vorgestellt. Durch Verwendung einer Kombination von *Ht1* und *Htn1* konnte die NCLB-Resistenz in *sh2-*Elitekörnermais verbessert werden.

Ein Ursprung der monogenischen *Htn1*-Resistenz ist die Mexikanische Landrasse 'Pepitilla' (Gevers, 1975). Die *Htn1*-Introgressionslinien zeigen eine Kartierung des Gens auf dem langen Arm des Chromosoms 8 ungefähr 10 cM distal von *Ht2* und 0,8 cM distal vom RFLP Marker umc117 (bin 8.06) (Simcox & Bennetzen, 1993). Anders als die übrigen Ht-Resistenzgene vermittelt *Htn1* eine Resistenz durch eine Verzögerung des Einsetzens der Sporulation und wirkt somit der Entwicklung von Läsionen entgegen. Im Ergebnis führt dies zu weniger und kleineren Läsionen sowie reduzierten Sporulationszonen (Raymundo *et al.,* 1981b, Simcox & Bennetzen, 1993). Chlorotisch-nekrotische Läsionen, wie sie bei *Ht1, Ht2* oder *Ht3*-vermittelter Resistenz auftreten, werden nicht ausgebildet (Gevers, 1975). Jedoch ist die Resistenzreaktion im heterozygoten Zustand des *Htn1-*Gens deutlich ineffektiver als im homozygoten Zustand (Raymundo *et al.,* 1981b).

Eine verbesserte züchterische Handhabbarkeit des *Htn1-*Gens setzt die Entwicklung von zusätzlichen spezifischen Markern voraus, um die Genotyp-Bestimmung weiter zu vereinfachen. Durch die MAS (marker assisted-selection) Technologie wird dann ein 'Stacking' oder 'Pyramiding' von mehreren Resistenzgenen effizient möglich (Min *et al.,* 2012). In vielen Studien zur Kartierung des Resistenzlocus und zur Identifikation der Resistenzquelle wurden die Introgressionslinien B37*Htn1* oder W22*Htn1* herangezogen (Raymundo *et al.,* 1981a, b; Simsox & Bennetzen, 1993, Bar-Zur *et al.,* 1998; Coates & White, 1998). Angaben zu Markern, die zur Selektion des Resistenzlocus für *Htn1* aus der Akzession Pepitilla verwendet werden könnten, sind dabei jedoch nur begrenzt vorhanden (Simsox & Bennetzen, 1993). Die bekannten Marker für *Htn1* funktional und flankierend zum Resistenzlocus aus der Akzession Pepitilla kartieren immer noch annähernd 22,2 cM auseinander, was im besten Fall das Selektieren eines großen Chromsosomfragments gestattet. Dabei besteht jedoch oft das Risiko, dass innerhalb dieses Fragments zwischen den Markern eine doppelte genetische Rekombination stattfindet, was eine falsch-positive Selektion für den *Htn1*-Resistenzlocus zur Folge haben könnte. Zudem steigt in einigen Fällen mit der Größe des introgressierten Chromosomenfragments auch die Wahrscheinlichkeit unerwünschte genetische Bereiche in eine Introgressionslinie zu übernehmen und über Generationen von Elitelinien mitzuführen. Solche genetischen Bereiche, insbesondere wenn sie eng gekoppelt sind mit dem *Htn1*-Locus, und zu deutlichnegativen Auswirkungen auf ein oder mehrere agronomische Merkmale führen, werden als Linkage Drag bezeichnet. Aus bekannten Studien, die Introgressionslinien mit Htn1 aus Pepitilla untersuchten und nutzten, sind jedoch solche negativen Effekte nicht bekannt. Auch die sehr umfangreichen Forschungsarbeiten von Welz (1998), die unter anderen auch an B37Htn1 durchgeführt wurden, postulieren, dass im Hinblick auf beispielsweise Ertrag und Reife durch die Introgression des Htn1-Locus keine signifikanten Nachteile auszumachen sind. So finden sich im Stand der Technik auch keine ernsthaften Bestrebungen das große Chromsosomfragment gezielt weiter zu verkürzen.

Dagegen offenbart WO 2011/163590 den Genotyp PH99N als alternative Quelle für eine NCLB-Resistenz auf Chromosom 8 bin 5, welche jedoch nicht der Akzession Pepitilla entspricht. Für erzeugte Rückkreuzungspopulation aus PH99N wurden im Wesentlichen nur Resistenzen gegenüber den *H. turcicum-*Rassen 0 und 1 identifiziert. Auch der Resistenz-Phänotyp wurde nicht eindeutig bestimmt. Dennoch schlussfolgern die Autoren, dass die Resistenz auf das *Htn1-*Gen zurückzuführen sei. Es gelang zwar für PH99N den Resistenzlocus auf ein Chromosomfragment von nur noch -224 kb Länge zu beschränken, eine resistente Maispflanze mit dem 224 kb-Fragment und somit dem vermeintlichen Htn1, wurde jedoch nicht offenbart. Zudem ist der Genotyp PH99N der Öffentlichkeit durch Hinterlegung auch nicht zugänglich gemacht.

Ein alternativer Ansatz zur Nutzbarmachung des *Htn1-*Gens ist die Identifizierung und Klonierung des Resistenzgens und deren Verwendung in einem transgenen Ansatz.

Mit der Absicht der Identifikation der Resistenzgene für NCLB veröffentlichten Chung *et al.* 2010 eine Studie zur Feinkartierung des Resistenzlocus bin 8.06. Das untersuchte Chromosomfragment stammt jedoch nicht aus Pepitilla, sondern aus der Maishybride DK888, welche eine multiple Krankheitsresistenz aufweist. Untersuchungen zur *Helmithosporium*-Rassenspezifität legten zunächst nahe, dass der Resistenzlocus auf DK888, bezeichnet als *qNLB8.06_{DK888},* eng verknüpft oder funktionell verbunden ist mit dem *Ht2-* und dem *Htn1-*Gen*,* da *Helminthosporium-*Stämme 23 und 23N virulent waren (Chung *et al.,* 2008). Ein endgültiger Nachweis für das Vorhandensein von *Htn1* wurde in Mangel eines reinen N-Isolates von *H. turcicum* jedoch nicht durchgeführt. Zudem entsprach der Resistenz-Phänotyp mit *qNLB8.06_{DK888}* auch nicht dem zu erwartenden Phänotyp im Hinblick auf Entstehung von chlorotischen Läsionen und der Verzögerung der Läsionsbildung. Weiterführende Komplementationsstudien in Chung *et al.* (2010) ergaben letztendlich Hinweise, dass *qNLB8.06_{DK888}* entweder identisch, allelisch, eng verknüpft oder funktionell verbunden ist mit *Ht2,* aber nicht mit *Htn1.* Der Resistenzlocus *qNLB8.06_{DK888}* konnte einem Chromosomfragment von 0,46 Mb zugeordneten werden. Genomische Annotationen von diesem Chromosomfragment wiesen auf 12 putative offene Leserahmen hin, von denen drei jeweils ein Tandem-Proteinkinase-ähnliches Gen (GRMZM2G135202; GRMZM2G164612) oder ein Proteinphosphatase-ähnliches Gen (GRMZM2G119720) darstellen könnten und jedes gleichermaßen als vielversprechendes Kandidatengen für das Resistenzgen *Ht2* favorisiert werden (Chung *et al.,* 2010). Ein funktioneller Nachweis wird nicht beschrieben.

Ferner annotiert auch WO 2011/163590 A1 das vermeintliche *Htn1-*Gen in der Resistenzquelle PH99N als ein Tandem-Proteinkinase-ähnliches Gen (GRMZM2G451147) und offenbart deren genetische Sequenz, weist aber deren Funktionalität zum Beispiel in einer transgenen Maispflanze auch nicht nach.

### Zusammenfassung der Erfindung

Die vorliegende Erfindung wurde vor dem Hintergrund des vorstehend beschriebenen Stands der Technik gemacht, wobei es Aufgabe der vorliegenden Erfindung ist, eine Maispflanze bereitzustellen, die eine Resistenz gegenüber dem Pathogen *Helminthosporium turcicum* aus dem Donor Pepitilla aufweist und hinsichtlich agronomischer Merkmale den bekannten Maispflanzen mit Resistenz aus dem Donor Pepitilla überlegen ist.

Die Aufgabe ist zum einen durch eine Maispflanze gelöst, in deren Genom ein Chromosomfragment aus dem Donor Pepitilla integriert ist, wobei das Chromosomfragment ein Intervall des Donors (nachfolgend erstes Intervall oder Intervall 1 genannt) umfasst, welches Donor-Allele gemäß dem Haplotyp nach Tabelle 2 aufzeigt und ein Polynukleotid aufweist, das in der Maispflanze Resistenz gegenüber *Helminthosporium turcicum* vermittelt, und wobei das Chromosomfragment ein weiteres Intervall des Donors (nachfolgend zweites Intervall oder Intervall 2 genannt) zwischen einem Marker in einer ersten Markerregion (M1), welche durch die Marker SYN14136 und PZE-108076510 flankiert ist, und einem Marker in einer zweiten Markerregion (M2), welche durch die Marker SYN24931 und PZE-108077560 flankiert ist, nicht enthält. Diese und weiter unten beschriebene alternative Lösungen der Aufgabe können auf einem Züchtungsprogramm zur Integration des *Htn1*-Locus aus Pepitilla in Maislinien beruhen. Wahlweise sind jedoch auch gentechnische Ansätze anwendbar, über welche Pflanzen der vorliegenden Erfindung hergestellt werden können. Beispielhaft werden gentechnische Ansätze weiter unten näher ausgeführt. Zur Erzeugung der Pflanzen der vorliegenden Erfindung kann auf diverse Genotypen aus dem Stand der Technik zurückgreifen kann. Insbesondere B37HTN1, das den Resistenzlocus der Landrasse 'Pepitilla' aufweist, wurde als Ausgangslinie verwendet. Neben Pepitilla selbst und B37HTN1 (auch aus dem Stand der Technik bekannt als B37HtN) kann man zur Integration des *HTN1*-Locus zur Herstellung einer erfindungsgemäßen Maispflanze nahezu jeden bekannten Maisgenotypen heranziehen, in dessen Genom, insbesondere auf Chromosom 8 bin 5 oder 6, eine Introgression des *HTN1*-Resistenzlocus aus Pepitilla inseriert wurde. Aus dem Stand der Technik sind hier zahlreiche Genotyp-Beispiele bekannt, zum Beispiel: W22Htn (z.B. Bar-Zur et al.,1998); H6314*Htn* (z.B. Bar-Zur et al.,1998), B73*HtN* (z.B. Shimoni et al., Journal of Phytopathology 131:4 (1991), 315-321), B68*HtN* und A632*HtN* (z.B. Carson, Plant Disease 79 (1995), 717-720) und A619*HtN* (z.B. Stanković et al, Genetika 39:2 (2007), 227 -240). In einer erfindungsgemäßen Maispflanze stammt das Chromosomfragment aus dem Donor Pepitilla, in einer bevorzugten Ausgestaltung der erfindungsgemäßen Maispflanze stammt das Chromosomfragment aus dem Donor B37HTN1 oder einem anderen oben genannten Maisgenotyp. B37HTN1 kann beispielsweise über das Maize Genetics COOP Stock Center mit der Stock ID 65749 bestellt werden.

Das in das Genom der erfindungsgemäßen Maispflanze integrierte Chromosomfragment stammt aus dem Donor Pepitilla, welcher bekanntermaßen den Resistenzlocus HTN1 aufweist. Die Introgression dieses Resistenzlocus ist auf dem langen Arm des Chromosoms 8, bin 8.05 - 8.06 lokalisiert. Das integrierte Chromosomfragment umfasst das erste Intervall des Donors, welches ein Polynukleotid aufweist, das in der erfindungsgemäßen Maispflanze Resistenz gegenüber *Helminthosporium turcicum* vermittelt. Dabei umfasst das Polynukleotid ein oder mehrere Resistenz-vermittelnde Gene des HTN1-Locus aus Pepitilla (Tabelle 1) oder Genallele davon. Gen oder Genallel können unter Befallsbedingungen mit *H. turcicum* einen Resistenzphänotyp mit den HTN1-typischen Merkmalen bewirken. Vorzugsweise umfasst das Polynukleotid ein oder mehrere Resistenz-vermittelnde Gene des HTN1-Locus bevorzugt aus Pepitilla ausgewählt aus RLK1 und EXT1 (siehe Tabelle 1) oder Genallele davon, welche unter Befallsbedingungen mit *H. turcicum* einen Resistenzphänotyp mit den HTN1-typischen Merkmalen bewirken. Besonders bevorzugt umfasst das Polynukleotid eine Nukleotidsequenz, welche ein Polypeptid gemäß SEQ ID NO: 2 oder SEQ ID NO: 6 oder ein Homolog eines Polypeptids gemäß SEQ ID NO: 2 oder SEQ ID NO: 6 kodiert, welche unter Befallsbedingungen mit *H. turcicum* einen Resistenzphänotyp mit den HTN1-typischen Merkmalen bewirken. Zu diesen HTN1-typischen Merkmalen gehören beispielsweise verzögertes Einsetzen der Sporulation, verminderte Entwicklung von Läsionen, Entwicklung von kleineren Läsionen, reduzierte Sporulationszonen und/oder keine oder nur vereinzelte chlorotisch-nekrotische Läsionen. Strukturell ist das Polynukleotid dadurch gekennzeichnet, dass es ein Nukleinsäuremolekül umfasst, (a) das eine Nukleotidsequenz gemäß SEQ ID NO: 1, 3, 5, 7, 9, 11, 13 oder 15 aufweist, (b) das eine Nukleotidsequenz mit einer Identität von mindestens 80%, 85%, 90%, 91%, 92%, 93%, 94%, 95%, 96%, 97%, 98% oder 99% zu einer der Nukleotidsequenzen gemäß SEQ ID NO: 1, 3, 5, 7, 9, 11, 13 oder 15 vorzugsweise über die gesamte Sequenzlänge, aufweist, (c) das mit dem komplementären Strang eines Nukleinsäuremoleküls nach (a) oder (b) unter stringenten Bedingungen hybridisiert, (d) das ein Polypeptid mit einer Aminosäuresequenz gemäß SEQ ID NO: 2, 4, 6, 8, 10, 12, 14 oder 16 kodiert, (e) das ein Polypeptid mit einer Aminosäuresequenz, die zu mindestens 80%, 85%, 90%, 91%, 92%, 93%, 94%, 95%, 96%, 97%, 98% oder 99% identisch ist mit einer der Aminosäuresequenzen nach (d), kodiert oder (f) das eine Teilsequenz einer Nukleinsäure nach (a) bis (e) aufweist. In einer bevorzugten Ausgestaltung ist das Polynukleotid dadurch gekennzeichnet, dass es ein Nukleinsäuremolekül umfasst, (aa) das eine Nukleotidsequenz gemäß SEQ ID NO: 1 oder 5 aufweist, (bb) das eine Nukleotidsequenz mit einer Identität von mindestens 80%, 85%, 90%, 91%, 92%, 93%, 94%, 95%, 96%, 97%, 98% oder 99% zu einer der Nukleotidsequenzen gemäß SEQ ID NO: 1 oder 5 vorzugsweise über die gesamte Sequenzlänge, aufweist, (cc) das mit dem komplementären Strang eines Nukleinsäuremoleküls nach (aa) oder (bb) unter stringenten Bedingungen hybridisiert, (dd) das ein Polypeptid mit einer Aminosäuresequenz gemäß SEQ ID NO: 2 oder 6 kodiert, (ee) das ein Polypeptid mit einer Aminosäuresequenz, die zu mindestens 80%, 85%, 90%, 91%, 92%, 93%, 94%, 95%, 96%, 97%, 98% oder 99% identisch ist mit einer der Aminosäuresequenzen nach (dd), kodiert oder (ff) das eine Teilsequenz einer Nukleinsäure nach (aa) bis (ee) aufweist. Eine Teilsequenz eines Nukleinsäuremoleküls im Sinne der vorliegenden Erfindung kann mindestens 20, 30, 40, 50, 60, 70, 80, 90 oder mindestens 100 aufeinander folgende Nukleotide, weiter mindestens 150, 200, 250, 300, 350, 400, 450, 500, 600, 700, 800, 900 oder 1000 aufeinander folgende Nukleotide umfassen. Das Polynukleotid kann im heterozygoten oder homozygoten Zustand im Genom der erfindungsgemäßen Maispflanze vorliegen; vorzugsweise liegt das Polynukleotid im homozygoten Zustand vor.

**Tabelle 1: Potentielle Resistenz-vermittelnde Gene des HTN1-Locus aus Pepitilla; Genname (Spalte 1); Verweis auf korrespondierende SEQ ID NOs der genomischen Exon-Sequenz (Spalte 2); Verweis auf korrespondierende SEQ ID NOs der vorhergesagten Aminosäure-/Proteinsequenz (Spalte 3); annotiertes homologes Gen aus dem B73-Referenzgenom (Spalte 4).**

| Genname | cDNA SEQ ID NO: | Proteinsequenz SEQ ID NO: | Homologes B73 Gen |
|---|---|---|---|
| RLK1 | 1 | 2 | GRMZM2G451147 |
| RLK4 | 3 | 4 | GRMZM2G144028 |
| EXT1 | 5 | 6 | GRMZM2G445338 |
| DUF1 | 7 | 8 | AC209075.3_FG007 |
| ZNF1 | 9 | 10 | GRMZM2G175661 |
| CYT1 | 11 | 12 | GRMZM2G092018 |
| RET1 | 13 | 14 | GRMZM2G091973 |
| HYD | 15 | 16 | GRMZM2G144021 |

Weiterhin ist das erste Intervall im Chromosomfragment, welches Donor-Allele gemäß dem Haplotyp nach Tabelle 2 aufzeigt, durch die Abfolge von Donor-Allelen gemäß dem Haplotyp nach Tabelle 2 charakterisiert, aber nicht auf diese Abfolge von Donor-Allelen gemäß dem Haplotyp nach Tabelle 2 beschränkt. Dies bedeutet, dass das erste Intervall mindestens das Donor-Allel, welches das Resistenz-vermittelnde Gen aus Tabelle 1 beschreibt, optional mit Donor-Allel des Markers MA0008, zeigt. Weiterhin zeigt das erste Intervall bevorzugt mindestens die Donor-Allele gemäß dem Haplotyp nach Tabelle 2 von MA0021 bis MA0022 (d.h. MA0021, MA0007, MA0008, MA0009, MA0010, MA0011, MA0012, MA0022) oder von MA0005 bis MA0022 (d.h. MA0005, MA0021, MA0007, MA0008, MA0009, MA0010, MA0011, MA0012 und MA0022) oder von MA0005 bis MA0013 (d.h. MA0005, MA0021, MA0007, MA0008, MA0009, MA0010, MA0011, MA0012, MA0022 und MA0013) oder von MA0005 bis MA0014 (d.h. MA0005, MA0021, MA0007, MA0008, MA0009, MA0010, MA0011, MA0012, MA0022, MA0013 und MA0014) oder von MA0005 bis MA0015 (d.h. MA0005, MA0021, MA0007, MA0008, MA0009, MA0010, MA0011, MA0012, MA0022, MA0013, MA0014 und MA0015) oder von MA0005 bis MA0016 (d.h. MA0005, MA0021, MA0007, MA0008, MA0009, MA0010, MA0011, MA0012, MA0022, MA0013, MA0014, MA0015 und MA0016), besonders bevorzugt von MA0005 bis MA0017 (d.h. MA0005, MA0021, MA0007, MA0008, MA0009, MA0010, MA0011, MA0012, MA0022, MA0013, MA0014, MA0015, MA0016 und MA0017), MA0005 bis MA0018 (d.h. MA0005, MA0021, MA0007, MA0008, MA0009, MA0010, MA0011, MA0012, MA0022, MA0013, MA0014, MA0015, MA0016, MA0017 und MA0018), MA0005 bis PZE-108095998 (d.h. MA0005, MA0021, MA0007, MA0008, MA0009, MA0010, MA0011, MA0012, MA0022, MA0013, MA0014, MA0015, MA0016, MA0017, MA0018 und PZE-108095998), MA0005 bis PZE-108096011 (d.h. MA0005, MA0021, MA0007, MA0008, MA0009, MA0010, MA0011, MA0012, MA0022, MA0013, MA0014, MA0015, MA0016, MA0017, MA0018, PZE-108095998 und PZE-108096011) oder MA0005 bis MA0019 (d.h. MA0005, MA0021, MA0007, MA0008, MA0009, MA0010, MA0011, MA0012, MA0022, MA0013, MA0014, MA0015, MA0016, MA0017, MA0018, PZE-108095998, PZE-108096011 und MA0019), ganz besonders bevorzugt von MA0005 bis PZE-108096610 (d.h. MA0005, MA0021, MA0007, MA0008, MA0009, MA0010, MA0011, MA0012, MA0022, MA0013, MA0014, MA0015, MA0016, MA0017, MA0018, PZE-108095998, PZE-108096011, MA0019 und PZE-108096610), MA0005 bis MA0020 (d.h. MA0005, MA0021, MA0007, MA0008, MA0009, MA0010, MA0011, MA0012, MA0022, MA0013, MA0014, MA0015, MA0016, MA0017, MA0018, PZE-108095998, PZE-108096011, MA0019, PZE-108096610 und MA0020), MA0005 bis PZE-108096791 (d.h. MA0005, MA0021, MA0007, MA0008, MA0009, MA0010, MA0011, MA0012, MA0022, MA0013, MA0014, MA0015, MA0016, MA0017, MA0018, PZE-108095998, PZE-108096011, MA0019, PZE-108096610, MA0020 und PZE-108096791) oder MA0005 bis MA0006 (d.h. MA0005, MA0021, MA0007, MA0008, MA0009, MA0010, MA0011, MA0012, MA0022, MA0013, MA0014, MA0015, MA0016, MA0017, MA0018, PZE-108095998, PZE-108096011, MA0019, PZE-108096610, MA0020, PZE-108096791 und MA0006) auf. Dieser resistente Haplotyp erlaubt eine eindeutige Spezifizierung und Identifikation der Resistenzquelle Pepitilla. Insbesondere ist das erste Intervall zwischen den Markern MA0004 und PZE-108097482, zwischen den Markern MA0004 und MA0022, zwischen den Markern MA0005 und PZE-108097482 oder zwischen den Markern MA0005 und MA0022 lokalisiert. Vorzugsweise beschreibt das erste Intervall einen Abschnitt des Chromosomfragments, welcher die HTN1-typische Resistenz vermitteln kann. Als solcher ist er Träger des oben genannten Polynukleotids.

**Tabelle 2: Resistenter Haplotyp aus B37HTN1;**

| Position in bp auf B73 AGPv02 | Allele Donor B37HTN1 | Marker Bezeichnung |
|---|---|---|
| 151831049 | C | MA0005 |
| 151907173 | G | MA0021 |
| 152045106 | T | MA0007 |
| 152045141 | T | MA0008 |
| 152045402 | T | MA0009 |
| 152045516 | C | MA0010 |
| 152045912 | T | MA0011 |
| 152046502 | T | MA0012 |
| 152046529 | A | MA0022 |
| 152133057 | G | MA0013 |
| 152133380 | A | MA0014 |
| 152144310 | A | MA0015 |
| 152250992 | A | MA0016 |
| 152301656 | A | MA0017 |
| 152304127 | A | MA0018 |
| 152433358 | A | PZE-108095998 |
| 152435855 | A | PZE-108096011 |
| 152630794 | C | MA0019 |
| 152703579 | G | PZE-108096610 |
| 152753635 | A | MA0020 |
| 152887338 | G | PZE-108096791 |
| 152888374 | A | MA0006 |

Weiterhin ist jede erfindungsgemäße Maispflanze eine Ht-resistente Maispflanze. Die durch die Integration des Chromosomfragments vermittelte Ht-Resistenz kann mittels Bestimmung von Boniturnoten in Phänotypisierungsexperimenten nach Schema gemäß Tabelle 3 und Beispiel 1. A) quantifiziert werden, wobei das Resistenzlevel von 1 nach 9 hin abnimmt. Erfindungsgemäße Ht-resistente Maispflanzen zeigen eine erhöhte Resistenz gegenüber *H. turcicum* von mindestens 1 Boniturnote, bevorzugt von mindestens 2 Boniturnoten oder mindestens 3 Boniturnoten und besonders bevorzugt von mindestens 4 Boniturnoten. Bevorzugt zeigt eine erfindungsgemäße Maispflanze Resistenz gegenüber mindestens einer Rasse von *Helmithosporium turcicum,* welche nicht der bekannten Rassenspezifität entspricht wie sie aus dem Stand der Technik bekannt ist. In einer besonders bevorzugten Ausführungsform kann ist eine erfindungsgemäße Maispflanze resistent gegenüber sämtlichen bekannten Rassen von *Helmithosporium turcicum,* d.h. die vermittelte Resistenz ist rassenunspezifisch und kann zur Ausbildung einer breiten Resistenz gegenüber *Helmithosporium turcicum* besonders vorteilhaft geeignet sein.

**Tabelle 3: Boniturnoten-Schema für Phänotypisierungsexperimente in Feldversuchen an verschiedenen Standorten mit natürlicher und künstlicher H. turcicum-Beimpfung (nach dem Deutschem Maiskomitee (DMK); AG Sortenwesen 27.02.02; (DMK J. Rath; RP Freiburg H.J. Imgraben)**

| Boniturnote | Phänotyp |
|---|---|
| 1 | Pflanzen zeigen keine Krankheitssymptome 0% |
| 2 | Befallsbeginn, erste kleine Flecken (kleiner als 2cm) sichtbar. Weniger als 5% der Blattfläche ist betroffen. |
| 3 | Einige Flecken haben sich auf einer Blattetage entwickelt. Zwischen 5-10% der Blattfläche ist betroffen. |
| 4 | 10-20% der Blattfläche ist betroffen. Deutliche Flecken auf mehreren Blattetagen. |
| 5 | 20-40% der Blattfläche ist betroffen. Flecken fangen an zusammenzufließen. |
| 6 | 40-60% der Blattfläche ist betroffen. Systematischer Befall auf den Blättern sichtbar. |
| 7 | 60-80% der Blattfläche ist betroffen. Etwa die Hälfte der Blätter ist wegen Pilzbefalls zerstört bzw. vertrocknet |
| 8 | 80-90% der Blattfläche ist betroffen. Mehr als die Hälfte der Blätter ist wegen Pilzbefalls zerstört bzw. vertrocknet |
| 9 | 90-100% der Blattfläche ist betroffen. Die Pflanzen sind nahezu vollständig vertrocknet. |

Die Beschreibung offenbart die genetische bzw. molekulare Struktur des HTN1-Locus durch Angabe eines Haplotyps, durch Kartierung prominenter Marker sowie durch Identifizierung von Kandidaten-Genen für die Resistenzvermittlung gegenüber dem Pathogen *Helminthosporium turcicum.*

Überraschenderweise erwies sich die erfindungsgemäße Maispflanze in Geno- und Phänotypisierungsexperimenten in Feld und Gewächshaus als agronomisch überlegen. Denn während andere konvertierte Linien aus einem Züchtungsprogramm zur Integration des HTN1-Locus aus Pepitilla sowie aus dem Stand der Technik bekannte konvertierte Linien wie B37HTN1 neben der vermittelten Ht-Resistenz unter Nichtbefallsbedingungen mit *H. turcicum* und unter vergleichbaren Umweltbedingungen (Temperatur, Nährstoffversorgung, Standort etc.) eine deutliche Verzögerung im Zeitpunkt der männlichen und/oder weiblichen Blüte im Vergleich zu der entsprechenden Linie ohne Introgression (z.B. isogene Linie oder Ausgangslinie) zeigen, entspricht bei der erfindungsgemäßen Maispflanze der Blühzeitpunkt demjenigen einer isogenen Vergleichsmaispflanze, in deren Genom ein Chromosomfragment aus dem Donor Pepitilla nicht integriert ist. Blühzeitpunkte entsprechen einander, wenn sie um weniger als 2 Tage voneinander abweichen. Die Größenordnung der beobachteten Verzögerung ist dabei stark abhängig von der Maissorte bzw. dem Maisgenotypen, den vorherrschenden Umweltfaktoren wie beispielsweise Bodenbeschaffenheit, Feuchtigkeit, Niederschlag, Temperatur etc. und/oder von biotischem Stress wie beispielsweise Pathogenbefall mit nicht *H. turcicum.* Die Verzögerung betrug mindestens 2 Tage, mindestens 3 Tage, mindestens 5 Tage oder mindestens 7 Tage. Dieser festgestellte Unterschied im Blühzeitpunkt ist auf Linkage Drag als Teil der Introgression zurückzuführen, was besonders überraschend ist, da derartige Beobachtungen aus dem Stand der Technik nicht bekannt sind. Der Blühzeitpunkt ist ein wichtiges agronomisches Merkmal. Er kann unmittelbar und wesentlich das Ertragspotential einer Maispflanze beeinflussen. Ein verspäteter Blühzeitpunkt führt dabei in der Regel zu einem verminderten Ertrag.

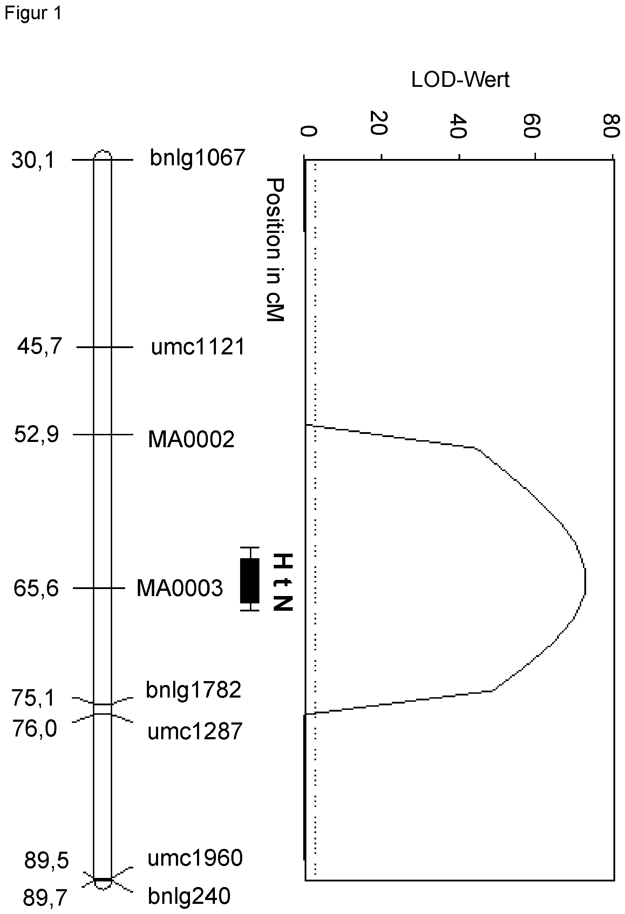

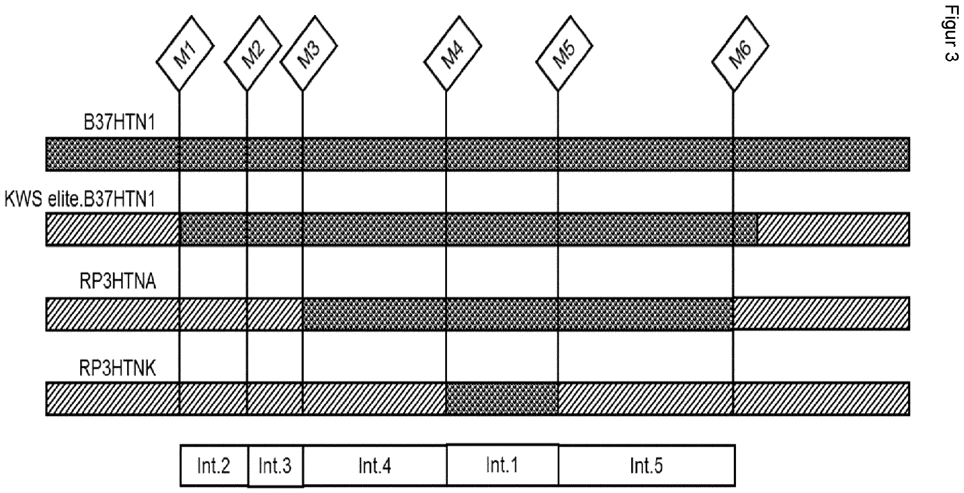

Zur Klärung der genetischen Ursache dieses Nachteils und zur Identifikation des Linkage Drags wurden beispielsweise ausgedehnte Rückkreuzungsprogramme, begleitet von Geno- und Phänotypisierungen, durchgeführt. Unterstützt wurden die Arbeiten von einer intensiven Entwicklung zielgenauer und spezifischer molekularer Marker auf dem HTN1-tragenden Chromosomfragment. Die Technologie zur Marker-gestützten Selektion (MAS) und die Durchführung von zielgerichteten Rückkreuzungsprogrammen (z.B. zum 'map based cloning') kann dem Stand der Technik entnommen werden (Gupta & Varshney, 2013). Der QTL mit der HTN1-Resistenz aus den Donor B37HTN1 bzw. Pepitilla wurde mit Hilfe der SSR Marker bnlg1067, umc1121, MA0002, MA0003, bnlg1782, umc1287, umc1960 und bnlg240 in den Filialgenerationen auf Chromosom 8 (bin 8.06) zwischen den Markern MA0002 (Tabelle 4) und umc1287 (Tabelle 5) in einem Bereich von 23,1 cM lokalisiert (siehe Figur 1). In Maispflanzen mit dem verzögerten Blühzeitpunkt gelang es die Lage des genomischen Donor-Sequenzabschnitts, der für den identifizierten Linkage Drag des Blühzeitpunktes verantwortlich ist, eindeutig auf einem weiteren, zweiten Intervall des Donors auf dem Chromosomfragment zu bestimmen (Beispiel 3B; Figur 3). In einer erfindungsgemäßen Maispflanze ist ein Chromosomfragment integriert, welches das zweite Intervall des Donors nicht enthält. Hier stammt das zweite Intervall beispielsweise von einem rekurrenten Elter, der nicht Träger des Linkage Drags des Blühzeitpunkts ist, oder von einem exogen eingebrachten homologen DNA-Fragment, welches nicht Täger des Linkage Drags ist, auf einem geeigneten Spendervektor für gezielte homologe Rekombination. Das zweite Intervall ist proximal gelegen und eng gekoppelt zu dem Resistenzlocus HTN1 bzw. zu dem ersten Intervall. Das zweite Intervall ist ein Intervall zwischen einem Marker in einer ersten Markerregion (M1), welche durch die Marker SYN14136 und PZE-108076510 flankiert ist, und einem Marker in einer zweiten Markerregion (M2), welche durch die Marker SYN24931 und PZE-108077560 flankiert ist. Die flankierenden Marker können der Tabelle 4 entnommen werden. Die Marker SYN14136, PZE-108076510, SYN24931 und PZE-108077560 sind SNP-Marker zur Verwendung im KBioscience-KASP-System (www.lgcgenomics.com/genotyping/kaspgenotyping-reagents/kasp-overview/). Sie definieren eindeutig die Markerregionen M1 und M2, zu beiden Seiten des Sequenzabschnitts, der im Donor B37HTN1 bzw. Pepitilla den Linkage Drag des Blühzeitpunktes trägt. Als polymorphe Marker sind sie überdies auch geeignet zwischen Pepitilla-Donor-Allel und beispielsweise dem Allel des rekurrenten Elter zu unterscheiden. Alle Angaben zur Nutzung dieser Marker als KASP-Marker können der Tabelle 4 entnommen werden. Geeignete exemplarische Primer-Hybridisierungsparameter für die PCR werden in Beispiel 2 wiedergegeben. Ein Fachmann ist darüber hinaus auch in der Lage, andere geeignete Hybridisierungsparameter zu bestimmen. Ferner ist es Routinetätigkeit eines Fachmanns in Kenntnis der beschriebenen Markerregionen neben den genannten Markern andere Marker, insbesondere polymorphe Marker, in M1 und/oder in M2 zu entwickeln. Unter Verwendung der hier aufgeführten Marker SYN14136, PZE-108076510, SYN24931 und PZE-108077560 oder selbst entwickelter Marker in M1 und/oder M2 fällt es dem Fachmann leicht festzustellen, ob in einer Maispflanze, in deren Genom ein Chromosomfragment mit HTN1-Resistenzlocus aus dem Donor Pepitilla integriert ist, das vorstehend beschriebene zweite Intervall des Donors enthalten ist oder nicht enthalten ist. Auch ist einem Fachmann bewusst, das beispielsweise im Laufe eines Züchtungsprozesses oder eines gentechnisch Ansatzes zur gezielten Rekombination ein chromosomales Intervall von dem Donor, das beispielsweise genomische Sequenzen umfasst, die Linkage Drag darstellen, durch genetische/homologe Rekombination von dem integrierten Chromosomfragment entfernt werden können. Dabei kann das Intervall des Pepitilla-Donors durch das korrespondierende Intervall des rekurrenten Eltergenoms oder von einem exogen eingebrachten homologen DNA-Fragment ersetzt werden. Marker im Allgemeinen und die hier offenbarten Marker im Besonderen können dabei insbesondere bei der Selektion unterstützend eingesetzt werden. Beispielhaft sei im Folgenden eine mögliche Verwendung von Markern zur Detektion eines Allels wiedergegeben: Das Detektieren eines Allels kann beispielsweise (a) das Isolieren von mindestens einem Nukleinsäuremolekül aus dem Genom einer Pflanze oder Pflanzenzelle/Maispflanze oder Maispflanzenzelle und (b) das Untersuchen des isolierten Nukleinsäuremolekül mit mindestens einem Marker umfassen, sowie optional (c) das Sequenzieren des Allels in einem und/oder mehreren Genotypen, (d) die Detektion von einem und/oder mehreren Polymorphismen und/oder (e) die Restriktion mit einer Restriktions-Endonuklease, die unterschiedlich große Fragmente an einem Markerallel erzeugen kann.

Eine bevorzugte Ausgestaltung der erfindungsgemäßen Maispflanze ist eine oben beschriebene Maispflanze, wobei das Chromosomfragment das zweite Intervall des Donors, welches flankiert ist a) von den Markern SYN14136 und PZE-108077560, b) von den Markern PZE-108076510 und PZE-108077560, c) von den Markern SYN14136 und SYN24931 oder d) von den Markern PZE-108076510 und SYN24931, nicht enthält.

In einer bevorzugten Ausgestaltung zeigt die erfindungsgemäße Maispflanze einen abweichenden Zeitpunkt der männlichen und/oder weiblichen Blüte im Vergleich mit der Pepitilla-konvertierten Linie oder -konvertierte Pflanze wie B37HTN1, welche das Intervall 2 zwischen einem Marker in einer ersten Markerregion (M1), die durch die Marker SYN14136 und PZE-108076510 flankiert ist, und einem Marker in einer zweiten Markerregion (M2), die durch die Marker SYN24931 und PZE-108077560 flankiert ist, enthält, wobei abweichender Zeitpunkt bedeutet, dass die konvertierten Linie oder konvertierte Pflanze eine Verzögerung von mindestens 2 Tagen, von mindestens 3 Tagen, von mindestens 5 Tagen oder von mindestens 7 Tagen aufweist.

Eine weitere bevorzugte Ausgestaltung der erfindungsgemäßen Maispflanze ist eine oben beschriebene Maispflanze, wobei das Chromosomfragment weiterhin ein Intervall des Donors (nachfolgend drittes Intervall oder Intervall 3 genannt) zwischen einem Marker in der zweiten Markerregion M2 und einem Marker in einer dritten Markerregion M3, welche durch die Marker PZE-108093423 (Tabelle 4) und PZE-108093748 (Tabelle 4) flankiert ist, nicht enthält. Die Marker PZE-108093423 und PZE-108093748 sind SNP-Marker zur Verwendung im KBioscience-KASP-System (www.lgcgenomics.com/genotyping/kaspgenotyping-reagents/kasp-overview/). Sie definieren eindeutig die Markerregion M3. Als polymorphe Marker sind sie überdies auch geeignet zwischen Donor-Allel und beispielsweise dem Allel des rekurrenten Elter zu unterscheiden. Alle Angaben zur Nutzung dieser Marker als KASP-Marker können der Tabelle 4 entnommen werden. Geeignete exemplarische Primer-Hybridisierungsparameter für die PCR werden in Beispiel 2 wiedergegeben. Ein Fachmann ist darüber hinaus auch in der Lage, andere geeignete Hybridisierungsparameter zu bestimmen. Ferner ist es Routinetätigkeit eines Fachmanns in Kenntnis der beschriebenen Markerregion neben den genannten Markern andere Marker, insbesondere polymorphe Marker, in M3 zu entwickeln. Unter Verwendung der oben genannten Marker der M2 und der hier aufgeführten Marker PZE-108093423 und PZE-108093748 oder selbst entwickelter Marker in M3 fällt es dem Fachmann leicht festzustellen, ob in einer Maispflanze, in deren Genom ein Chromosomfragment mit HTN1-ResistenzLocus aus dem Donor Pepitilla integriert ist, das vorstehend beschriebene dritte Intervall des Donors enthalten ist oder nicht enthalten ist.

Eine weitere bevorzugte Ausgestaltung der erfindungsgemäßen Maispflanze ist eine oben beschriebene Maispflanze, wobei das Chromosomfragment einen genetischen Abschnitt, der das zweite Intervall und das dritte Intervall des Donors umfasst und flankiert ist a) von den Markern SYN14136 und PZE-108093423, b) von den Markern PZE-108076510 und PZE-108093423, c) von den Markern SYN14136 und PZE-108093748 oder d) von den Markern PZE-108076510 und PZE-108093748, nicht enthält.

In einem weiteren Aspekt konnten weitere genetische Abschnitte auf dem Chromosomfragment bestimmt werden, welche unter Nichtbefallsbedingungen mit *H*. *turcicum* einen signifikanten negativen Einfluss auf das Ertragspotential einer Maispflanze, in deren Genom ein Chromosomfragment mit HTN1-Resistenzlocus aus dem Donor Pepitilla integriert ist, verursachen können. So zeigen unabhängig von der oben beschriebenen Verzögerung des Blühzeitpunktes konvertierte Linien ebenso wie aus dem Stand der Technik bekannte konvertierte Linien wie B37HTN1 neben der vermittelten Ht-Resistenz einen deutlich reduzierten Ertrag, insbesondere einen deutlich reduzierten Silageertrag im Vergleich zu der entsprechenden Linie ohne Introgression (z.B. isogene Linie oder Ausgangslinie). Dies gilt selbst für Linien, in deren Genom ein genetischer Abschnitt des Donors, bestehend aus Intervall 2 (zwischen einem Marker aus M1 und M2) oder Intervall 2 und 3 (zwischen einem Marker aus M1 und M3) nicht mehr vorhanden ist. Derartige Beobachtungen waren für einen Fachmann nicht zu erwarten, da er aus dem Stand der Technik keinen Hinweis auf einen derartigen Linkage Drag in HTN1-Introgressionslinien erhält. Zur Klärung der genetischen Ursache dieses agronomischen Nachteils wurden beispielsweise ausgedehnte Rückkreuzungsprogramme, begleitet von Geno- und Phänotypisierungen durchgeführt. Unterstützt wurden die Arbeiten von einer intensiven Entwicklung zielgenauer und spezifischer molekularer Marker auf dem HTN1-tragenden Chromosomfragment. In Maispflanzen mit dem reduziertem Ertrag (Silageertrag) gelang es die Lage der genomischen Sequenzabschnitte, die für den identifizierten Linkage Drag des Silageertrags verantwortlich sind, eindeutig auf zwei weiteren Intervallen des Donors (nachfolgend viertes Intervall oder Intervall 4 und fünftes Intervall oder Intervall 5 genannt) auf dem Pepitilla-Chromosomfragment zu bestimmen (Beispiel 3C; Figur 3). Eine erfindungsgemäße Maispflanze, welche im HTN1-Introgressionsfragment aus Pepitilla, anstatt des Linkage Drag tragenden vierten und/oder fünften Intervalls des Donors ein entsprechendes Intervall ohne Linkage Drag z.B. aus dem rekurrenten Elter aufweist, zeigt keinen reduzierten Silageertrag und somit einen Ertrag, insbesondere einen Silageertrag, welcher demjenigen einer vergleichbaren Linie ohne Introgression (z.B. isogene Linie oder Ausgangslinie) entspricht. Der Silageertrag einer erfindungsgemäßen Maispflanze ohne vierten und/oder fünften Intervall des Donors im Vergleich mit einer vergleichbare Maispflanze mit Linkage Drag des Silageertrags kann um mehr als 2%, als 3%, als 4%, als 5%, als 6%, als 7%, als 8%, als 9%, als 10%, als 15% oder als 20% höher sein. Das vierte Intervall ist proximal gelegen und eng gekoppelt zu dem Resistenzlocus HTN1 bzw. zu dem ersten Intervall. Das fünfte Intervall ist distal gelegen und eng gekoppelt zu dem Resistenzlocus HTN1 bzw. zu dem ersten Intervall.

Daher ist eine besonders bevorzugten Ausgestaltung der erfindungsgemäßen Maispflanze eine oben beschriebene Maispflanze, wobei das Chromosomfragment weiterhin i) das vierte Intervall des Donors zwischen einem Marker in der dritten Markerregion M3 und einem Marker in einer vierten Markerregion M4, welche durch die Marker MA0004 und MA0005 flankiert ist, oder ii) einen genetischen Abschnitt mit dem vierten Interval zwischen einem Marker in der dritten Markerregion M3 und einem Marker in einer siebten Markerregion M7, welche durch die Marker MA0005 und MA0021 flankiert ist, nicht enthält und/oder wobei das Chromosomfragment weiterhin i) das fünfte Intervall des Donors zwischen einem Marker in einer fünften Markerregion M5, welche durch die Marker MA0006 und PZE-108097482 flankiert ist, und einem Marker in einer sechsten Markerregion M6, welche durch die Marker PZE-108107671 und SYN4196 flankiert ist, oder ii) einen genetischen Abschnitt mit dem fünften Interval zwischen einem Marker in einer achten Markerregion M8, welche durch die Marker MA0022 und MA0013 flankiert ist, und einem Marker in einer sechsten Markerregion M6, welche durch die Marker PZE-108107671 und SYN4196 flankiert ist, nicht enthält. Die flankierenden Marker können der Tabelle 4 entnommen werden. Die Marker MA0004, MA0005, MA0006, MA0013, MA0021, MA0022, PZE-108097482, PZE-108107671 und SYN4196 sind SNP-Marker zur Verwendung im KBioscience-KASP-System (www.lgcgenomics.com/genotyping/kaspgenotyping-reagents/kasp-overview/). Sie definieren eindeutig die Markerregionen M4, M5, M6, M7 und M8, welche zusammen mit M3 die Sequenzabschnitte festlegen, welche im Donor B37HTN1 bzw. Pepitilla den Linkage Drag des Silageertrages tragen. Als polymorphe Marker sind sie überdies auch geeignet zwischen Donor-Allel und beispielsweise dem Allel des rekurrenten Elter zu unterscheiden. Alle Angaben zur Nutzung dieser Marker als KASP-Marker können der Tabelle 4 entnommen werden. Geeignete exemplarische Primer-Hybridisierungsparameter für die PCR werden in Beispiel 2 wiedergegeben. Ein Fachmann ist darüber hinaus auch in der Lage, andere geeignete Hybridisierungsparameter zu bestimmen. Ferner ist es Routinetätigkeit eines Fachmanns in Kenntnis der beschriebenen Markerregionen neben den genannten Markern andere Marker, insbesondere polymorphe Marker, in M4, in M5, in M6, in M7 und/oder in M8 zu entwickeln. Unter Verwendung der hier aufgeführten Marker MA0004, MA0005, MA0006, MA0013, MA0021, MA0022, PZE-108097482, PZE-108107671 und SYN4196 oder selbst entwickelter Marker in M4, in M5, in M6, in M7 und/oder M8 zusammen mit oben beschriebenen Markern in M3 fällt es dem Fachmann leicht festzustellen, ob in einer Maispflanze, in deren Genom ein Chromosomfragment mit HTN1-Resistenzlocus aus dem Donor Pepitilla integriert ist, das vorstehend beschriebene vierte Intervall des Donors und/oder das vorstehend beschriebene fünfte Intervall enthalten ist oder nicht enthalten ist.

Eine weitere besonders bevorzugte Ausgestaltung der erfindungsgemäßen Maispflanze ist eine oben beschriebene Maispflanze, wobei das Chromosomfragment (i) einen genetischen Abschnitt, der das zweite Intervall, das dritte Intervall und das vierte Intervall des Donors umfasst und flankiert ist a) von den Markern SYN14136 und MA0004, b) von den Markern PZE-108076510 und MA0004, c) von den Markern SYN14136 und MA0005 oder d) von den Markern PZE-108076510 und MA0005, nicht enthält, oder (ii) einen genetischen Abschnitt, der das zweite Intervall und das dritte Intervall des Donors umfasst und flankiert ist a) von den Markern SYN14136 und PZE-108093423, b) von den Markern PZE-108076510 und PZE-108093423, c) von den Markern SYN14136 und PZE-108093748 oder d) von den Markern PZE-108076510 und PZE-108093748, und das fünfte Intervall des Donors nicht enthält, oder (iii) einen genetischen Abschnitt, der das zweite Intervall, das dritte Intervall und das vierte Intervall des Donors umfasst und flankiert ist a) von den Markern SYN14136 und MA0004, b) von den Markern PZE-108076510 und MA0004, c) von den Markern SYN14136 und MA0005 oder d) von den Markern PZE-108076510 und MA0005, und das fünfte Intervall des Donors nicht enthält.

Eine weitere besonders bevorzugte Ausgestaltung der erfindungsgemäßen Maispflanze ist eine oben beschriebene Maispflanze, wobei das Chromosomfragment (i) einen genetischen Abschnitt, der das zweite Intervall, das dritte Intervall und das vierte Intervall des Donors umfasst und flankiert ist a) von den Markern SYN14136 und MA0021 oder b) von den Markern PZE-108076510 und MA0021, nicht enthält, oder (ii) einen genetischen Abschnitt, der das zweite Intervall, das dritte Intervall und das vierte Intervall des Donors umfasst und flankiert ist a) von den Markern SYN14136 und MA0021 oder b) von den Markern PZE-108076510 und MA0021, und das fünfte Intervall des Donors nicht enthält, oder (iii) einen genetischen Abschnitt, der das zweite Intervall, das dritte Intervall und das vierte Intervall des Donors umfasst und flankiert ist a) von den Markern SYN14136 und MA0021 oder b) von den Markern PZE-108076510 und MA0021, und einen zweiten genetischen Abschnitt, der das fünfte Intervall des Donors umfasst und flankiert ist a) von den Markern MA0022 und PZE-108107671, b) von den Markern MA0022 und SYN4196, c) von den Markern MA0013 und PZE-108107671 oder von den Markern MA0013 und SYN4196, nicht enthält, oder (iv) einen genetischen Abschnitt, der das zweite Intervall und das dritte Intervall des Donors umfasst und flankiert ist a) von den Markern SYN14136 und PZE-108093423, b) von den Markern PZE-108076510 und PZE-108093423, c) von den Markern SYN14136 und PZE-108093748 oder d) von den Markern PZE-108076510 und PZE-108093748, und einen zweiten genetischen Abschnitt, der das fünfte Intervall des Donors umfasst und flankiert ist a) von den Markern MA0022 und PZE-108107671, b) von den Markern MA0022 und SYN4196, c) von den Markern MA0013 und PZE-108107671 oder von den Markern MA0013 und SYN4196, nicht enthält, oder (v) einen genetischen Abschnitt, der das zweite Intervall, das dritte Intervall und das vierte Intervall des Donors umfasst und flankiert ist a) von den Markern SYN14136 und MA0021 oder b) von den Markern PZE-108076510 und MA0021, und einen zweiten genetischen Abschnitt, der das fünfte Intervall des Donors umfasst und flankiert ist a) von den Markern MA0022 und PZE-108107671, b) von den Markern MA0022 und SYN4196, c) von den Markern MA0013 und PZE-108107671 oder von den Markern MA0013 und SYN4196, nicht enthält.

Die der vorliegenden Erfindung zugrundeliegende Aufgabe ist alternativ durch eine Maispflanze gelöst, in deren Genom ein Chromosomfragment aus dem Donor Pepitilla integriert ist, wobei das Chromosomfragment das erste Intervall des Donors umfasst, welches Donor-Allele gemäß dem Haplotyp nach Tabelle 2 aufzeigt und das Polynukleotid aufweist, das in der Maispflanze Resistenz gegenüber *Helminthosporium turcicum* vermittelt, und wobei das Chromosomfragment i) das vierte Intervall des Donors zwischen einem Marker in der dritten Markerregion, welche durch die Marker PZE-108093423 und PZE-108093748 flankiert ist, und einem Marker in der vierten Markerregion, welche durch die Marker MA0004 und MA0005 flankiert ist, oder ii) einen genetischen Abschnitt mit dem vierten Intervall zwischen einem Marker in der dritten Markerregion M3 und einem Marker in der siebten Markerregion M7, welche durch die Marker MA0005 und MA0021 flankiert ist, nicht enthält. Die obige Beschreibung im Hinblick beispielsweise auf Marker, auf das Polynukleotid oder auf die Phänotypisierung gilt entsprechend auch für diese und jede weitere alternative Lösung der Aufgabe sowie offenbarten Ausgestaltungen.

Eine bevorzugte Ausgestaltung dieser erfindungsgemäßen Maispflanze ist eine oben beschriebene Maispflanze, wobei das Chromosomfragment i) das vierte Intervall des Donors, welches flankiert ist a) von den Markern PZE-108093423 und MA0004, b) von den Markern PZE-108093748 und MA0004, c) von den Markern PZE-108093423 und MA0005 oder d) von den Markern PZE-108093748 und MA0005, nicht enthält oder ii) einen genetischen Abschnitt, der das vierte Intervall des Donors umfasst und flankiert ist a) von den Markern PZE-108093423 und MA0021 oder b) von den Markern PZE-108093748 und MA0021, nicht enthält.

Eine weitere bevorzugte Ausgestaltung der erfindungsgemäßen Maispflanze ist eine vorstehend beschriebene Maispflanze, wobei das Chromosomfragment weiterhin das dritte Intervall des Donors zwischen einem Marker in der zweiten Markerregion M2 und einem Marker in der dritten Markerregion M3 nicht enthält.

Eine weitere bevorzugte Ausgestaltung der erfindungsgemäßen Maispflanze ist eine oben beschriebene Maispflanze, wobei das Chromosomfragment einen genetischen Abschnitt, der das dritte Intervall und das vierte Intervall des Donors umfasst und flankiert ist a) von den Markern SYN24931 und MA0004, b) von den Markern PZE-108077560 und MA0004, c) von den Markern SYN24931 und MA0005, d) von den Markern PZE-108077560 und MA0005, e) von den Markern SYN24931 und MA0021 oder f) von den Markern PZE-108077560 und MA0021 nicht enthält.

Eine weitere bevorzugte Ausgestaltung der erfindungsgemäßen Maispflanze ist eine vorstehend beschriebene Maispflanze, wobei das Chromosomfragment i) weiterhin das fünfte Intervall des Donors zwischen einem Marker in der fünften Markerregion M5 und einem Marker in der sechsten Markerregion M6 oder ii) einen genetischen Abschnitt mit dem fünften Interval zwischen einem Marker in der achten Markerregion M8 und einem Marker in der sechsten Markerregion M6 nicht enthält.

Eine weitere besonders bevorzugte Ausgestaltung der erfindungsgemäßen Maispflanze ist eine vorstehend beschriebene Maispflanze, wobei das Chromosomfragment i) einen genetischen Abschnitt, der das das dritte Intervall und das vierte Intervall des Donors umfasst und flankiert ist a) von den Markern SYN24931 und MA0004, b) von den Markern PZE-108077560 und MA0004, c) von den Markern SYN24931 und MA0005 oder d) von den Markern PZE-108077560 und MA0005, und das fünfte Intervall nicht enthält, oder ii) einen genetischen Abschnitt, der das das dritte Intervall und das vierte Intervall des Donors umfasst und flankiert ist a) von den Markern SYN24931 und MA0004, b) von den Markern PZE-108077560 und MA0004, c) von den Markern SYN24931 und MA0005 oder d) von den Markern PZE-108077560 und MA0005, und einen zweiten genetischen Abschnitt, der das das fünfte Intervall umfasst und flankiert ist a) von den Markern MA0022 und SYN4196, b) von den Markern MA0022 und PZE-108107671, c) von den Markern MA0013 und SYN4196 oder von den Markern MA0013 und PZE-108107671, nicht enthält.

Eine weitere besonders bevorzugte Ausgestaltung der erfindungsgemäßen Maispflanze ist eine vorstehend beschriebene Maispflanze, wobei das Chromosomfragment i) einen genetischen Abschnitt, der das das dritte Intervall und das vierte Intervall des Donors umfasst und flankiert ist a) von den Markern SYN24931 und MA00021 oder b) von den Markern PZE-108077560 und MA00021, und das fünfte Intervall nicht enthält, oder ii) einen genetischen Abschnitt, der das das dritte Intervall und das vierte Intervall des Donors umfasst und flankiert ist a) von den Markern SYN24931 und MA00021 oder b) von den Markern PZE-108077560 und MA00021, und einen zweiten genetischen Abschnitt, der das das fünfte Intervall umfasst und flankiert ist a) von den Markern MA0022 und PZE-108107671, b) von den Markern MA0022 und SYN4196, c) von den Markern MA0013 und PZE-108107671 oder von den Markern MA0013 und SYN4196, nicht enthält.

Die der vorliegenden Erfindung zugrundeliegende Aufgabe ist ferner alternativ durch eine Maispflanze gelöst, in deren Genom ein Chromosomfragment aus dem Donor Pepitilla integriert ist, wobei das Chromosomfragment das erste Intervall des Donors umfasst, welches Donor-Allele gemäß dem Haplotyp nach Tabelle 2 aufzeigt und das Polynukleotid aufweist, das in der Maispflanze Resistenz gegenüber *Helminthosporium turcicum* vermittelt, und wobei das Chromosomfragment i) das fünfte Intervall des Donors zwischen einem Marker in der fünften Markerregion, welche durch die Marker MA0006 und PZE-108097482 flankiert ist, und einem Marker in der sechsten Markerregion, welche durch die Marker PZE-108107671 und SYN4196 flankiert ist, oder ii) einen genetischen Abschnitt mit dem fünften Intervall zwischen einem Marker in der achten Markerregion M8, welche durch die Marker MA0022 und MA0013 flankiert ist, und einem Marker in der sechsten Markerregion M6, welche durch die Marker PZE-108107671 und SYN4196 flankiert ist, nicht enthält.

Eine weitere bevorzugte Ausgestaltung der erfindungsgemäßen Maispflanze ist eine vorstehend beschriebene Maispflanze, wobei das Chromosomfragment weiterhin das dritte Intervall des Donors zwischen einem Marker in der zweiten Markerregion M2 und einem Marker in der dritten Markerregion M3 nicht enthält.

Eine weitere besonders bevorzugte Ausgestaltung der erfindungsgemäßen Maispflanzen ist eine der oben beschriebene Maispflanze, wobei das Chromosomfragment flankiert ist a) von einem Marker in der zweiten Markerregion M2 und einem Marker in der sechsten Markerregion M6, b) von einem Marker in der dritten Markerregion M3 und einem Marker in der sechsten Markerregion M6, c) von einem Marker in der vierten Markerregion M4 und einem Marker in der sechsten Markerregion M6, d) von einem Marker in der siebten Markerregion M7 und einem Marker in der sechsten Markerregion M6, e) von einem Marker in der Markerregion M1 und einem Marker in der Markerregion M5, f) von einem Marker in der zweiten Markerregion M2 und einem Marker in der fünften Markerregion M5, g) von einem Marker in der dritten Markerregion M3 und einem Marker in der fünften Markerregion M5, h) von einem Marker in der vierten Markerregion M4 und einem Marker in der fünften Markerregion M5, i) von einem Marker in der siebten Markerregion M7 und einem Marker in der fünften Markerregion M5, j) von einem Marker in der Markerregion M1 und einem Marker in der Markerregion M8, k) von einem Marker in der zweiten Markerregion M2 und einem Marker in der achten Markerregion M8, I) von einem Marker in der dritten Markerregion M3 und einem Marker in der achten Markerregion M8, m) von einem Marker in der vierten Markerregion M4 und einem Marker in der achten Markerregion M8, oder n) von einem Marker in der siebten Markerregion M7 und einem Marker in der achten Markerregion M8.

Eine weitere ganz besonders bevorzugte Ausgestaltung der erfindungsgemäßen Maispflanzen ist eine der oben beschriebene Maispflanze, wobei das Chromosomfragment flankiert ist a) durch die Marker SYN24931 und SYN4196, b) durch die Marker PZE-108077560 und SYN4196, c) durch die Marker SYN24931 und PZE-108107671, d) durch die Marker PZE-108077560 und PZE-108107671, e) durch die Marker PZE-108093423 und SYN4196, f) durch die Marker PZE-108093748 und SYN4196, g) durch die Marker PZE-108093423 und PZE-108107671, h) durch die Marker PZE-108093748 und PZE-108107671, i) durch die Marker MA0004 und SYN4196, j) durch die Marker MA0005 und SYN4196, k) durch die Marker MA0004 und PZE-108107671, I) durch die Marker MA0005 und PZE-108107671, m) durch die Marker MA0021 und SYN4196, n) durch die Marker MA0021 und PZE-108107671, o) durch die Marker PZE-108076510 und MA0006, p) durch die Marker SYN14136 und MA0006, q) durch die Marker PZE-108076510 und PZE-108097482, r) durch die Marker SYN14136 und PZE-108097482, s) durch die Marker SYN24931 und PZE-108097482, t) durch die Marker PZE-108077560 und PZE-108097482, u) durch die Marker SYN24931 und MA0006, v) durch die Marker PZE-108077560 und MA0006, w) durch die Marker PZE-108093423 und PZE-108097482, x) durch die Marker PZE-108093748 und PZE-108097482, y) durch die Marker PZE-108093423 und MA0006, z) durch die Marker PZE-108093748 und MA0006, aa) durch die Marker MA0004 und PZE-108097482, ab) durch die Marker MA0005 und PZE-108097482, ac) durch die Marker MA0004 und MA0006, ad) durch die Marker MA0005 und MA0006, ae) durch die Marker MA0021 und PZE-108097482, af) durch die Marker MA0021 und MA0006, ag) durch die Marker PZE-108076510 und MA0013, ah) durch die Marker SYN14136 und MA0013, ai) durch die Marker PZE-108076510 und MA0022, aj) durch die Marker SYN14136 und MA0022, ak) durch die Marker SYN24931 und MA0013, al) durch die Marker PZE-108077560 und MA0013, am) durch die Marker SYN24931 und MA0022, an) durch die Marker PZE-108077560 und MA0022, ao) durch die Marker PZE-108093423 und MA0013, ap) durch die Marker PZE-108093748 und MA0013, aq) durch die Marker PZE-108093423 und MA0022, ar) durch die Marker PZE-108093748 und MA0022, as) durch die Marker MA0004 und MA0013, at) durch die Marker MA0005 und MA0013, au) durch die Marker MA0004 und MA0022, av) durch die Marker MA0005 und MA0022, aw) durch die Marker MA0021 und MA0013, ax) durch die Marker MA0021 und MA0022.

Eine weitere besonders bevorzugte Ausgestaltung der erfindungsgemäßen Maispflanzen ist eine der oben beschriebene Maispflanze, wobei das Chromosomfragment a) zwischen einem Marker in der zweiten Markerregion M2 und einem Marker in der sechsten Markerregion M6, b) zwischen einem Marker in der dritten Markerregion M3 und einem Marker in der sechsten Markerregion M6, c) zwischen einem Marker in der vierten Markerregion M4 und einem Marker in der sechsten Markerregion M6, d) zwischen einem Marker in der siebten Markerregion M7 und einem Marker in der sechsten Markerregion M6, e) zwischen einem Marker in der ersten Markerregion M1 und einem Marker in der fünften Markerregion M5 f) zwischen einem Marker in der zweiten Markerregion M2 und einem Marker in der fünften Markerregion M5, g) zwischen einem Marker in der dritten Markerregion M3 und einem Marker in der fünften Markerregion M5, h) zwischen einem Marker in der vierten Markerregion M4 und einem Marker in der fünften Markerregion M5, i) zwischen einem Marker in der siebten Markerregion M7 und einem Marker in der fünften Markerregion M5, j) zwischen einem Marker in der Markerregion M1 und einem Marker in der Markerregion M8, k) zwischen einem Marker in der zweiten Markerregion M2 und einem Marker in der achten Markerregion M8, I) zwischen einem Marker in der dritten Markerregion M3 und einem Marker in der achten Markerregion M8, m) zwischen einem Marker in der vierten Markerregion M4 und einem Marker in der achten Markerregion M8, oder n) zwischen einem Marker in der siebten Markerregion M7 und einem Marker in der achten Markerregion M8 lokalisiert ist.

**Tabelle 4: KASP-Marker Primer-Sequenzen und die Zuordnung zum B37HTN1-Donor Allele stammend aus der Landrasse Pepitilla (Allel X und Allel Y: beschreiben die biallelischen Werte der SNPs)**

| SNP-Marker | Marker-position AGPv02 | Primer Allele X(5'-3') | Primer Allele Y(5' -3') | Gemeinsamer Primer (5'-3') | B37HTN1 Donor Allele (SNP) | Markerregion |
|---|---|---|---|---|---|---|
| | [bp] | [SEQ ID NO] | [SEQ ID NO] | [SEQ ID NO] | | |
| SYN14136 | 131681497 | 17 | 18 | 19 | A | M1 |
| PZE-108076510 | 131905855 | 20 | 21 | 22 | G | M1 |
| SYN24931 | 132877982 | 23 | 24 | 25 | A | M2 |
| PZE-108077560 | 133189880 | 26 | 27 | 28 | A | M2 |
| PZE-108093423 | 150279048 | 29 | 30 | 31 | A | M3 |
| PZE-108093748 | 150562764 | 32 | 33 | 34 | G | M3 |
| PZE-108107671 | 161543406 | 35 | 36 | 37 | C | M6 |
| SYN4196 | 161766769 | 38 | 39 | 40 | C | M6 |
| MA0004 | 151688652 | 41 | 42 | 43 | A | M4 |
| MA0005 | 151831049 | 44 | 45 | 46 | C | M4/M7 |
| MA0021 | 151907173 | 241 | 242 | 243 | G | M7 |
| MA0006 | 152888310 | 47 | 48 | 49 | A | M5 |
| PZE-108097482 | 153139646 | 50 | 51 | 52 | A | M5 |
| MA0002 | 147720853 | 53 | 54 | 55 | A | |
| MA0003 | 151346184 | 56 | 57 | 58 | C | |
| MA0007 | 152045106 | 59 | 60 | 61 | T | |
| MA0008 | 152045141 | 62 | 63 | 64 | T | |
| MA0009 | 152045402 | 65 | 66 | 67 | T | |
| MA0010 | 152045516 | 68 | 69 | 70 | C | |
| MA0011 | 152045912 | 71 | 72 | 73 | T | |
| MA0012 | 152046502 | 74 | 75 | 76 | A | |
| MA0022 | 152046529 | 244 | 245 | 246 | A | M8 |
| MA0013 | 152133057 | 77 | 78 | 79 | G | M8 |
| MA0014 | 152133380 | 80 | 81 | 82 | T | |
| MA0015 | 152144310 | 83 | 84 | 85 | A | |
| MA0016 | 152250992 | 86 | 87 | 88 | A | |
| MA0017 | 152301656 | 89 | 90 | 91 | A | |
| MA0018 | 152304127 | 92 | 93 | 94 | A | |
| MA0019 | 152630794 | 95 | 96 | 97 | C | |
| MA0020 | 152753635 | 98 | 99 | 100 | A | |
| PZE-108095998 | 152433358 | 101 | 102 | 103 | T | |
| PZE-108096011 | 152435855 | 104 | 105 | 106 | A | |
| PZE-108096610 | 152703579 | 107 | 108 | 109 | C | |
| PZE-108096791 | 152887338 | 110 | 111 | 112 | G | |

Die vorliegende Erfindung bezieht sich weiterhin auch auf einen Samen oder ein Korn, ein Gewebe, ein Organ, einen Teil und eine Zelle der oben beschriebenen erfindungsgemäßen Maispflanzen. Dabei ist der Samen oder das Korn ein Samen oder ein Korn, in deren Genom das Chromosomfragment der oben beschriebenen Ausgestaltungen der Erfindung integriert ist.

In einem weiteren Aspekt betrifft die vorliegende Erfindung ein Verfahren zur Identifizierung einer *H. turcicum* resistenten Maispflanze, in deren Genom ein Chromosomfragment aus dem Donor Pepitilla integriert ist, umfassend den Nachweis von mindestens zwei Allelen im Genom der Pflanze, wobei mindestens ein Allel in einem genomischen Abschnitt lokalisiert ist, welcher durch einen Marker in der ersten Markerregion M1, der zweiten Markerregion M2, der dritten Markerregion M3, der vierten Markerregion M4 oder der siebten Markerregion M7 und durch das oben beschriebene Polynukleotid, das in der Maispflanze Resistenz gegenüber *H. turcicum* vermittelt, flankiert ist, und wobei mindestens ein Allel in einem genomischen Abschnitt lokalisiert ist, welcher durch das Polynukleotid und durch einen Marker in der sechsten Markerregion M6, der fünften Markerregion M5 oder der achten Markerregion M8 flankiert ist. Die Markerregionen sowie beispielhafte Marker in diesen Markerregionen sind oben beschrieben. Vorzugsweise ist die identifizierte Maispflanze eine erfindungsgemäße Maispflanze. Die Erfindung betrifft weiterhin auch eine Maispflanze, welche mit dem genannten Verfahren zur Identifizierung identifiziert wurde.

In einem weiteren Aspekt erfasst die vorliegende Erfindung ein Verfahren zur Erhöhung des Ertrags einer *H. turcicum* resistenten Maispflanze, in deren Genom ein Chromosomfragment aus dem Donor Pepitilla integriert ist, wobei das Verfahren einen Schritt umfasst, welcher die Entfernung des zweiten Intervalls des Donors, vierten Intervalls des Donors oder fünften Intervalls des Donors bewirkt und wobei das Chromosomfragment das oben beschriebene erste Intervall des Donors umfasst, welches Donor-Allele gemäß dem Haplotyp nach Tabelle 2 aufzeigt und ein Polynukleotid aufweist, das in der Maispflanze Resistenz gegenüber *Helminthosporium turcicum* vermittelt. Beispielsweise kann die Entfernung durch genetische Rekombination während eines Kreuzungsprozesses zwischen zwei Maispflanzen erreicht werden, wobei eine Elter-Maispflanze den HTN1-Resistenzlocus aus Pepitilla trägt. Neben der Verwendung von konventionellen Züchtungstechniken zur Erzeugung einer genetischen Rekombination, die als Ergebnis das Ersetzen von mindestens einem der oben identifizierten Donor-Intervalle mit Linkage Drag durch genomische Sequenzen des rekurrenten Elter, welche bevorzugt frei sind von unerwünschten Genen, hat, stellt die moderne Biotechnologie dem Fachmann diverse weitere Werkzeuge zur Verfügung, welche ein präzises Genom-Engineering ermöglichen. Zu den bekannten Werkzeugen zählen beispielsweise (Meganukleasen (Silva et al., 2011), Homing-Endonukleasen (Chevalier 2002), Zinkfinger-Nukleasen, TALE-Nukleasen (WO 2010/079430; WO 2011/072246) oder CRISPR (Gaj et al., 2013). Hierbei handelt es sich um artifiziell Nuklease-Fusionsproteine, die in der Lage sind gezielt doppelsträngige Nukleinsäuremoleküle wie pflanzliche DNA zu schneiden und damit Doppelstrangbrüche an gewünschten Positionen im Genom zu erzeugen. Unter Nutzbarmachung der zelleigenen Mechanismen zur Reparatur von induzierten Doppelstrangbrüchen kann dann eine homologe Rekombination oder ein ,nonhomologous end-joining' bewirkt werden, welche zur Entfernung der Linkage Drag-tragenden Intervalle des Donors führen kann. Geeignete Zielsequenzen im Genom für die Erkennungsdomänen vorstehender Nukleasen können beispielsweise den Sequenzinformationen zu den SNP-Markern (Tabelle 4) oder in deren Intervallen entnommen werden. Ein Fachmann ist jedoch auch in der Lage, andere Sequenzen, bevorzugt innerhalb oder zwischen den oben beschriebenen sechs Markerregionen, zu identifizieren, welche als Zielsequenzen für die Erkennungsdomäne der Nukleasen geeignet sind.

Im Folgenden sollen hierzu zwei gentechnische Ansätze näher erläutert werden, mit deren Hilfe die Eliminierung von Linkage Drag-tragenden Nukleotidsequenzen aus einem pflanzlichen Genom unterstützt wird oder unmittelbar erreicht wird. Folgende Verfahren, sowie auch die konventionellen Züchtungsverfahren, können zur Herstellung der erfindungsgemäßen Maispflanzen herangezogen werden.

Wie bereits ausgeführt sind vorstehende molekulare Werkzeuge in der Lage an definierten Orten im Genom einer Pflanze DNA-Doppelstrangbrüche zu induzieren. Als besonders vorteilhaft zeigt sich hierbei die Verwendung von TALE-Nukleasen (TALENs) oder Zinkfinger-Nukleasen (ZFNs). Die TALE- oder ZF-Erkennungsdomäne erlaubt es an beliebiger Stelle im Genom spezifisch zu binden. In Kenntnis der Sequenz am Zielbereich kann man die TALE- oder ZF Erkennungsdomäne maßgeschneidert designen, so dass sie ausschließlich an gewünschter Stelle im Genom bindet. Ist die Erkennungssequenz beispielsweise mit eine unspezifischen Endonukease wie Fokl fusioniert, kann man an definierter Stelle im Genom einen Doppelstrangbruch (DSB) induzieren, was ein gezieltes Genom-Engineering ermöglicht (Tzfira et al., 2012; Li et al., 2011; Puchta and Hohn 2010). Dem Fachmann ist der Umgang mit Fokl-Endonukleasen sowie die Erstellung von geeigneten TALENs und ZFNs aus dem Stand der Technik bekannt.

Ein induzierter Doppelstrangbruch kann beispielsweise eine homologe Rekombination zwischen einem endogenen Ziel-Genlocus (z.B. eine der vorstehenden Markerregionen) und einem exogen eingebrachten homologen DNA-Fragment, welches beispielsweise nicht Täger von Linkage Drag ist (z.B. auf einem geeigneten Spendervektor), stimulieren. Dieses sogenannte gene replacement' oder genome editing` kann in vitro durchgeführt werden und bedarf keines Schrittes der Kreuzung zwischen zwei Pflanzen. Hierfür müssen die zu modifizierenden Pflanzen zum einen mit Nukleinsäuren, kodierend für die designten TALENs oder ZFNs, und zum anderen mit dem exogenen DNA-Fragment transient transformiert werden. Das DNA-Fragment kann hierbei aus einer Pflanze derselben Spezies stammen und entspricht beispielsweise dem chromosomalen Abschnitt, welcher ersetzt werden soll, nur ohne Linkage Drag. Nach Abschluss der induzierten homologen Rekombination können Zellen mit modifizierten Genom zu Pflanzen regeneriert werden und dahingehend selektiert werden, ob der Linkage Drag erfolgreich entfernt wurde und die zuvor transient transformierten DNA-Elemente im Zuge der regenerativen Zellteilung wieder verloren gegangen sind . Hierzu können auch oben beschriebene Marker eingesetzt werden. Methoden zur Transformation und Regeneration sind aus dem Stand der Technik bekannt und werden auch weiter unten nochmals erläutert.

Weiterhin können vorstehende TALENs und ZFNs auch im Prozess der Meiose transgen eingesetzt werden, wo sie an vorbestimmten Stellen im Genom Doppelstrangbrüche induzieren und somit die Wahrscheinlichkeit für eine Rekombination an diesen Stellen im Schritt des Crossing over` erhöhen. Dadurch kann die Elimierung von Linkage Drag deutlich begünstigt werden. Einem Fachmann ist bekannt, wie er nach Abschluss der Meiose aus den haploiden Zellen Linkage-Drag-freie und TALENs bzw. ZFNs-freie Pflanzen erzeugt.ln einem weiteren Aspekt betrifft die vorliegende Erfindung ein Verfahren zur Herstellung einer erfindungsgemäßen Maispflanze, welches die folgenden Schritte umfasst: (A) Bereitstellen einer ersten Maispflanze, in deren Genom ein Chromosomfragment aus dem Donor Pepitilla integriert ist, wobei das Chromosomfragment ein erstes Intervall des Donors umfasst, welches Donor-Allele gemäß dem Haplotyp nach Tabelle 2 aufzeigt und ein Polynukleotid aufweist, das in der Maispflanze Resistenz gegenüber *Helminthosporium turcicum* vermittelt, und wobei das Chromosomfragment ein zweites Intervall des Donors und/oder das vierte Intervall des Donors und/oder das fünfte Intervall des Donors enthält, (B) Bereitstellen einer zweiten Maispflanze, (C) Kreuzen der Maispflanze aus (A) mir der Maispflanze aus (B) und (D) Selektieren einer erfindungsgemäßen Maispflanze, vorzugsweise unter Verwendung von mindestens einem oben beschriebenen Marker. Alternativ betrifft die vorliegende Erfindung ein Verfahren zur Herstellung einer erfindungsgemäßen Maispflanze, welches die folgenden Schritte umfasst: (A) Transientes Transformieren einer Maispflanzenzelle mit einer ersten Nukleotidsequenz, welche für ein erstes Protein mit Endonuklease-Aktivität (z.B. ein TALE- oder ZF-Endonuklease- Fusionsprotein) kodiert, das in der Lage ist zwischen Markerregion M2 und M4 eine Doppelstrangbruch der DNA im Genom der Maispflanzenzelle zu induzieren, und mit einer zweiten Nukleotidsequenz, welche für ein zweites Protein mit Endonuklease-Aktivität (z.B. ein TALE- oder ZF-Endonuklease- Fusionsprotein) kodiert, das in der Lage ist zwischen Markerregion M5 und M6 eine Doppelstrangbruch der DNA im Genom der Maispflanzenzelle zu induzieren, (B) Transientes Einbringen eines Spendervektors in die erste Maispflanzenzelle, welcher ein Chromosomfragment aus dem Donor Pepitilla trägt, wobei das Chromosomfragment ein erstes Intervall des Donors umfasst, welches Donor-Allele gemäß dem Haplotyp nach Tabelle 2 aufzeigt und ein Polynukleotid aufweist, das in der Maispflanze Resistenz gegenüber *Helminthosporium turcicum* vermittelt, und wobei das Chromosomfragment weiterhin die chromosomalen Abschnitte des Donors Pepitilla zwischen den Stellen der Doppelstrangbrüche aus (A) aufweist, sodass zwischen dem Genom der ersten Maispflanzenzelle und dem Chromsomfragment des Spendervektors eine homologe Rekombination stattfindet, (C) Regenation einer Maispflanze aus der Maispflanzenzelle, (D) Identifikation einer erfindungsgemäßen Maispflanze, vorzugsweise unter Verwendung von mindestens einem oben beschriebenen Marker. Besonders bevorzugt sind transient eingebrachte erste und zweite Nunkleinsäuresequenzen und Spedervektor wieder verloren gegangen. Dem Fachmann ist bekannt wir er dieses erreicht und nachweisen kann.

In einem weiteren Aspekt sind von der vorliegenden Erfindung die oben beschriebenen Marker als Oligonukleotide, insbesondere Primer-Oligonukleotide erfasst. Vorzugsweise sind die Oligonukleotide isolierte Oligonukleotide. Ein Oligonukleotid umfasst ein Nukleinsäuremolekül mit einer Nukleotidsequenz, ausgewählt aus einer der SEQ ID NOs: 41-49, 53-100 und 229-250. Ferner betrifft die vorliegende Erfindung die Verwendung eines Oligonukleotids, welches ein Nukleinsäuremolekül mit einer Nukleotidsequenz, ausgewählt aus einer der SEQ ID NOs: 17-250, umfasst, zur Identifizierung einer *H. turcicum* resistenten Maispflanze. Vorzugsweise stammt die Resistenz aus dem Donor Pepitilla und ist HTN1.

Die der vorliegenden Erfindung zugrundeliegende Aufgabe ist weiterhin alternativ durch eine transgene Pflanze, insbesondere eine transgene Maispflanze, gelöst, welche eine unten beschriebene transgene Pflanzenzelle umfasst. Ferner betrifft die Erfindung auch einen Teil dieser erfindungsgemäßen Pflanze, wobei ein Teil eine Zelle, ein Gewebe, ein Organ oder ein Zusammenschluss von mehreren Zellen, Geweben oder Organen sein kann. Ein Zusammenschluss von mehreren Organen ist z.B. eine Blüte oder ein Samen. In einer besonderen Ausgestaltung betrifft die Erfindung einen Samen von der transgenen Pflanze, wobei der Samen das weiter unten beschriebene erfindungsgemäße Polynukleotid als Transgen umfasst. Bevorzugt zeigt eine transgene Pflanze der vorliegenden Erfindung, insbesondere eine Pflanze der Spezies *Zea mays,* gegenüber *H. turcicum* eine höhere Resistenz als eine korrespondierende nicht-transformierte Pflanze (isogene Pflanze ohne das Transgen). Eine erfindungsgemäße transgene Ht-resistente Pflanze zeigt eine erhöhte Resistenz gegenüber *H. turcicum* von mindestens 1 Boniturnote, bevorzugt von mindestens 2 Boniturnoten oder mindestens 3 Boniturnoten und besonders bevorzugt von mindestens 4 Boniturnoten (siehe Boniturschema in Tabelle 3).

Ferner stellt die Erfindung ein Verfahren zur Herstellung einer transgenen Pflanze bereit, welches einen Schritt des Einbringens des erfindungsgemäßen Polynukleotids oder des weiter unten beschriebenen Vektors der vorliegenden Erfindung in eine pflanzliche Zelle und optional einen Schritt der Selektion einer transgenen Pflanzenzelle umfasst. Weiterhin ist ein solches Verfahren zur Herstellung einer transgenen Pflanze durch einen anschließenden Schritt gekennzeichnet, der die Regenerierung der transgenen Pflanze aus der im ersten Schritt erzeugten transgenen Pflanzenzelle einschließt. Methoden zur Regeneration sind dem Fachmann aus dem Stand der Technik bekannt.

In einem zusätzlichen Aspekt offenbart die vorliegende Erfindung das Polynukleotid, welches ein oder mehrere Resistenz-vermittelnde Gene des HTN1-Locus aus Pepitilla (Tabelle 1) oder ausgewählt aus RLK1 und EXT1 (siehe Tabelle 1) oder Genallele davon. Gen oder Genallel können unter Befallsbedingungen mit *H. turcicum* einen Resistenzphänotyp mit den HTN1-typischen Merkmalen bewirken. Strukturell ist das Polynukleotid dadurch gekennzeichnet, dass es ein Nukleinsäuremolekül umfasst, (a) das eine Nukleotidsequenz gemäß SEQ ID NO: 1, 3, 5, 7, 9, 11, 13 oder 15 aufweist, (b) das eine Nukleotidsequenz mit einer Identität von mindestens 80%, 85%, 90%, 91%, 92%, 93%, 94%, 95%, 96%, 97%, 98% oder 99% zu einer der Nukleotidsequenz gemäß SEQ ID NO: 1, 3, 5, 7, 9, 11, 13 und 15 vorzugsweise über die gesamte Sequenzlänge, aufweist, (c) das mit dem komplementären Strang eines Nukleinsäuremoleküls nach (a) oder (b) unter stringenten Bedingungen hybridisiert, (d) das ein Polypeptid mit einer Aminosäuresequenz gemäß SEQ ID NO: 2, 4, 6, 8, 10, 12, 14 oder 16 kodiert, oder (e) das ein Polypeptid mit einer Aminosäuresequenz, die zu mindestens 80%, 85%, 90%, 91%, 92%, 93%, 94%, 95%, 96%, 97%, 98% oder 99% identisch ist mit einer der Aminosäuresequenzen nach (d), kodiert. In einer bevorzugten Ausgestaltung ist das Polynukelotid dadurch gekennzeichnet, dass es ein Nukleinsäuremolekül umfasst, (aa) das eine Nukleotidsequenz gemäß SEQ ID NO: 1 oder 5 aufweist, (bb) das eine Nukleotidsequenz mit einer Identität von mindestens 80%, 85%, 90%, 91%, 92%, 93%, 94%, 95%, 96%, 97%, 98% oder 99% zu einer der Nukleotidsequenz gemäß SEQ ID NO: 1 oder 5 vorzugsweise über die gesamte Sequenzlänge, aufweist, (cc) das mit dem komplementären Strang eines Nukleinsäuremoleküls nach (aa) oder (bb) unter stringenten Bedingungen hybridisiert, (dd) das ein Polypeptid mit einer Aminosäuresequenz gemäß SEQ ID NO: 2 oder 6 kodiert, oder (ee) das ein Polypeptid mit einer Aminosäuresequenz, die zu mindestens 80%, 85%, 90%, 91%, 92%, 93%, 94%, 95%, 96%, 97%, 98% oder 99% identisch ist mit einer der Aminosäuresequenzen nach (dd), kodiert. Vorzugsweise kann das Polynukleotid isoliert und/oder gereinigt von seiner natürlichen genetischen Umgebung sein oder liegt in im Wesentlichen reiner oder homogener Form vor. Bevorzugt ist das Polynukelotid DNA, und besonders bevorzugt cDNA, d.h. das Polynukleotid umfasst die cDNA vom einem oder mehreren Resistenz-vermittelnde Genen (Tabelle 1). Es kann aber auch als RNA vorliegen. Dem Fachmann ist bekannt, wie er aus der hier offenbarten Sequenzinformation die genomische DNA-Sequenz ableiten kann. Ein erfindungsgemäßes Polynukleotid kodiert mindestens ein Polypeptid, das in der Lage ist, eine Resistenz gegenüber dem Pathogen *Helminthosporium turcicum* in einer Pflanze, in welcher das Polypeptid exprimiert wird, zu vermitteln. Bevorzugt vermittelt das Polypeptid, welches durch das erfindungsgemäße Polynukleotid oder Teilen davon kodiert wird, insbesondere in einer Pflanze der Gattung *Zea* oder in einer Pflanze der Spezies *Zea mays* eine Resistenz gegenüber dem Pathogen *Helminthosporium turcicum.*

Die vorliegende Erfindung betrifft weiterhin auch ein Polypeptid, welches in der Lage ist eine Resistenz gegenüber *H. turcicum* in einer Pflanze, in welcher das Polypeptid exprimiert wird, zu vermitteln und welches durch das erfindungsgemäße Polynukleotid oder einem Teil davon kodiert wird. das Polypeptid weist bevorzugt eine Aminosäuresequenz gemäß SEQ ID NO: 2, 4, 6, 8, 10, 12, 14 oder 16 oder besonders bevorzugt eine Aminosäuresequenz gemäß SEQ ID NO: 2 oder 6. Bei dem Polypeptid kann es sich um ein isoliertes Polypeptid handeln.

In einem weiteren Aspekt betrifft die vorliegende Erfindung einen Vektor, welcher das erfindungsgemäße Polynukleotid umfasst. Bei dem Vektor kann es sich um ein Plasmid, ein Cosmid, eine Phage oder einen Expressionsvektor, einen Transformationsvektor, Shuttle-Vektor oder Klonierungsvektor handelt, er kann doppel- oder einzelsträngig, linear oder zirkulär sein oder kann einen prokaryotischen oder eukaryotischen Wirt entweder durch Integration in dessen Genom oder extrachromosomal transformieren. Bevorzugt ist das erfindungsgemäße Polynukleotid in einem Expressionsvektor mit einer oder mehreren regulatorischen Sequenzen, welche die Transkription und optional die Expression in einer prokaryotischen oder eukaryotischen Wirtszelle erlauben, operativ verknüpft. Beispielsweise steht das Polynukleotid unter der Kontrolle eines geeigneten Promotors oder eines Terminators. Geeignete Promotoren können Promotoren sein, welche konstitutiv induziert werden (Bsp.: 35S-Promoter aus dem "Cauliflower mosaic virus" (Odell et al. 1985), besonders geeignet sind solche Promotoren, welche pathogen-induzierbar sind (Bsp.: PR1-Promotor aus Petersilie (Rushton et al., 1996). Besonders geeignete Pathogeninduzierbare Promotoren sind synthetische bzw. chimäre Promotoren, welche in der Natur nicht vorkommen, aus mehreren Elementen zusammengesetzt sind und einen Minimalpromotor beinhalten sowie stromaufwärts des Minimalpromotors mindestens ein cis-regulatorisches Element aufweisen, welches als Bindungsstelle für spezielle Transkriptionsfaktoren dient. Chimäre Promotoren werden den gewünschten Anforderungen nach konzipiert und werden durch unterschiedliche Faktoren induziert oder reprimiert. Beispiele für solche Promotoren finden sich in WO 2000/29592 und WO 2007/147395. Ein geeigneter Terminator ist beispielsweise der nos-Terminator (Depicker et al., 1982).

Zusätzlich zu den oben beschriebenen Vektoren, stellt die vorliegende Erfindung auch ein Verfahren bereit, das die Einbringung eines beschriebenen Vektors in eine Wirtszelle umfasst. Der Vektor kann beispielsweise durch Konjugation, Mobilisation, biolistische Transformation, Agrobakterium-vermittelte Transformation, Transfektion, Transduktion, Vakuuminfiltration oder Elektroporation eingebracht werden. Solche Verfahren ebenso wie Verfahren zur Präparation von beschriebenen Vektoren sind dem Fachmann geläufig (Sambrook et al. 2001).

In einem weiteren Aspekt betrifft die vorliegende Erfindung eine Wirtszelle, welche das erfindungsgemäße Polynukleotid oder den Vektor der vorliegenden Erfindung umfasst. Eine Wirtszelle im Sinne der Erfindung kann eine prokaryotische (z.B. bakteriell) oder eukaryotische Zelle (z.B. eine pflanzliche Zelle oder eine Hefezelle) sein. Vorzugsweise ist die Wirtszelle ein Agrobakterium wie *Agrobacterium tumefaciens* oder *Agrobacterium rhizogenes* oder eine Pflanzenzelle, welche das erfindungsgemäße Polynukleotid oder den Vektor der vorliegenden Erfindung umfassen. Dem Fachmann sind sowohl zahlreiche Methoden wie Konjugation oder Elektroporation bekannt, mit denen er das erfindungsgemäße Polynukleotid oder den Vektor der vorliegenden Erfindung in ein Agrobakterium einbringen kann, als auch Methoden wie diverse Transformationsverfahren (biolistische Transformation, Agrobakterium-vermittelte Transformation), mit denen er das erfindungsgemäße Polynukleotid oder den Vektor der vorliegenden Erfindung in eine Pflanzenzelle einbringen kann (Sambrook et al. 2001).

In einem weiteren Aspekt betrifft die vorliegende Erfindung eine transgene Pflanzenzelle, welche das erfindungsgemäße Polynukleotid als Transgen oder den Vektor der vorliegenden Erfindung umfasst. Eine solche transgene Pflanzenzelle ist beispielsweise eine Pflanzenzelle, welche mit dem erfindungsgemäßen Polynukleotid oder mit dem Vektor der vorliegenden Erfindung, vorzugsweise stabil, transformiert ist. In einer bevorzugten Ausgestaltung der transgenen Pflanzenzelle ist, das Polynukleotid mit einer oder mehreren regulatorischen Sequenzen, welche die Transkription und optional die Expression in der Pflanzenzelle erlauben, operativ verknüpft. Das Gesamtkonstrukt aus dem erfindungsgemäßen Polynukleotid und der/den regulatorischen Sequenz(en) können dann das Transgen darstellen. Solche regulatorische Sequenzen sind beispielsweise ein Promoter oder ein Terminator. Dem Fachmann sind zahlreiche in Pflanzen anwendbare, funktionelle Promotoren und Terminatoren bekannt. Bevorzugt zeigt eine transgene Pflanzenzelle der vorliegenden Erfindung, insbesondere eine Zelle einer Pflanze der Spezies *Zea mays* gegenüber *H. turcicum* eine höhere Resistenz als eine korrespondierende nicht-transformierte Pflanzenzelle (die (isogene) Pflanzenzelle ohne das Transgen). Erfindungsgemäße transgene Ht-resistente Pflanzenzelle zeigen eine erhöhte Resistenz gegenüber *H. turcicum* von mindestens 1 Boniturnote, bevorzugt von mindestens 2 Boniturnoten oder mindestens 3 Boniturnoten und besonder bevorzugt von mindestens 4 Boniturnoten (siehe Boniturschema in Tabelle 3). Weiterhin betrifft die vorliegende Erfindung auch ein Verfahren zur Herstellung einer transgenen Pflanzenzelle der vorliegenden Erfindung, welches einen Schritt des Einbringens des erfindungsgemäßen Polynukleotids oder des Vektors der vorliegenden Erfindung in eine pflanzliche Zelle umfasst. Beispielsweise kann das Einbringen durch Transformieren stattfinden, vorzugsweise durch stabiles Transformieren. Die geeigneten Techniken zur Einbringung wie biolistische Transformation, Agrobakterium-vermittelte Transformation oder Elektroporation sind dem Fachmann bekannt (Sambrook et al. 2001).

In einem weiteren Aspekt betrifft die vorliegende Erfindung auch ein Verfahren zur Vermittlung oder Erhöhung einer Resistenz gegenüber *H. turcicum* in einer Pflanze, bevorzugt einer Pflanze der Spezies *Zea mays,* welche einen Schritt des Transformierens einer Pflanzenzelle mit dem erfindungsgemäßen Polynukleotid oder dem Vektor der vorliegenden Erfindung umfasst. Bevorzugt führt dieses Verfahren zu erhöhter Resistenz gegenüber *H. turcicum* von mindestens 1 Boniturnote, bevorzugt von mindestens 2 Boniturnoten oder mindestens 3 Boniturnoten und besonder bevorzugt von mindestens 4 Boniturnoten (siehe Boniturschema in Tabelle 3).

In einem zusätzlichen Aspekt schließt die vorliegende Erfindung auch ein Verfahren zur Modifikation des Resistenzphänotyps einer Pflanze, insbesondere einer Maispflanze, gegenüber dem Pathogen *Helminthosporium turcicum,* welches einen Schritt des Mutierens des Resistenz-vermittelnden Gens des HTN1-Locus aus Pepitilla oder eines Genallele davon umfasst, ein. Vorzugsweise kodiert das Resistenz-vermittelnde Gene des HTN1-Locus aus Pepitilla für ein Polypeptid gemäß SEQ ID NO: 2 oder ein Homolog eines Polypeptid gemäß SEQ ID NO: 2, welches unter Befallsbedingungen mit *H. turcicum* einen Resistenzphänotyp mit den HTN1-typischen Merkmalen bewirkt. Das Resistenz-vermittelnde Gen des HTN1-Locus aus Pepitilla oder eines Genallele davon kann transgen oder endogen im Genom der Pflanze vorliegen. Modifikation des Resistenzphänotyps kann eine Änderung der Pathogen-Rassenspezifität und/oder eine Änderung des Resistenzlevels gemessen als Boniturnote basierend auf phänotypische Kennzeichen wie beispielsweise betroffene Blattfläche (siehe Tabelle 3) oder gemessen als AUDPC-Wert (siehe Beispiel 1 C) meinen. Bevorzugt liegt das Resistenzlevel nach Modifikation des Resistenzphänotyps zwischen dem Resistenzlevel einer Pflanze, die das nicht-mutierte Resistenz-vermittelnde Gen des HTN1-Locus aus Pepitilla exprimiert, und dem Resistenzlevel einer isogenen Pflanze, die das Resistenz-vermittelnde Gen des HTN1-Locus aus Pepitilla nicht exprimiert; es kann aber auch über dem Resistenzlevel einer Pflanze, die das nicht-mutierte Resistenz-vermittelnde Gen des HTN1-Locus aus Pepitilla exprimiert, liegen. Besonders bevorzugt liegt das Resistenzlevel zwischen dem Resistenzlevel einer Pflanze, die das Polypeptid gemäß SEQ ID NO: 2 exprimiert, und dem Resistenzlevel einer isogenen Pflanze, die das Polypeptid gemäß SEQ ID NO: 2 nicht exprimiert; es kann aber auch über dem Resistenzlevel einer Pflanze, die das Polypeptid gemäß SEQ ID NO: 2 exprimiert, liegen. Der Ausdruck Mutieren kann hierbei als eine vom Menschen durchgeführte Änderung der genetischen Sequenz (Mutation) verstanden werden. Beispiele hierfür sind, dass Pflanzen, Pflanzenzellen oder Pflanzenteile einer hohen Dosis von chemischen, radiologischen oder anderen Mutagensien ausgesetzt werden und anschließend auf Mutanten selektiert wird. Alternativ kann das Mutieren auch durchgeführt werden zum Beispiel mit Hilfe von Tilling, TALE-Nukleasen, Zink-Finger-Nukleasen oder einem CRISPR/Cas System oder durch Fusion, Insertion, Deletion oder Austausche in der DNA-Sequenz oder der Aminosäuresequenz. Zur Durchführung des Schritts des Mutierens erhält der Fachmann aus bekannten Stands der Technik eine ausreichende technische Anleitung zum Handeln. Bevorzugt führt das Mutieren des Resistenz-vermittelnden Gens des HTN1-Locus aus Pepitilla zu mindestens einem Aminosäureaustausch, mindestens zwei Aminosäureaustauschen, mindestens drei Aminosäureaustauschen, mindestens fünf oder mehr Aminosäureaustauschen. Im Falle von mehreren Aminosäureaustauschen können diese auch auf unterschiedlichen Genallelen des Resistenz-vermittelnden Gens des HTN1-Locus aus Pepitilla vorliegen, d.h. die Mutation kann heterozygot aber auch homozygot vorliegen.

In einer bevorzugten Ausgestaltung des Verfahrens zur Modifikation des Resistenzphänotyps einer Pflanze führt das Mutieren des Resistenz-vermittelnden Gens des HTN1-Locus aus Pepitilla zu einer Punktmutation in der Nukleotidsequenz gemäß SEQ ID NO: 1 an der Position 1365 mit dem Basenaustausch eines G zu einem A oder an der Position 1490 mit dem Basenaustausch eines G zu einem A. Weiterhin betrifft diese Ausgestaltung auch das Mutieren, das zu einem Aminosäureaustausch in der Aminosäuresequenz gemäß SEQ ID NO: 2 an Position 455 von M (Methionin) zu I (Isoleucin) oder an Position 497 von G (Glycin) zu E (Glutaminsäure) führt. In einer weiteren bevorzugten Ausgestaltung des Verfahrens führt das Mutieren des Resistenz-vermittelnden Gens des HTN1-Locus aus Pepitilla zu einer Punktmutation, welche einen Aminosäureaustausch zur Folge hat, in der Nukleotidsequenz gemäß SEQ ID NO: 1 zwischen der Position 1365 und der Position 1490 oder die Ausgestaltung betrifft das Mutieren, das zu einem Aminosäureaustausch in der Aminosäuresequenz gemäß SEQ ID NO: 2 zwischen Position 455 und Position 497 führt.

In einem weiteren Aspekt betrifft die Erfindung ein Verfahren zur Herstellung einer Pflanze, insbesondere einer Maispflanze, mit einem modifizierten Resistenzphänotyp gegenüber dem Pathogen *Helminthosporium turcicum,* welches einen Schritt des Mutierens des Resistenz-vermittelnden Gens des HTN1-Locus aus Pepitilla oder eines Genallele davon in mindestens einer Zelle der Pflanze oder in mindestens einer Zelle, aus welcher die Pflanze regeneriert wird, umfasst. Weiterhin kann das Verfahren somit einen Schritt der Regeneration von mindestens einer Pflanze aus der mindestens einen mutierten Zelle und Selektion der regenerierten Pfalnezn auf Basis des modifizierten Resistenzphänotyps gegenüber dem *Pathogen Helminthosporium turcicum.* Vorzugsweise kodiert das Resistenz-vermittelnde Gen des HTN1-Locus aus Pepitilla für ein Polypeptid gemäß SEQ ID NO: 2 oder ein Homolog eines Polypeptid gemäß SEQ ID NO: 2, welches unter Befallsbedingungen mit *H. turcicum* einen Resistenzphänotyp mit den HTN1-typischen Merkmalen bewirkt. Das Resistenz-vermittelnde Gen des HTN1-Locus aus Pepitilla oder eines Genallele davon kann transgen oder endogen im Genom der Pflanze vorliegen. Ein modifizierter Resistenzphänotyp kann eine Änderung der Pathogen-Rassenspezifität und/oder eine Änderung des Resistenzlevels gemessen als Boniturnote basierend auf phänotypische Kennzeichen wie beispielsweise betroffene Blattfläche (siehe Tabelle 3) oder gemessen als AUDPC-Wert (siehe Beispiel 1 C) meinen. Bevorzugt liegt das Resistenzlevel des modifizierten Resistenzphänotyps zwischen dem Resistenzlevel einer Pflanze, die das nicht-mutierte Resistenz-vermittelnde Gen des HTN1-Locus aus Pepitilla exprimiert, und dem Resistenzlevel einer isogenen Pflanze, die das Resistenz-vermittelnde Gen des HTN1-Locus aus Pepitilla nicht exprimiert; es kann aber auch über dem Resistenzlevel einer Pflanze, die das nicht-mutierte Resistenz-vermittelnde Gen des HTN1-Locus aus Pepitilla exprimiert, liegen. Besonders bevorzugt liegt das Resistenzlevel zwischen dem Resistenzlevel einer Pflanze, die das Polypeptid gemäß SEQ ID NO: 2 exprimiert, und dem Resistenzlevel einer isogenen Pflanze, die das Polypeptid gemäß SEQ ID NO: 2 nicht exprimiert; es kann aber auch über dem Resistenzlevel einer Pflanze, die das Polypeptid gemäß SEQ ID NO: 2 exprimiert, liegen. Der Ausdruck Mutieren kann hierbei als eine vom Menschen durchgeführte Änderung der genetischen Sequenz (Mutation) verstanden werden. Beispiele hierfür sind, dass Pflanzen, Pflanzenzellen oder Pflanzenteile einer hohen Dosis von chemischen, radiologischen oder anderen Mutagensien ausgesetzt werden und anschließend auf Mutanten selektiert wird. Alternativ kann das Mutieren auch durchgeführt werden zum Beispiel mit Hilfe von Tilling, TALE-Nukleasen, Zink-Finger-Nukleasen oder einem CRISPR/Cas System oder durch Fusion, Insertion, Deletion oder Austausche in der DNA-Sequenz oder der Aminosäuresequenz. Zur Durchführung des Schritts des Mutierens erhält der Fachmann aus bekannten Stands der Technik eine ausreichende technische Anleitung zum Handeln. Bevorzugt führt das Mutieren des Resistenz-vermittelnden Gens des HTN1-Locus aus Pepitilla zu mindestens einem Aminosäureaustausch, mindestens zwei Aminosäureaustauschen, mindestens drei Aminosäureaustauschen, mindestens fünf oder mehr Aminosäureaustauschen. Im Falle von mehreren Aminosäureaustauschen können diese auch auf unterschiedlichen Genallelen des Resistenz-vermittelnden Gens des HTN1-Locus aus Pepitilla vorliegen, d.h. die Mutation kann heterozygot aber auch homozygot vorliegen.

In einer bevorzugten Ausgestaltung des Verfahrens zur Herstellung einer Pflanze mit einem modifizierten Resistenzphänotyp gegenüber dem Pathogen *Helminthosporium turcicum* führt das Mutieren des Resistenz-vermittelnden Gens des HTN1-Locus aus Pepitilla zu einer Punktmutation in der Nukleotidsequenz gemäß SEQ ID NO: 1 an der Position 1365 mit dem Basenaustausch eines G zu einem A oder an der Position 1490 mit dem Basenaustausch eines G zu einem A. Weiterhin betrifft diese Ausgestaltung auch das Mutieren, das zu einem Aminosäureaustausch in der Aminosäuresequenz gemäß SEQ ID NO: 2 an Position 455 von M (Methionin) zu I (Isoleucin) oder an Position 497 von G (Glycin) zu E (Glutaminsäure) führt. In einer weiteren bevorzugten Ausgestaltung des Verfahrens führt das Mutieren des Resistenz-vermittelnden Gens des HTN1-Locus aus Pepitilla zu einer Punktmutation, welche einen Aminosäureaustausch zur Folge hat, in der Nukleotidsequenz gemäß SEQ ID NO: 1 zwischen der Position 1365 und der Position 1490 oder die Ausgestaltung betrifft das Mutieren, das zu einem Aminosäureaustausch in der Aminosäuresequenz gemäß SEQ ID NO: 2 zwischen Position 455 und Position 497 führt.

Die Erfindung schließt ebenfalls Pflanzen oder einen Teil davon ein, welche durch ein Verfahren zur Herstellung einer Pflanze mit einem modifizierten Resistenzphänotyp gegenüber dem Pathogen *Helminthosporium turcicum* hergestellt werden können.

Die Erfindung erfasst ferner eine Pflanze oder einen Teil davon, welche eine Mutation in dem Resistenz-vermittelnden Gen des HTN1-Locus aus Pepitilla oder eines Genallele davon aufweist. Vorzugsweise führt die Muation zu einem modifizierten Resistenzphänotyp wie oben beschrieben. Vorzugsweise kodiert das Resistenz-vermittelnde Gen des HTN1-Locus aus Pepitilla für ein Polypeptid gemäß SEQ ID NO: 2 oder ein Homolog eines Polypeptid gemäß SEQ ID NO: 2, welches unter Befallsbedingungen mit *H. turcicum* einen Resistenzphänotyp mit den HTN1-typischen Merkmalen bewirkt. Das Resistenz-vermittelnde Gen des HTN1-Locus aus Pepitilla oder eines Genallele davon kann transgen oder endogen im Genom der Pflanze vorliegen. In einer bevorzugten Ausgestaltung der Pflanze oder des Teils davon ist die Mutation eine Punktmutation in der Nukleotidsequenz gemäß SEQ ID NO: 1 an der Position 1365 mit dem Basenaustausch eines G zu einem A oder an der Position 1490 mit dem Basenaustausch eines G zu einem A. Weiterhin betrifft diese Ausgestaltung auch eine Mutation, die zu einem Aminosäureaustausch in der Aminosäuresequenz gemäß SEQ ID NO: 2 an Position 455 von M (Methionin) zu I (Isoleucin) oder an Position 497 von G (Glycin) zu E (Glutaminsäure) führt. In einer weiteren bevorzugten Ausgestaltung der Pflanze oder des Teils davon ist die Mutation des Resistenz-vermittelnden Gens des HTN1-Locus aus Pepitilla eine Punktmutation, welche einen Aminosäureaustausch zur Folge hat, in der Nukleotidsequenz gemäß SEQ ID NO: 1 zwischen der Position 1365 und der Position 1490 oder die Ausgestaltung betrifft eine Mutation, die zu einem Aminosäureaustausch in der Aminosäuresequenz gemäß SEQ ID NO: 2 zwischen Position 455 und Position 497 führt.

Einige der in dieser Anmeldung verwendeten Begriffe werden nachfolgend näher erläutert:
Der Begriff "Allel" betrifft eine von zwei oder mehr Nukleotidsequenzen an einem bestimmten Locus im Genom. Ein erstes Allel findet sich auf einem Chromosom, ein zweites auf einem zweiten Chromosom an derselben Position. Unterscheiden sich die beiden Allele liegen diese heterozygot vor, sind es dieselben Allele liegen sie homozygot vor. Verschiedene Allele eines Gens (Genallele) unterscheiden sich in mindestens einem SNP. Je nach Kontext der Beschreibung meint ein Allel auch nur ein einzelnes SNP, das beispielsweise eine Unterscheidung zwischen Donor von HTN1 (Pepitilla) und rekurrentem Elter erlaubt.

Der Ausdruck "Chromosomfragment" ist ein spezifischer chromosomaler DNA-Abschnitt eines bestimmten Chromosoms, der mindestens ein Gen umfasst. Ein integriertes Chromosomfragment stammt aus einer Donor-Quelle. Im Sinne der Erfindung kann die sequenzielle Abfolge der Gene innerhalb eines integrierten Chromosomfragments derjenigen Abfolge entsprechen, wie sie im ursprünglichen Chromosomfragment der Donor-Quelle vorliegt. Somit kann das integrierte Chromosomfragment über die gesamte Länge unverändert zum entsprechenden Chromosomfragment in der Donor-Quelle vorliegen. Ein Chromosomfragment oder ein Teil davon kann einen spezifischen "Haplotyp" darstellen, wobei das Chromosomfragment dann bestimmte SNPs aufweist, durch die der Haplotyp auch eindeutig spezifiziert ist und identifiziert werden kann.

Die Begriffe "distal" und "proximal" bezeichnen die Position einer chromosomalen Intervalls bzw. eines genetischen Abschnitts in Relation zu einem bestimmten Bezugsort (beispielsweise einem bestimmten Polynukleotid, einem anderen chromosomalen Intervall oder einem Gen) auf einem Gesamtchromosom, wobei distal bedeutet, dass das Intervall oder der Abschnitt auf der dem Chromosom-Centromer abgewandten Seite des Bezugsortes lokalisiert ist, und proximal bedeutet, dass das Intervall oder der Abschnitt auf der dem Chromosom-Centromer zugewandten Seite des Bezugsortes lokalisiert ist.

"Eng gekoppelt" oder "eng verknüpft" meint zwei Loci, zwei intervalle, zwei genetische Abschnitte oder zwei Marker (Markerloci), welche weniger als 15 cM, weniger als 12 cM, welche weniger als 10 cM, welche weniger als 8 cM, welche weniger als 7 cM, welche weniger als 6 cM, welche weniger als 5 cM, welche weniger als 4 cM, welche weniger als 3 cM, welche weniger als 2 cM, welche weniger als 1 cM, welche weniger als 0,5 cM, welche weniger als 0,2 cM, welche weniger als 0,1 cM, festgelegt durch die IBM2 neighbors 4 genetic map öffentlich zugänglich auf der Maize GDB website, voneinander entfernt sind.

Der Begriff "Ertrag" im Sinne der vorliegenden Erfindung betrifft die Produktivität pro Flächeneinheit eines bestimmten Pflanzenproduktes mit kommerziellem Wert. Beispielsweise wird der Ertrag von Mais gewöhnlich gemessen in metrischen Tonnen Samen oder Korn pro Hektar (ha) und Saison oder in metrischen Tonnen Trockenbiomasse pro Hektar (ha) und Saison. Sofern nicht ausdrücklich anders angegeben oder spezifiziert, kann der Ertrag die absolute Frisch- oder Trockenmasse, die relative Frisch- oder Trockenmasse, den Silageertrag (auch Silomaisertrag oder Total Dry Matter Yield genannt) oder den Körnerertrag betreffen. Der Ertrag wird durch genetische und umweltbedingte Faktoren beeinflusst und setzt sich prinzipiell aus zahlreichen agronomischen Eigenschaften zusammen, die auf genetischen Elementen beruhende Merkmale einer Pflanze darstellen und im Verlauf der Saison zum letztendlichen Ertrag beitragen. Zu diesen individuellen agronomischen Eigenschaften gehören beispielsweise Saataufgang, vegetative Vitalität, Stresstoleranz, Krankheitsresistenz oder -toleranz, Herbizidresistenz, Verzweigungsneigung, Blühzeitpunkt, Samenansatz, Samendichte, Standfestigkeit und Lagerneigung, Druschfähigkeit (gleichmäßige Abreife), etc.

Der Ausdruck "genetischer Abschnitt mit" einem näher spezifizierten Intervall ist zu verstehen als ein genetischer Abschnitt, welcher das näher spezifizierte Intervall einschließt oder umfasst, d.h. nicht beschränkt ist auf das näher spezifizierte Intervall. Zum Beispiel meint ein genetischer Abschnitt mit dem fünften Intervall zwischen einem Marker in der achten Markerregion M8, welche durch die Marker MA0022 und MA0013 flankiert ist, und einem Marker in der sechsten Markerregion M6, welche durch die Marker PZE-108107671 und SYN4196 flankiert ist, dass der genetische Abschnitt das fünfte Intervall umfasst und der genetische Abschnitt zwischen einem Marker in der achten Markerregion M8, welche durch die Marker MA0022 und MA0013 flankiert ist, und einem Marker in der sechsten Markerregion M6, welche durch die Marker PZE-108107671 und SYN4196 flankiert ist, lokalisiert ist.

Unter "Hybridisieren" oder "Hybridisierung" wird ein Vorgang verstanden, bei dem sich ein einzelsträngiges Nukleinsäuremolekül an einen weitestgehend komplementären Nukleinsäurestrang anlagert, d.h. mit diesem Basenpaarungen eingeht. Standardverfahren zur Hybridisierung sind beispielsweise in Sambrook et al. 2001 beschrieben. Vorzugsweise wird darunter verstanden, dass mindestens 60%, weiter bevorzugt mindestens 65%, 70%, 75%, 80% oder 85%, besonders bevorzugt 90%, 91%, 92%, 93%, 94%, 95%, 96%, 97%, 98% oder 99% der Basen des Nukleinsäuremoleküls eine Basenpaarung mit dem weitestgehend komplementären Nukleinsäurestrang eingehen. Die Möglichkeit einer solchen Anlagerung hängt von der Stringenz der Hybridisierungsbedingungen ab. Der Begriff "Stringenz" bezieht sich auf die Hybridisierungsbedingungen. Hohe Stringenz ist dann gegeben, wenn eine Basenpaarung erschwert ist, niedrige Stringenz, wenn eine Basenpaarung erleichtert ist. Die Stringenz der Hybridisierungsbedingungen hängt beispielsweise von der Salzkonzentration bzw. lonenstärke und der Temperatur ab. Generell kann die Stringenz durch eine Erhöhung der Temperatur und/oder eine Erniedrigung des Salzgehaltes erhöht werden. Unter "stringenten Hybridisierungsbedingungen" sind solche Bedingungen zu verstehen, bei denen eine Hybridisierung überwiegend nur zwischen homologen Nukleinsäuremolekülen stattfindet. Der Begriff "Hybridisierungsbedingungen" bezieht sich dabei nicht nur auf die bei der eigentlichen Anlagerung der Nukleinsäuren herrschenden Bedingungen, sondern auch auf die bei den anschließenden Waschschritten herrschenden Bedingungen. Stringente Hybridisierungsbedingungen sind beispielsweise Bedingungen, unter denen überwiegend nur solche Nukleinsäuremoleküle hybridisieren, die mindestens 70%, vorzugsweise mindestens 75%, mindestens 80%, mindestens 85%, mindestens 90% oder mindestens 95% Sequenzidentität aufweisen. Stringente Hybridisierungsbedingungen sind beispielsweise: Hybridisieren in 4 x SSC bei 65 °C und anschließendes mehrfaches Waschen in 0,1 × SSC bei 65 °C für insgesamt etwa 1 Stunde. Der hier verwendete Begriff "stringente Hybridisierungsbedingungen" kann auch bedeuten: Hybridisieren bei 68 °C in 0,25 M Natriumphosphat, pH 7,2, 7 % SDS, 1 mM EDTA und 1 % BSA für 16 Stunden und anschließendes zweimaliges Waschen mit 2 x SSC und 0,1 % SDS bei 68 °C. Bevorzugt findet eine Hybridisierung unter stringenten Bedingungen statt.

Der Begriff "Intervall" oder "chromosomales Intervall" meint einen durchgängigen linearen Abschnitt auf einer genomischen DNA, welcher in einem einzelnen Chromosom in planta oder auf einem Chromosomfragment vorliegt und welcher gewöhnlicherweise durch die Angabe von zwei Markern, welche die Endpunkte des Intervalls zur distalen und proximalen Seite hin darstellen, definiert ist. Dabei können die Marker, die das Intervall endständig definieren selbst auch Teil des Intervalls sein. Weiterhin können zwei unterschiedliche Intervalle auch überlappen. In der Beschreibung wird ein Intervall durch die Angabe "zwischen Marker A und Marker B" spezifiziert. Ein endständiger Marker eines Intervalls kann auch in einer definierten Markerregion zu einer Seite des Intervalls lokalisiert sein. Eine Markerregion wird dann durch die Angabe von zwei flankierenden Markern definiert und stellt einen chromosomalen Abschnitt dar, auf dem neben den flankierenden Markern noch weitere Marker liegen können. Flankierende Marker bestimmen die Endpunkte einer Markerregion und sind selbst noch Teil der Markerregion. Sind beide endständigen Marker eines Intervalls Marker in unterschiedlichen Markerregionen zu beiden Seiten eines Intervalls, wird in der Beschreibung ein Intervall durch die Angabe "zwischen einem Marker in einer Markerregion X, welche durch die Marker C und D flankiert ist, und einem Marker in einer Markerregion Y, welche durch die Marker E und F flankiert ist" spezifiziert. Eine Markerregion kann sich über bis zu 500.000 Baasenpaare (bp) erstrecken, kann bevorzugt zwischen 100.000 und 400.000 bp groß sein oder kann besonders bevorzugt zwischen 140.000 und 315.000 bp groß sein.

Unter "Introgression" versteht man im Zusammenhang mit der vorliegenden Erfindung die Übertragung von mindestens einem gewünschten Genallel auf einem genetischen Locus von einem genetischen Hintergrund in einen anderen. Beispielsweise kann eine Introgression eines gewünschten Genallels an einen spezifischen Locus auf einen Nachkommen durch sexuelle Kreuzung zwischen zwei Eltern derselben Spezies übertragen werden. Alternativ kann zum Beispiel die Übertragung eines Genallels auch durch Rekombination zwischen zwei Donorgenomen in einem fusionierten Protoplasten auftreten, wobei mindestens ein Donor-Protoplast das gewünschte Genallel in seinem Genom trägt. In jedem Fall können die Nachkommen, die dann das gewünschte Genallel umfassen, anschließend wiederholt mit einer Linie, die einen vorzüglichen genetischen Hintergrund aufweist, rückgekreuzt und auf das gewünschte Genallel selektiert werden. Das Ergebnis ist eine Fixierung des gewünschten Genallels in einem ausgewählten genetischen Hintergrund.

Unter einem "isolierten Nukleinsäuremolekül" oder "isolierten Polynukleotid" wird ein aus seiner natürlichen bzw. ursprünglichen Umgebung herausgelöstes Nukleinsäuremolekül oder Polynukleotid verstanden. Der Begriff umfasst auch ein synthetisch hergestelltes Nukleinsäuremolekül. Unter einem "isolierten Polypeptid" wird ein aus seiner natürlichen bzw. ursprünglichen Umgebung herausgelöstes Polypeptid verstanden. Der Begriff umfasst auch ein synthetisch hergestelltes Polypeptid.

Unter einer "Pathogeninfektion" ist der früheste Zeitpunkt zu verstehen, zu dem ein Pathogen mit einem pflanzlichen Wirtsgewebe interagiert. Beispielsweise gehören bei Pilzen wie Ascomyceten oder bei Oomyceten das Auswachsen von Hyphen oder die Bildung von spezifischen Infektionsstrukturen wie Penetrationshyphen und Appressorien. Im Detail kann die Infektion durch *Helminthosporium turcicum* bereits mittels diverser Färbetechniken (z.B. Trypan-Blau) untersucht (Chung et al., BMC Plant Biology 10 (2010), 103; Walsh et al. (2008), Posterpräsentation P192, 50th Maize Genetics Conference in Washington D.C.).

"Donor Pepitilla", "Akzession Pepitilla" oder "Pepitilla" meint neben der Landrasse Pepitilla selbst auch andere Maisgenotypen, in deren Genom, insbesondere auf Chromosom 8 bin 5 oder 6, eine Introgression des *HTN1*-Resistenzlocus bevorzugt aus Pepitilla inseriert wurde. Solche sind beispielsweise W22Htn (z.B. Bar-Zur et al.,1998), H6314Htn (z.B. Bar-Zur et al.,1998), B73*HtN* (z.B. Shimoni et al., Journal of Phytopathology 131:4 (1991), 315-321), B68*HtN* und A632*HtN* (z.B. Carson, Plant Disease 79 (1995), 717-720) und A619*HtN* (z.B. Stanković et al, Genetika 39:2 (2007), 227 -240). Weiterhin schließt Pepitilla jede Resistenzquelle, welche den Resistenzphänotyp mit den HTN1-typischen Merkmalen nach Introgression in eine anfällige Maislinie/Maispflanze vermitteln. Zu diesen HTN1-typischen Merkmalen gehören beispielsweise verzögertes Einsetzen der Sporulation, verminderte Entwicklung von Läsionen, Entwicklung von kleineren Läsionen, reduzierte Sporulationszonen und/oder keine oder nur vereinzelte chlorotisch-nekrotische Läsionen.

Ein "Locus" ist eine Position auf einem Chromosom, wo sich ein oder mehrere Gene befinden, welche ein agronomisches Merkmal verursachen oder beeinflussen. Insbesondere meint Locus hier den HTN1-Resistenzlocus, welcher Resistenz gegenüber den Pathogen *Helminthosporium turcicum* bzw. mindestens gegenüber eine Rasse von *Helminthosporium turcicum* vermittelt.

Eine "Maispflanze" ist eine Pflanze der Spezies *Zea mays* sowie deren Subspezies wie beispielsweise *Zea mays* ssp. *mays, Zea mays* ssp. *mexicana* oder *Zea mays* ssp. *parviglumis* verstanden.

Ein "Marker" ist eine Nukleotidsequenz, die als Bezugs- oder Orientierungspunkt verwendet wird. Ein Marker zum Erkennen eines Rekombinationsereignisses sollte geeignet sein, Unterschiede oder Polymorphismen innerhalb einer pflanzlichen Population zu überwachen. Für Marker finden sich diese Unterschiede auf DNA-Ebene und sind beispielsweise Polynukleotidsequenzunterschiede wie zum Beispiel SSRs (simple sequence repeats), RFLPs (restriction fragment length polymorphisms), FLPs (fragment length polymorphisms) oder SNPs (single nucleotide polymorphisms). Die Marker können von genomischen oder exprimierten Nukleinsäuren wie beispielsweise gespleißter RNA, cDNA oder ESTs abgeleitet sein und können sich auch auf Nukleinsäuren beziehen, die als Sonden oder Primer-Paare eingesetzt werden und als solche geeignet sind, ein Sequenzfragment unter Verwendung PCR-basierter Verfahren zu amplifizieren. Marker, welche genetische Polymorphismen zwischen Teilen einer Population betreffen, können mittels etablierter Verfahren aus dem Stand der Technik nachgewiesen werden (An Introduction to Genetic Analysis. 7th Edition, Griffiths, Miller, Suzuki et al., 2000). Dazu gehören z.B.: DNA-Sequenzierung, PCR-basierte, sequenzspezifische Amplifikation, Nachweis von RFLPs, Nachweis von Polynukleotidpolymorphismen mittels Allelspezifischer Hybridisierung (ASH), Detektion von SSRs, SNPs oder AFLPs. Darüber hinaus sind auch Verfahren zur Detektion von ESTs (expressed sequence tags) und RAPD (randomly amplified polymorphic DNA) bekannt. Je nach Kontext kann der Begriff Marker in der Beschreibung auch eine spezifische Chromosomposition in dem Genom einer Spezies, wo ein spezifischer Marker (z.B. SNP) gefunden werden kann, meinen. Ein solche Marker-Position kann genutzt werden, um die Anwesenheit eines gekoppelten Locus zu verfolgen, z.B. ein gekoppelter Locus, der zur Expression eines bestimmten phänotypischen Merkmals beiträgt (z.B. HTN1 oder Linkage Drag). Beispielsweise kann der Marker-Locus auch genutzt werden, um die Segregation von Allelen an einem Locus (QTL oder Einzelgen), die genetisch oder physikalisch eng gekoppelt sind mit dem Marker-Position, zu beobachten.

"Operativ verknüpft" meint verbunden in einem gemeinsamen Nukleinsäuremolekül, in einer Weise, dass die verbundenen Elemente derart zueinander positioniert und orientiert sind, dass eine Transkription des Nukleinsäuremolekül stattfinden kann. Eine DNA, welche operativ mit einem Promotor verknüpft ist, steht unter der transkriptionellen Kontrolle dieses Promotors.

Pflanzliche "Organe" meinen beispielsweise Blätter, Sprossachse, Stamm, Wurzeln, vegetative Knospen, Meristeme, Embryos, Antheren, Ovula oder Früchte. Pflanzliche "Teile" meinen einen Zusammenschluss mehrerer Organe, z.B. eine Blüte oder ein Samen, oder einen Teil eines Organs, z.B. einen Querschnitt aus der Sprossachse. Pflanzliche "Gewebe" sind zum Beispiel Kallusgewebe, Speichergewebe, meristematische Gewebe, Blattgewebe, Sprossgewebe, Wurzelgewebe, Pflanzentumorgewebe oder reproduktives Gewebe. Unter pflanzlichen "Zellen" sind beispielsweise isolierte Zellen mit einer Zellwand oder Aggregate davon oder Protoplasten zu verstehen.

Eine "Pflanze" im Sinne der Erfindung kann, sofern nicht anders angegeben von jeder Spezies aus den dikotyledonen, monokotyledonen und gymnospermen Pflanzen sein. Vorzugsweise sind Pflanzen monokotyl und sind in Agrikultur oder Hortikultur oder zur Erzeugung von Bioenergie (Bioethanol, Biogas etc.) interessant. Hierzu zählen beispielhaft *Gossypium* sp., *Zea mays, Brachypodium distachyon, Triticum* sp., *Hordeum vulgare, Oryza sativa, Sorghum* sp., *Musa* sp., *Saccharum officinarum, Secale cereale, Avena* sp., Rasen- und Futtergras. Eine erfindungsgemäße Pflanze ist vorzugsweise eine Pflanze der Gattung *Zea,* insbesondere der Spezies *Zea mays,* oder *Sorghum.*

Im Zusammenhang mit der vorliegenden Erfindung betrifft der Begriff "regulatorische Sequenz" eine Nukleotidsequenz, welche die Spezifität und/oder die Expressionsstärke beeinflusst, zum Beispiel indem die regulatorische Sequenz eine bestimmte Gewebespezifität vermittelt. Eine solche regulatorische Sequenz kann stromaufwärts des Transkriptionsinitiationspunkts eines Minimalpromotors, aber auch stromabwärts davon wie beispielsweise in einer transkribierten aber nicht translatierten Leader-Sequenz oder innerhalb eines Introns lokalisiert sein.

Der Ausdruck "Resistenz" oder "resistent" gegenüber einem Pathogen ist als die Widerstandsfähigkeit einer Pflanze oder pflanzlichen Zelle gegenüber die schädlichen Einflüsse des Pathogens zu verstehen und erstreckt sich von einer Verzögerung in der Krankheitsentwicklung bis hin zu einer kompletten Unterdrückung der Krankheitsentwicklung. Im Zusammenhang mit der vorliegenden Erfindung ist eine Pflanze/Pflanzenzelle resistent oder besitzt eine Pflanze/Pflanzenzelle eine Resistenz gegenüber dem Pathogen *Helminthosporium turcicum (H. turcicum* oder *Ht),* d.h. somit gegenüber der Blattkrankheit Northern Corn Leaf Blight (NCLB). Die Resistenz wird vermittelt durch ein oder mehrere Proteine, die durch ein Gen oder durch Gene (resistenzvermittelnden Gene) aus der Akzession Pepitilla kodiert werden. Die Resistenz kann vollständig oder partiell sein und kann Pathogenrassen-spezifisch oder nicht-Pathogenrassen-spezifisch ausgeprägt sein. Im Fall einer Pathogenrassen-spezifischen Resistenz können zu den virulenten Rassen von *Helminthosporium turcicum* beispielsweise N, 1N, 2N, 23N oder 123N, zu den avirulenten Rassen beispielsweise 0, 1, 2, 3, 12, 23 oder 123 gehören. Eine vermittelte Resistenz kann eine neu-erworbene Resistenz sein oder die Erhöhung einer bereits existierenden partiellen Resistenz.

Eine "Transgene Pflanze" bezieht sich auf einen Pflanze, in dessen Genom mindestens ein Polynukleotid, vorzugsweise ein heterologes Polynukleotid, integriert ist. Bevorzugt ist das Polynukleotid stabil integriert, was bedeutet, dass das integrierte Polynukleotid in der Pflanze stabil erhalten bleibt, exprimiert wird und auch stabil an die Nachkommen vererbt werden kann. Das stabile Einbringen eines Polynukleotids in das Genom einer Pflanze schließt auch die Integration in das Genom einer Pflanze der vorhergehenden Parentalgeneration mit ein, wobei das Polynukleotid stabil weitervererbt werden kann. Der Begriff "heterolog" bedeutet, dass das eingebrachte Polynukleotid beispielsweise von einer Zelle oder einem Organismus mit einem anderen genetischen Hintergrund derselben Spezies oder einer anderen Spezies stammt, oder homolog ist zu der prokaryotischen oder eukaryotischen Wirtszelle, dann aber in einer unterschiedlichen genetischen Umgebung lokalisiert ist und sich so von einem eventuell natürlicherweise vorhandenen korrespondierenden Polynukleotid unterscheidet. Ein heterologes Polynukleotid kann zusätzlich zu einem entsprechenden endogenen Gen vorhanden sein.

Nachfolgend ist eine Zusammenfassung der Erfindung aufgeführt.

Ein erster Aspekt der Erfindung bezieht sich auf eine Maispflanze, in deren Genom ein Chromosomfragment aus dem Donor Pepitilla integriert ist, wobei das Chromosomfragment ein Intervall des Donors umfasst, welches Donor-Allele gemäß dem Haplotyp nach Tabelle 2 aufzeigt und ein Polynukleotid aufweist, das in der Maispflanze Resistenz gegenüber *Helminthosporium turcicum* vermittelt, und wobei das Chromosomfragment
a) ein Intervall des Donors zwischen einem Marker in einer ersten Markerregion, welche durch die Marker SYN14136 und PZE-108076510 flankiert ist, und einem Marker in einer zweiten Markerregion, welche durch die Marker SYN24931 und PZE-108077560 flankiert ist, und/oder
b) ein Intervall des Donors zwischen einem Marker in einer dritten Markerregion, welche durch die Marker PZE-108093423 und PZE-108093748 flankiert ist, und einem Marker in einer vierten Markerregion, welche durch die Marker MA0004 und MA0005 flankiert ist, und/oder
c) ein Intervall des Donors zwischen einem Marker in einer fünften Markerregion, welche durch die Marker MA0006 und PZE-108097482 flankiert ist, und einem Marker in einer sechsten Markerregion, welche durch die Marker PZE-108107671 und SYN4196 flankiert ist,
nicht enthält.

Eine bevorzugte Ausführungsform bezieht sich auf eine erfindungsgemäße Maispflanze, wobei das Chromosomfragment weiterhin ein Intervall des Donors zwischen einem Marker in der zweiten Markerregion und einem Marker in der dritten Markerregion nicht enthält.

Eine weitere bevorzugte Ausführungsform bezieht sich auf eine erfindungsgemäße Maispflanze, wobei der Blühzeitpunkt der Maispflanze und/oder der Silageertrag der Maispflanze demjenigen einer Vergleichsmaispflanze, in deren Genom das Chromosomfragment aus dem Donor Pepitilla nicht integriert ist, entspricht.

Eine ebenfalls bevorzugte Ausführungsform bezieht sich auf eine erfindungsgemäße Maispflanze, wobei das Polynukleotid ein Nukleinsäuremolekül umfasst,
(a) das eine Nukleotidsequenz gemäß SEQ ID NO: 1, 3, 5, 7, 9, 11, 13.oder 15 aufweist,
(b) das eine Nukleotidsequenz mit einer Identität von mindestens 80% zu einer der Nukleotidsequenzen gemäß SEQ ID NO: 1, 3, 5, 7, 9, 11, 13, oder 15 vorzugsweise über die gesamte Sequenzlänge, aufweist,
(c) das mit dem komplementären Strang eines Nukleinsäuremoleküls nach (a) oder (b) unter stringenten Bedingungen hybridisiert,
(d) das ein Polypeptid mit einer Aminosäuresequenz gemäß SEQ ID NO: 2, 4, 6, 8, 10, 12, 14 oder 16 kodiert,
(e) das ein Polypeptid mit einer Aminosäuresequenz, die zu mindestens 80% identisch ist mit einer der Aminosäuresequenzen nach (d), kodiert, oder
(f) das eine Teilsequenz einer Nukleinsäure nach (a) bis (e) aufweist.

Ein weiterer Aspekt der vorliegenden Erfindung bezieht sich auf eine Zelle, Gewebe oder Teil der erfindungsgemäßen Maispflanze.

Noch ein weiterer Aspekt bezieht sich auf ein Korn oder Samen einer erfindungsgemäßen Maispflanze.

Ebenfalls ein weiterer Aspekt der vorliegenden Erfindung bezieht sich auf ein Verfahren zur Identifizierung einer *Helminthosporium turcicum* resistenten Maispflanze, in deren Genom ein Chromosomfragment aus dem Donor Pepitilla integriert ist, umfassend den Nachweis von mindestens zwei Allelen im Genom der Pflanze, wobei mindestens ein Allel in einem genomischen Abschnitt lokalisiert ist, welcher durch einen Marker in der ersten Markerregion, der zweiten Markerregion, der dritten Markerregion oder der vierten Markerregion und durch das Polynukleotid; das in der Maispflanze Resistenz gegenüber *Helminthosporium turcicum* vermittelt, flankiert ist, und wobei mindestens ein Allel in einem genomischen Abschnitt lokalisiert ist, welcher durch das Polynukleotid und durch einen Marker in der sechsten Markerregion oder der fünften Markerregion flankiert ist.

Ein weiterer Aspekt der vorliegenden Erfindung bezieht sich auch ein Verfahren zur Erhöhung des Ertrags einer *H. turcicum* resistenten Maispflanze, in deren Genom ein Chromosomfragment aus dem Donor Pepitilla integriert ist, wobei das Chromosomfragment ein Intervall des Denars umfasst, welches Donor-Allele gemäß dem Haplotyp nach Tabelle 2 aufzeigt und ein Polynukleotid aufweist, das in der Maispflanze Resistenz gegenüber *Helminthosporium turcicum* vermittelt, umfassend den Schritt der Entfernung
a) eines Intervalls des Denars zwischen einem Marker in einer ersten Markerregion, welche durch die Marker SYN14136 und PZE-108076510 flankiert ist, und einem Marker in einer zweiten Markerregion, welche durch die Marker SYN24931 und PZE-108077560 flankiert ist, und/oder
b) eines Intervalls des Donors zwischen einem Marker in einer dritten Markerregion, welche durch die Marker PZE-108093423 und PZE-108093748 flankiert ist, und einem Marker in einer vierten Markerregion, welche.durch die Marker MA0004 und MA0005 flankiert ist, und/oder
c) eines Intervalls des Donors zwischen einem Marker in einer fünften Markerregion, welche durch die Marker MA0006 und PZE-108097482 flankiert ist, und einem Marker in einer sechsten Markerregion, welche durch die Marker PZE-108107671 und SYN4196 flankiert ist.

Ebenfalls ein weiterer Aspekt der Erfindung bezieht sich auf ein Oligonukleotid, das ein Nukleinsäuremolekül mit einer Nukleotidsequenz, ausgewählt aus einer der SEQ ID NOs: 41-49, 53-100 oder 229-250, umfasst.

Noch ein weiterer Aspekt der Erfindung bezieht sich auf die Verwendung eines Oligonukleotids, das ein Nukleinsäuremolekül mit einer Nukleotidsequenz, ausgewählt aus einer der SEQ ID NOs: 17-250, umfasst, zur Identifizierung einer *Helminthosporium turcicum-resistenten* Maispflanze.

Ein Aspekt der Erfindung bezieht sich auf ein Polynukleotid, das ein Nukleinsäuremolekül umfasst,
(a) das eine Nukleotidsequenz gemäß SEQ ID NO: 1, 3, 5, 7, 9, 11, 13 oder 15 aufweist,
(b) eine Nukleotidsequenz mit einer Identität von mindestens 80% zu einer der · Nukleotidsequenzen gemäß SEQ ID NO: 1, 3, 5, 7; 9, 11, 13 oder 15 vorzugsweise über die gesamte Sequenzlänge, aufweist,
(c) das mit dem komplementären Strang eines Nukleinsäuremoleküls nach (a) oder (b) unter stringenten Bedingungen hybridisiert,
(d) das ein Polypeptid mit einer Aminosäuresequenz gemäß SEQ ID NO: 2, 4, 6, 8, 10, 12, 14 oder 16 kodiert, oder
(e) das ein Polypeptid mit einer Aminosäuresequenz, die zu mindestens 80% identisch ist mit einer der Aminosäuresequenzen nach (d), kodiert.

Ein weiterer Aspekt der Erfindung bezieht sich auf einen Vektor oder ein mobiles genetisches Element, umfassend ein erfindungsgemäßes Polynukleotid.

Ebenfalls ein weiterer Aspekt der Erfindung bezieht sich auf eine transgene Pflanzenzelle, umfassend ein erfindungsgemäßes Polynukleotid als Transgen oder einen erfindungsgemäßen Vektor oder mobiles genetisches Element.

Noch ein weiterer Aspekt der Erfindung bezieht sich auf eine transgene Pflanze, umfassend ein erfindungsgemäßes Polynukleotid oder Transgen oder einen erfindungsgemäßen Vektor oder mobiles genetisches Element.

Ebenfalls ein weiterer Aspekt der Erfindung bezieht sich auf ein Korn oder Samen der erfindungsgemäßen transgenen Pflanze, wobei das Korn oder der Samen ein erfindungsgemäßes Polynukleotid als Transgen umfasst.

Ausgestaltungen und Ausführungsformen der vorliegenden Erfindung werden in exemplarischer Weise mit Bezug auf die angehängten Figuren und Sequenzen beschrieben:
Figur 1: Errechneter QTL-Bereich von 23,1 cM auf Chromosom 8 mittels 8 Markern in 528 F2-Individuen der Kreuzung RP1 × RP1HTN1. Der schwarze Balken (HtN) zeigt das *confidence-*Intervall an. Positions-Angaben der Marker in cM.
Figur 2: Silageertrag-Versuch über 5 Standorten in Deutschland und in zwei Wiederholungen mit dem rekurrenten Elter RP3 und der A-Version des Donorfragments aus B37HTN1 (RP3HTNA) und der K-Version des Donorfragments aus B37HTN1 (RP3HTNK). Balken geben signifikante Unterschiede gemäß t-Test mit p=0,05 wieder.
Figur 3: Beschreibung der Markerregionen M1 bis M6, welche die chromosomalen Intervalle (Int.1 bis Int.5) definieren, die in den Introgressionslinien das Resistenz-vermittelnde Polynukleotid aufweisen und im vom Donor-stammenden Chromosomfragment den Linkage Drag tragen. Chromosomale Abschnitte des Donor 'Pepitilla' sind gepunktet, solche des rekurrenten Elter (ohne Linkage Drag) sind diagonal gestreift dargestellt. Intervall 1 (Int.1) umfasst den Resistenzlocus HTN1, Intervall 2 (Int.2) umfasst Sequenzbereiche, welche im Donor für den Linkage Drag des Blühzeitpunkts verantwortlich sind, Intervalle 4 und 5 (Int.4 und Int.5) umfassen Sequenzbereiche, welche im Donor für den Linkage Drag des Silageertrages verantwortlich sind.
Figur 4: BAC-contig auf seiner RP4HTN1 BAC-Bank mit entsprechendem Sequenz scaffold und Gen-Annotationen. Kandidatengene sind als karierte Kästchen wiedergegeben.

Schwarze Pfeile stellen weitere annotierte Gene dar, welche keine Kandidatengene der HTN-Resistenz sind.

### 1. Phänotypisierungexperimente

### A) Durchführung von Feld-Versuchen zur Bestimmung der HT-Resistenz unter natürlicher und künstlicher Beimpfung/Infektion und des Blühzeitpunkts:

An einem Standort wurden pro zu untersuchenden Mais-Genotyp jeweils mindestens 20 Individuen in einer Reihe ausgepflanzt. Die Inokulation erfolgte natürlich oder künstlich. Die natürliche Beimpfung/Infektion erfolgt durch natürlich auftretende Sporen von *H. turcicum.* Die künstliche Beimpfung/Infektion erfolgte mittels infiziertem und gemahlenem Blattmaterial, welches auf die zu untersuchenden Pflanzen gegeben wurde. Letztere Art der Inokulation ermöglichte die Simulation eines vergleichbaren *H*. *turcicum*-Befalls in unterschiedlichen Versuchsjahren und an unterschiedlichen Standorten unabhängig von den jeweils vorherrschenden natürlichen Befallsbedingungen. Als Kontroll-Genotypen wurden entsprechend der untersuchten Kreuzungspopulation ein anfälliger Elter und ein Elter mit HtN1-Introgression aus dem Donor B37HTN1 kultiviert. Die Bonitur des Merkmals HT-Resistenz fand zu mindestens drei Zeitpunkten während der Vegetationsperiode statt. Das einheitlich verwendete Boniturschema ist in Tabelle 3 wiedergegeben.

Der Donor B37HTN1 als Quelle der HT-Resistenz wurde in verschiedene genetische Hintergründe aus Elite-Linien mit unterschiedlichen stark ausgeprägten Anfälligkeit gegenüber *H. turcicum* eingekreuzt und nahisogene Linien entwickelt, welche sich von den anfälligen Ausgangslinien im Wesentlichen nur durch die Introgression aus B37HTN1 unterscheiden. In Phänotypisierungsexperimenten wurden nach künstlicher Inokulation wie oben beschrieben solche Linien selektiert, welche durch Einbringen der Resistenz-vermittelnden Introgression aus B37HTN1 eine Verbesserung der HT-Resistenz um mindestens 2 bis 3 Boniturnoten, bevorzugt von 3 bis 4 Boniturnoten aufzeigten. Beispielhaft wird die vorliegende Erfindung im Weiteren an den beiden selektierten rekurrenten Eltern RP1 und RP3 näher beschrieben. Die Ergebnisse der genannten Phänotypisierungsexperimente sind in Tabelle 5 zusammengefasst. Der rekurrente Elter RP1 ohne Introgression zeigte durchschnittliche Boniturnoten von 7 bis 9, die sich durch die Introgression aus B37HTN1 um 3 bis 4 Noten verbessert wurden. Der rekurrente Elter RP3 zeigte Boniturnoten zwischen 4 und 6 ohne Introgression und eine Verbesserung um 2 bis 3 Boniturnoten durch die Introgression. Der rekurrente Elter RP4 zeigte eine Boniturnoten von 6 ohne Introgression und eine Verbesserung um 2-3 Boniturnoten durch die Introgression.

**Tabelle 5: Phänotypisierungsdaten zur HT-Resistenz aus Genotypen RP1, RP3, und RP4 mit und ohne Resistenz-vermittelnder Introgression aus B37HTN1 (Boniturnoten wurden gemäß Schema aus Tabelle 3 bestimmt).**

| Genotyp | Durchschnittliche Boniturnoten (n=20) ohne Introgression aus B37HTN1 | Verbesserung der HT-Resistenz mit Introgression aus B37HTN1 |
|---|---|---|
| RP1 | 7 bis 9 | 3 bis 4 |
| RP3 | 4 bis 6 | 2 bis 3 |
| RP4 | 6 | 2 bis 3 |

Neben der HT-Resistenz wurde zudem für jeden Genotyp der Zeitpunkt der weiblichen und männlichen Blüte in Tagen nach Aussaat erfasst. Der Zeitpunkt der weiblichen Blüte ist durch das Auftreten von Narbenfäden, der männlichen Blüte durch das Auftreten der Rispe festgelegt. Die Ergebnisse sind unter Beispiel 3. B) näher ausgeführt.

### B) Durchführung von Feld-Versuchen zur Bestimmung des Korn- und Silage-Ertrags:

Neben vorstehenden Daten zu HT-Resistenz und Blühzeitpunkt wurden Ertragsdaten für RP3, enthaltend unterschiedlich lange Resistenz-vermittelnde Introgressionsfragmente aus B37HTN1 bzw. Pepitilla, und für eine vergleichende Elite-Linie ermittelt. Die Linien RP3, RP3HTNA und RP3HTNK wurden mit einem Tester (Flintmais, interpool Einfachkreuzung) des komplementären Genpools (Flintmais) bestäubt, um Saatgut für Testhybriden zu erzeugen. Diese Testhybriden wurden jeweils zweifach in einem Feldversuch an fünf für den Maisanbau repräsentativen Standorten in Deutschland angebaut. Die Testhybriden sind an diese Anbauregion in Bezug auf die Reife gut angepasst. Der Feldversuch wurde in zwei Wiederholungen in 4-reihigen Parzellen mit 6 m Länge und 0,75 m Reihenabstand durchgeführt. Die Bestandsdichte betrug 9 Pflanzen je m² in der ersten und 11 Pflanzen je m² in der zweiten Wiederholung. Zum Zeitpunkt der Silomaisreife wurden nur die beiden mittleren Reihen jeder Parzelle geerntet, um Konkurrenzeffekte zu minimieren. Am Erntegut wurde das Gewicht je Parzelle und der Wassergehalt bestimmt, um den Silomaisertrag (auch Silageertrag oder Total Dry Matter Yield) und den Trockensubstanzgehalt (Total Dry Matter Content) zu berechnen.

### C) Durchführung von Gewächshaus-Versuchen zur Bestimmung der HT-Resistenz:

Es wurden 20 Individuen pro Genotyp in Töpfen angezogen. Als Kontrollen dienten je nach Kreuzung Genotypen eines anfälligen Elters und eines nah-isogenen Elters (NIL) mit Resistenz-vermittelnder Introgression aus B37HTN1. 14 Tagen nach Aussaat erfolgte eine künstliche Infektion (siehe oben). Nach weiteren 2 bis 3 Wochen entwickelten sich erste Krankheitssymptome. Ab dem Zeitpunkt des Auftretens erster Symptome wurden alle zwei Tage die Bonituren des Merkmals HT-Resistenz sowie die Anzahl der Pflanzen mit Symptomen erfasst. Daraus wurde dann der AUDPC (*area under disease progress curve*) bestimmt. Die Befallshäufigkeit (in % / Zeit x Zeitraum) wurde für die Einteilung der untersuchten Pflanzen verwendet, wobei AUDPC von 0 - 100 resistent, 101 - 450 heterozygot, und > 450 anfällig entspricht.

### 2. Markerentwicklung für die HTN1-Target-Region

Neben den Boniturversuchen wurde die Target-Region um den HTN1-Resistenzlocus auf Chromosom 8 (bin 8.06) in zahlreichen Genotypen mittels neuer und/oder optimierter molekularer Marker genauer untersucht und feinkartiert. Hierzu eingesetzte molekulare Marker wurden auf Grund von Einzel-Nukleotid-Polymorphismen (SNP) oder bereits öffentlich verfügbaren *simple sequence repeat* Marker (SSR) entwickelt:
Die DNA aus den Genotypen zur Anwendung der Marker wurde entweder mittels der Methode NucleoSpin 96 Plant II nach Herstellerangaben (MACHEREY-NAGEL GmbH & Co. KG, Deutschland) oder mittels der Methode Klear Gene DNA Extraction 384 (LGC Genomics GmbH, Deutschland) isoliert.

Die Primersequenzen der SSR-Marker waren bereits aus der öffentlichen Datenbank des National Center for Biotechnology Information (NCBI) unter http://www.ncbi.nlm.nih.gov/unists bekannt; die Primersequenzen der Marker bnlg1782, umc1960, bnlg240, umc1121, bnlg1067 und umc1287, welche zur Untersuchung der Targetregion verwendet wurden, zusammen mit den vorgenommenen Modifikationen sind in Tabelle 6 zusammengefasst.

**Tabelle 6: Primer Sequenzen der SSR Marker (NED: 2'-chloro-5'-fluoro-7',8'-fused phenyl-1.4-dichloro-6-carboxyfluorescein; FAM: 6-carboxyfluorescein; M13: Kernsequenz der Phage M13)**

| Marker | Forward Primer Sequenz (5'-3') | Modifikation | Reverse Primer Sequenz (5'-3') | Modifikation | Zusätzlicher Primer + Modifikation |
|---|---|---|---|---|---|
| | [SEQ ID NO] | | [SEQ ID NO] | | |
| bnlg1782 | 113 | NED | 114 | keine | |
| umc1960 | 115 | NED | 116 | keine | |
| bnlg240 | 117 | FAM | 118 | keine | |
| umc1121 | 119 | FAM | 120 | keine | |
| bnIg1067 | 121 | FAM | 122 | keine | |
| umc1287 | 123 | keine | 124 | keine | M13 + FAM |

Der PCR-Reaktions-Mix von bnlg1782, umc1960, bn!g240, umc1121 und bnlg1067 umfasste 10 µl und bestand aus einer Einfach-Konzentration des 4x PufferB (Solis BioDyne, Estland), 0,5 pmol des forward Primers, 0,5 pmol des reversen Primers, 10-30 ng DNA, 0,25 Units HotFirepol TAQ-Polymerase (Solis BioDyne, Estland). Der Reaktionsmix von umc1287 umfasste 10 µl und bestand aus einer Einfach-Konzentration des 4x PufferB (Solis BioDyne, Estland), 0,5 pmol des forward Primers, 2,5 pmol des reversen Primers, 0,3 pmol des zusätzlichen Primers M13, 10-30 ng DNA, 0,25 Units HotFirepol TAQ-Polymerase (Solis BioDyne, Estland).

Die PCR-Reaktion wurde mit einer anfänglichen Denaturierungszeit von 900 Sekunden bei 94 °C, einem Amplifikations-Zyklus von 25-40 Zyklen mit 15 Sekunden 94 °C, 30 Sekunden 50-55 °C und 120 Sekunden bei 72°C, und einem abschließenden Schritt für 300 Sekunden bei 72°C durchgeführt. Im Anschluss erfolgte eine 2 h Inkubation der PCR-Reaktion bei 65 °C. Die Auftrennung der PCR-Produkte erfolgte auf einem ABI3730xl (Life Technologies^{™}, USA) nach den Angaben des Herstellers für die Auftrennung von Fragmenten von 50-400 bp.

Die SNP Marker wurden entweder (a) aus öffentlich verfügbaren Ressourcen, (b) aus einer vergleichenden Amplicon-Sequenzierung oder (c) aus einem Sequenz-Vergleich von BAC-Sequenzen aus RP4HTN1 (siehe Abschnitt Molekulare Analyse) und B73 Referenz-Genom AGPv02 (www.maizesequence.org) entwickelt und verwendet.
(a) Aus der öffentlich verfügbare SNP Ressource des Mais Community 50K-Illumina-Chip (Ganal et al., 2011) wurden SNPs in KASP-Marker (LGC Genomics GmbH, Deutschland) umgewandelt. Dazu wurden neue Primer entwickelt, die im KASP-Marker-Assay die Amplifikation der entscheidenden Allele gewährleisteten (siehe Tabelle 4). Der gesamte Arbeitsablauf wurde mit der Kraken^{™} Software (LGC Genomics GmbH, Deutschland) durchgeführt. Für einen KASP-Marker-Assay wurden 5-20 ng DNA, 0,02 µl eines Oligo-Assay-Mixes (12 µM Primer Allele 1 (forward); 12 µM Primer Allele 2 (forward); 30 µM reverse Primer) und 1,5 µl eines 1xKASPar Reagent Kits für 1536-Platten verwendet. Ein Standard-PCR-Setup bestand aus 94°C für 15 min, 10 Zyklen mit 94°C für 20 Sekunden, 61-55°C touch down für 1 Minute, 26 Zyklen mit 94°C für 20 Sekunden und 55°C für 1 Minute. Die Auswertung der Allele pro Genotyp wurde mittels der Kraken^{™} Software (LGC Genomics GmbH, Deutschland) durchgeführt.
(b) Die vergleichende Amplicon-Sequenzierung wurde mittels Sanger-Sequenzierung durchgeführt. Die Genotypen in der vergleichenden Sequenzierung umfassten jeweils den Donor B37HTN1, sowie B37, RP1, RP1HTN1, RP3, RP3HTN1 (Versionen A, B, K), RP4, RP4HTN1. Die DNA wurde aus vermahlenen Körnern mittels der CTAB-Methode (Maniatis et al., 1982) isoliert. Die Primersequenzen für die Amplicon-Sequenzierung sind in Tabelle 4 aufgelistet. Ein Standard-PCR-Protokoll für die Amplifikation der entsprechenden Bereiche bestand aus einer Denaturierung bei 94°C für 5 Minuten, 35 Zyklen mit jeweils 94°C für 1 Minute, 60°C für 1 Minute und 72°C für 2 Minuten und einem abschließendem Schritt bei 72°C für 10 Minuten. Die PCR-Produkte wurden mit der Sanger-Methode (Sanger & Coulson, 1975) sequenziert. Die Sequenzauswertung erfolgte mit der DNAStar Lasergene Software (DNASTAR Inc., USA). Die detektierten Polymorphismen wurden wie unter (a) beschrieben in KASP-Marker umgewandelt.
(c) Die BAC-Sequenz-Contigs wurden mittels Blast-Algorithmus (http://blast.ncbi.nlm.nih.gov/Blast.cgi) gegen das B73 Referenz-Genom AGPv02 projiziert um Einzel-Nukleotid-Polymorphismen (SNP) zu detektieren. Die Polymorphismen wurden mit der Lasergene Software (DNASTAR Inc., USA) detektiert und sind zusammen mit den flankierenden Sequenzen in Tabelle 4 aufgeführt. Zu den flankierenden Sequenzen eines SNPs wurden Primer entwickelt und die identifizierten SNPs wie unter (a) beschrieben in KASP-Marker umgewandelt.

### 3. Lokalisierung des HTN1-Resistenzlocus auf Chromosom 8 durch Verwendung der SSR-Marker

### A) Lokalisierung des HTN1-Resistenzlocus:

Der HTN1-Resistenzlocus aus den Donoren B37HTN1 wurde wie unter Beispiel 1.A) beschrieben in Elite-Linien eingekreuzt und mit Hilfe der SSR und SNP Marker aus Beispiel 2. auf Chromosom 8 (bin 8.06) lokalisiert (siehe Figur 1). NILs der Kreuzungen RP1 × RP1HTN1 und RP3 × RP3HTN1 wurden an zwei Standorten über mehrere Jahre mit zwei Wiederholungen unter natürlichen Infektionsbedingungen gemäß Boniturschema nach Tabelle 3 phänotypisiert. Die NILs zeigten im Mittel eine um 4 Boniturnoten verbesserte Resistenzantwort im Vergleich zur Originallinie. Die Entwicklung der lokalen Läsionen auf den Blättern war um etwa 2 Wochen im Vergleich zum anfälligen Genotyp verschoben. Eine QTL-Kartierung wurde mit 528 F2 Individuen (Kreuzung RP1 x RP1HTN1) unter Verwendung von den 8 Markern (aus Figur 1 QTL Kartierung - Marker sind Tabellen 4 und 6 aufgeführt) durchgeführt. Der QTL-Bereich, welcher den HTN1-Resistenzlocus umfasst, wurde auf Chromosom 8 zwischen den Markern MA0002 und umc1287 in einem Bereich von 23,1 cM lokalisiert.

### B) Einkreuzen des B37HTN1 Donor Fragmentes in eine Elite-Maislinien und Identifikation und Eliminierung des Linkage Drags für verspäteten Blühzeitpunkt:

Der Donor B37HTN1 wurde mit KWS.elite, einer Elite-Maislinie der KWS SAAT AG (Deutschland) gekreuzt und über fünf Generationen fortgesetzt mit KWS.elite rückgekreuzt. In jeder Rückkreuzungsgeneration wurden molekulare Marker genutzt um Pflanzen zu selektieren, die heterozygot für die HTN-Zielregion waren. Anschließend wurde eine selektierte Pflanze der fünften Rückkreuzungsgeneration fortgesetzt geselbstet und mit molekularen Markern wurden homozygote Pflanzen für die HTN-Zielregion identifiziert.

Diese Linien wurden in Feldversuchen an mehreren Standorten getestet. Dabei wurden für die Genotypen B37HTN1, KWS.elite und KWS.elite.B37HTN1 wie unter Beispiel 1. beschrieben die phänotypischen Daten der HT-Resistenz und der Blühzeitpunkte erfasst. Die Genotypen mit HTN1-Introgression zeigten die erwartete HT-Resistenz mit Boniturnoten von 1 bis 3, während die Ausgangs-Linie KWS.elite Boniturnoten von 5-7 erhielt. Unerwarteterweise wies die KWS.elite.B37HTN1 zudem im Vergleich zu KWS.elite einen um mindestens 2 Tage verschobenen Zeitpunkt sowohl der weiblichen als auch der männlichen Blüte auf. Diese verschobenen Blühzeitpunkte stellen ein auf Linkage Drag beruhendes negatives agronomisches Merkmal für Mais dar, welches in dieser Form nach Introgression der HT-Resistenz aus B37HTN1 noch nicht beschreiben wurde. Marker-Analysen ermöglichten die Lokalisierung des Linkage Drags, welcher für den verzögerten Blühzeitpunkt verantwortlich ist, in einem Bereich zwischen zwei Markerregionen auf der Introgression aus B37HTN1, zwischen M1 und M2. Dafür wurden die Genotypen B37HTN1, KWS.elite und KWS.elite.B37HTN1 beispielsweise mit den KASP-Markern SYN14136, PZE-108076510, SYN24931 und PZE-108077560 analysiert (siehe Figur 3 und Tabelle 4). SYN14136 und PZE-108076510 wurden zur spezifischen Detektion der Markerregion M1, SYN24931 und PZE-108077560 zur spezifischen Detektion des Region M2 herangezogen. Danach liegt die Markerregion M1 5' vom Locus des Linkage Drags und die Markerregion M2 3' dazu. Die Marker-Analyse zeigte, dass B37HTN1 und KWS.elite.B37HTN1, beide mit einer um zwei Tage späteren Blüte, gemeinsame Allele für die Regionen M1 und M2 sowie dem Intervall zwischen diesen Regionen zeigten, während KWS.elite mit einem normalen Blühzeitpunkt andere Allele für die Regionen M1 und M2 und dem Intervall dazwischen aufwiesen.

Der Donor B37HTN1 wurde mit RP3 gekreuzt und über drei Generationen fortgesetzt mit RP3 rückgekreuzt. In jeder Rückkreuzungsgeneration wurden molekulare Marker genutzt. Zunächst wurden Pflanzen, die heterozygot für die HTN1-Zielregion waren selektiert und anschließend wurden diese Pflanzen mit gleichmäßig über das Genom verteilten Markern untersucht, um gegen das Donorgenom zu selektieren. Anschließend wurde eine selektierte Pflanze der dritten Rückkreuzungsgeneration fortgesetzt geselbstet und mit molekularen Markern wurden homozygote Pflanzen für die HTN1-Zielregion identifiziert.

Weiterhin wurde der Donor B37HTN1 auch mit den rekurrenten Elter RP3 und RP4 gekreuzt und eine Linie RP3HTNA und RP4HTNA über mehrere Schritte der Rückkreuzung erzeugt. Die Phänotypisierung auf HT-Resistenz zeigte eine Verbesserung der Boniturnoten von 5 bis 7 für die Ausgangslinie RP3 auf 1 bis 3 für RP3HTNA und eine Verbesserung der Boniturnoten von 6 für die Ausgangslinie RP4 auf 2 bis 3 für RP4HTNA. Die Phänotypisierung auf Blühzeitpunkte zeigte vergleichbare Blühzeitpunkte für RP3 und RP3HTNA und RP4 und RP4HTNA. Durch Verwendung der KASP-Marker SYN14136, PZE-108076510, SYN24931 und PZE-108077560 konnte nachgewiesen werden, dass RP3 und RP3HTNA gemeinsame Allele für die Regionen M1 und M2 tragen. Diese entsprachen nicht dem Donor B37HTN1. Im Ergebnis liegt somit der Blühzeitpunkt-verzögernde chromosomale Abschnitt der Introgression aus B37HTN1 auf einem chromosomalen Intervall zwischen den Markerregionen M1 und M2. Mit der Linie RP3HTNA war es somit gelungen diesen Linkage Drag zu entfernen. Die verwendeten KASP-Marker SYN14136, PZE-108076510, SYN24931 und PZE-108077560 stellten sich als wirksames Werkzeuge zur "assistierten Selektion" dar.

Die Phänotypisierung der RP3 und RP3HTNA umfasste zudem auch die Erfassung des Korn- und des Silageertrags. Während der Kornertrag in den Genotypen nicht signifikant unterschiedlich war, zeigte das Merkmal Silageertrag bei RP3HTNA eine eindeutige statistisch belegbare Reduktion um mindestens 14 Dezitonnen pro Hektar (dt/ha) gegenüber RP3, oder eine Reduktion um mehr als 5%.

Mit Hilfe der entwickelten KASP-Marker SYN14136, PZE-108076510, SYN24931 und PZE-108077560 konnten aus der Kreuzung von B37HTN1 und dem rekurrenten Elter RP1 eine Linie RP1HTN1 selektiert werden, die keinen Blühzeitverzögernden Linkage Drag mehr aufwies, aber ebenfalls eine Silageertragsreduktion, wie sie für RP3HTNA beobachtet wurde, zeigte. Zur verfeinerten molekularen Charakterisierung wurde RP1HTN1 weiterentwickelt und eine F2-Population mit 724 Individuen aus der Kreuzung RP1 x RP1HTN1 erstellt. Anschließend wurde die F3-Generation geselbstet und selektierte F4-Pflanzen wurden geno- und phänotypisiert. Die Genotypisierung wurde mittels Marker der Tabelle 6 im detektierten QTL-Bereich von 23,1 cM durchgeführt. Die Phänotypisierung wurde an mehreren Standorten in zwei Wiederholungen durchgeführt (siehe Beispiel 1.). Es wurden rekombinante Pflanzen für den QTL-Bereich selektiert und mit den phänotypischen Daten korreliert. Die Selektion umfasste Pflanzen, die unterschiedliche Bereiche der Targetregion abdeckten, sowie heterozygote Pflanzen, mit dem Ziel neue rekombinante Pflanzen zu erhalten. Es wurden in jedem Jahr zwei Rückkreuzungen mit RP1 und selektierten Einzelpflanzen durchgeführt und somit neue Rekombinante erstellt. Neue Rekombinante wurden im Feld- und Gewächshausversuchen phänotypisiert (siehe unter 1.) und für die Entwicklung neuer molekularer Marker gemäß 2. genotypisiert.

Die Anwendung dieser neuen Marker auf den Genotyp RP3HTNA ermöglichte die Identifizierung einer Markerregion M3, welche im 5'-Bereich die Introgression begrenzt und mit den flankierenden Markern PZE-108093423 und PZE-108093748 beschrieben werden kann. Dabei sollte PZE-108093423 das Allele des rekurrenten Elter RP3 und PZE-108093748 das Allele des Donors B37HTN1 aufweisen. Im 3'-Bereich wird die Introgression von RP3HTNA von den Markern PZE-108107671 und SYN4196 in einer weiteren Markerregion M6 beschrieben (siehe Figur 3). Dabei trägt PZE-108107671 das Allele des Donors B37HTN1 und SYN4196 das Allele des rekurrenten Elter RP3. Die Introgression aus RP3HTNA (nachfolgend A-Version genannt) entspricht zwischen den Markerregionen M3 und M6 dem Donor B37HTN1, aber außerhalb dieses Bereichs dem rekurrenten Elter, oder einer anderen Linie, die nicht die Allele im Bereich zwischen M1 und M2 des Donors B37HTN1 trägt. Diese A-Version wurde in verschiedene andere genetische Hintergründe eingebracht und erneut Ertragsprüfungen, Resistenz-Phänotypisierungen und Blühzeitpunkt-Bestimmung unterzogen. Die Ergebnisse waren vergleichbar mit denjenigen beschrieben für RP3HTNA. So war der Blühzeitpunkt im Vergleich zur entsprechenden Linie ohne Introgression nicht verschoben und die Linie zeigte eine verbesserte Resistenz gegenüber *Helminthosporium turcicum,* im Vergleich mit der Ausgangslinie, allerdings weiterhin die Reduktion des Silageertrags.

### C) Identifikation und Eliminierung des Linkage Drags für reduzierten Silageertrag:

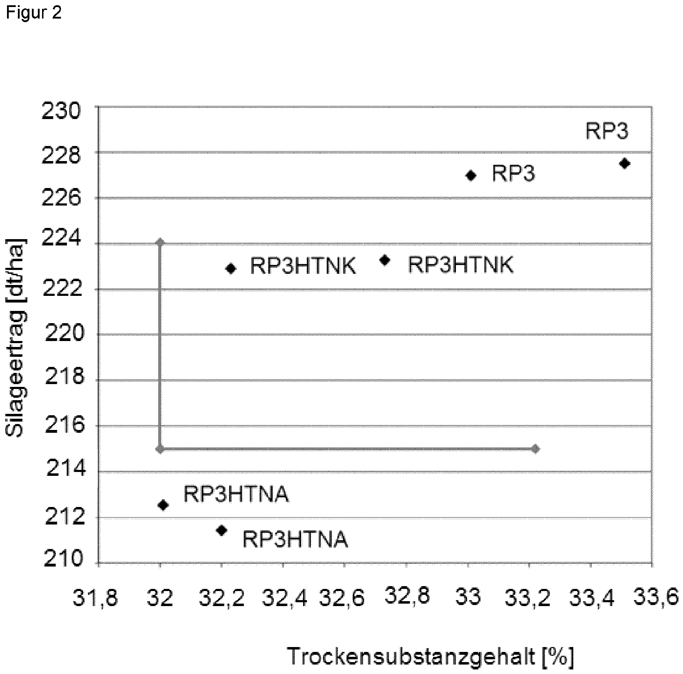

Als Donor wurde die Linie RP3HTNA verwendet. Diese wurde mit RP3 gekreuzt und über sechs Generationen fortgesetzt geselbstet. In jeder Selbstungsgeneration wurden molekulare Marker im Zielbereich genutzt, um das Donorfragment zu verkleinern. Da alle Regionen des Genoms außerhalb der Zielregion bereits in der Linie RP3HTNA auf RP3 Genom selektiert worden waren, wurde mit Markern nur noch der Bereich um die HTN-Zielregion untersucht. Dabei wurden homozygote Pflanzen für eine verkleinerte HTN-Zielregion identifiziert. Parallel dazu fand eine intensive Markerentwicklung im Target-Bereich statt. Neben vielen anderen gelang es eine Linie RP3HTNK zu identifizieren, die das B37HTN1-Donor-Fragment von einer Markerregion M4, flankiert durch die Marker MA0004 und MA0005, wobei MA0004 das Allele des rekurrenten Elter RP3 und MA0005 das Allele des Donors B37HTN1 in RP3HTNK beschreibt, bis zu einer Markerregion M5, flankiert durch die Marker MA0006 und PZE-108097482, wobei MA0006 das Allele des Donors B37HTN1 und PZE-108097482 das Allele des rekurrenten Elter RP3 beschreibt, trägt. Die Introgression aus RP3HTNK (nachfolgend K-Version genannt) bewirkt in RP3HTNK eine verbesserte HTN1-Resistenz um 3 bis 4 Boniturnoten im Vergleich zu RP3, einen gleichen Blühzeitpunkt wie ihre Ausgangslinie RP3 (keine Verzögerung in der Blüte) und keine signifikante Ertragsreduktion des Silage-Ertrags (siehe Figur 2) mehr. Mit Hilfe der beschriebenen Marker konnten auch aus der Ausgangslinie RP1 durch Kreuzung Linkage Drag-freie Linien erstellt werden, welche die K-Version der Introgression aufwiesen.

### D) Resistenz-vermittelnder Haplotyp aus B37HTN1 bzw. aus Pepitilla

Die K-Version besitzt einen Haplotyp aus B37HTN1 bzw. Pepitilla, der die in Tabelle 4 beschriebenen Donor-Allele an den physischen Positionen bezogen auf B73 AGPv02 in bp trägt. Exemplarisch sei die Haplotypbeschreibung hier an Marker MA0008 beschrieben: Verwendet man den Marker MA0008 und bestimmt die Allele für B37HTN1, RP3, RP3HTNA, RP3HTNK so wird das Allele "T" für B37HTN1, RP3HTNA, RP3HTNK bestimmt und das Allele "C" für RP3. Dieser Marker unterscheidet für diesen Locus ebenfalls die mutmaßliche HTN1-Resistenzquelle PH99N (WO 2011/163590), die an dieser Position ebenfalls ein Allel "C" enthält, von der für hier verwendeten Resistenzquelle.

### 4. Molekulare Analyse der fein-kartierten Region

Weiterhin wurde das durch Introgression inserierte und trunkierte Chromosomfragment molekular untersucht. Der Resistenzlocus *Htn1* aus der Akzession Pepitilla wurde so auf eine distinkte Zielregion, ein chromosomales Intervall von 700 kb, reduziert und in dem Genotyp RP4HTN1 sequenziert. Wie im Folgenden näher ausgeführt wurden BAC Klone aus RP4HTN1 isoliert, sequenziert und zu einem Sequenz Scaffold assembliert. Der Sequenz scaffold wurde annotiert und den annotierten Genen aus diesem Intervall wurden EST/cDNA-Sequenzinformationen gegenübergestellt. Aus einer Vielzahl von annotierten Genen wurden dann durch differentielle Expressionsstudien die Kandidaten-Gene identifiziert (siehe Tabelle 1).

### A) BAC Bank- und Pool-Erstellung, BAC Bank Sichtung, BAC Sequenzierung

Eine BAC Bank wurde aus dem Genotyp RP4HTN1 erstellt. Es erfolgte die Erstellung der BAC Bank und der 3D-Matrix-Pools aus Blattmaterial sowie die Sichtung der 3D-Matrix-Pools. Die Primer für die Sichtung der 3D-Matrix-Pools wurden auf Grund der B73 AGPv01 Sequenz von 149957158 bp bis 152977351 bp auf Chromosom 8 (www.maizesequence.org) und dem Programm Primer 3 (http://simgene.com/Primer3; Rozen & Skaletsky, 2000). Die Parameter für die Primerauswahl waren ein durchschnittlicher GC-Gehalt von 50%, Primerlänge von 20-25 bp, Schmelztemperatur zwischen 70-90°C und Amplicon Länge zwischen 70-80 bp. Mit den Primerpaaren in Tabelle 7 wurden die 3D-Pools mittels RT-PCR gesichtet. Die Werte der beiden Parameter Schmelz-Temperatur und CP-Wert sind für die BAC-Klone angegeben. Es konnten 26 BAC Klone für den ausgewählten Bereich identifiziert werden. Alle BAC Klone wurden aus der BAC Bank isoliert und als E.coli-Kultur zur DNA Isolation und Sequenzierung genutzt. Die Sequenzierung wurde mit einem Standard GS-FLX Titanium Laufs (454 Life Sciences, USA) durchgeführt. Die erhaltene Sequenzinformation der BAC Klone 144N24, 119F13, 219G11, 86N21, 16B06, 84L18, 128D02, 25M23, 96H10, 19J24, 136A01, 75H06, 135F07 ist in Tabelle 8 zusammengestellt.

**Tabelle 7: Primerpaare zur Detektion von BAC-Klonen aus der RP4HTN-BAC-Bank**

| BAC Klon ID | Primerpaar 1 | Sequenz Primerpaar 1 (5'-3') | Schmelz-Temp °C (50% des Amplicons ist einzelsträngig) im Genotyp RP4HTN1 | CP-Value (Cyclus, wenn die exponentielle Phase der PCR beginnt) | Amplicon Größe (bp) |
|---|---|---|---|---|---|
| | Primerpaar 2 | | | | |
| 58A14 | 579ZMPM0_2F; 579ZMPM0_2R | 125; 126 | 77,4 | 28,5 | 74 |
| | 579ZMPM0_4F; 579ZMPM0_4R | 127; 128 | 80,96 | 26,52 | 77 |
| 144N24 | 579ZMPM0_5F; 579ZMPM0_5R | 129; 130 | 79,09 | 27,09 | 76 |
| | 579ZMPM0_17F; 579ZMPM0_17R | 131; 132 | 83,06 | 25,53 | 78 |
| 219G11 | 579ZMPM0_16F; 579ZMPM0_16R | 133; 134 | 84,7 | 25,96 | 78 |
| | 579ZMPM0_25F; 579ZMPM0_25R | 135; 136 | 78,95 | 26,09 | 80 |
| 119F13 | 579ZMPM0_22F; 579ZMPM0_22R | 137; 138 | 80,89 | 25,98 | 73 |
| | 579ZMPM0_34F; 579ZMPM0_34R | 139; 140 | 80,1 | 24,43 | 76 |
| 86N21 | 579ZMPM0_35F; 579ZMPM0_35R | 141; 142 | 80,9 | 25,27 | 70 |
| | 579ZMPM0_38F; 579ZMPM0_38R | 143; 144 | 83,86 | 26,01 | 71 |
| 16B6 | 579ZMPM0_37F; 579ZMPM0_37R | 145; 146 | 79,22 | 25,71 | 80 |
| | 579ZMPM0_41F; 579ZMPM0_41R | 147; 148 | 75,93 | 26,6 | 74 |
| 84L18 | 579ZMPM0_41F; 579ZMPM0_41R | 149; 150 | 75,93 | 26,6 | 74 |
| | 579ZMPM0_46F; 579ZMPM0_46R | 151; 152 | 80,54 | 25,68 | 78 |
| 128D2 | 579ZMPM0_180F; 579ZMPM0_180R2 | 153, 154 | 84,41 | 25,99 | 77 |
| | 579ZMPM0_48F; 579ZMPM0_48R | 155; 156 | 83,96 | 25,33 | 77 |
| 25M23 | 579ZMPM0_48F; 579ZMPM0_48R | 157; 158 | 83,96 | 25,33 | 77 |
| | 579ZMPM0_56F; 579ZMPM0_56R | 159; 160 | 77 | 29,12 | 79 |
| 19J24 | 579ZMPM0_51F; 579ZMPM0_51R | 161; 162 | 87,76 | 27,75 | 77 |
| | 579ZMPM0_199F; 579ZMPM0_199R | 163; 164 | 82,49 | 26,56 | 79 |
| 96H10 | 579ZMPM0_63F; 579ZMPM0_63R | 165; 166 | 85,78 | 26,08 | 63 |
| | 579ZMPM0_208F; 579ZMPM0_208R | 167; 168 | 79,87 | 26,84 | 79 |
| 136A1 | 579ZMPM0_206F; 579ZMPM0_206R | 169; 170 | 89,81 | 32,09 | 70 |
| | 579ZMPM0_86F; 579ZMPM0_86R | 171; 172 | 81,81 | 30,07 | 71 |
| 135F7 | 579ZMPM0_79F; 579ZMPM0_79R | 173; 174 | 75,82 | 25,43 | 72 |
| | 579ZMPM0_278F; 579ZMPM0_278R | 175; 176 | 78,13 | 22,69 | 78 |
| 75H6 | 579ZMPM0_209F; 579ZMPM0_209R | 177; 178 | 75,41 | 24,93 | 77 |
| | 579ZMPM0_86F; 579ZMPM0_86R | 179; 180 | 81,81 | 30,07 | 71 |
| 11702 | 579ZMPM0_87F; 579ZMPM0_87R | 181; 182 | 81,89 | 27,7 | 76 |
| | 579ZMPM0_91F; 579ZMPM0_91R | 183; 184 | 80,13 | 26,93 | 75 |
| 173H23 | 579ZMPM0_216F; 579ZMPM0_216R | 185; 186 | 82,3 | 25,76 | 80 |
| | 579ZMPM0_95F; 579ZMPM0_95R | 187; 188 | 79,5 | 24,97 | 73 |
| 118N19 | 579ZMPM0_99F; 579ZMPM0_99R | 189; 190 | 76,84 | 24,69 | 80 |
| | 579ZMPM0_244F; 579ZMPM0_244R | 191; 192 | 80,07 | 25,38 | 80 |
| 42L23 | 579ZMPM0_241F; 579ZMPM0_241R | 193; 194 | 81,16 | 25,79 | 79 |
| | 579ZMPM0_109F; 579ZMPM0_109R | 195; 196 | 77,89 | 25,28 | 74 |
| 112N13 | 579ZMPM0_109F; 579ZMPM0_109R | 197; 198 | 77,89 | 25,28 | 74 |
| | 579ZMPM0_247F; 579ZMPM0_247R | 199; 200 | 80,76 | 24,82 | 71 |
| 97K23 | 579ZMPM0_112F; 579ZMPM0_112R | 201; 202 | 79,22 | 25,2 | 77 |
| | 579ZMPM0_125F; 579ZMPM0_125R | 203; 204 | 83,44 | 28,17 | 74 |
| 18J17 | 579ZMPM0_253F; 579ZMPM0_253R | 205; 206 | 77,5 | 32,34 | 71 |
| | 579ZMPM0_125F; 579ZMPM0_125R | 207; 208 | 83,44 | 28,17 | 74 |
| 5M22 | 579ZMPM0_128F; 579ZMPM0_128R | 209; 210 | 77,99 | 24,05 | 77 |
| | 579ZMPM0_136F; 579ZMPM0_136R | 211; 212 | 78,65 | 26,46 | 78 |
| 146I6 | 579ZMPM0_131F; 579ZMPM0_131R | 213; 214 | 76,58 | 26,54 | 78 |
| | 579ZMPM0_137F; 579ZMPM0_137R | 215; 216 | 83,7 | 25,42 | 73 |
| 147015 | 579ZMPM0_138F; 579ZMPM0_138R | 217; 218 | 79,38 | 24,8 | 79 |
| | 579ZMPM0_147F; 579ZMPM0_147R | 219; 220 | 79,63 | 26,77 | 80 |
| 88K17 | 579ZMPM0_145F; 579ZMPM0_145R | 221; 222 | 81,51 | 27,61 | 76 |
| | 579ZMPM0_262F; 579ZMPM0_262R | 223; 224 | 75,7 | 25,82 | 80 |
| 180G22 | 579ZMPM0_161F; 579ZMPM0_161R | 225; 226 | 80,21 | 25,16 | 73 |
| | 579ZMPM0_265F; 579ZMPM0_265R | 227; 228 | 79,3 | 24,7 | 79 |

**Tabelle 8: Sequenzgehalt der 13 analysierten BAC-Klone**

| BAC | # Reads | # Reads ohne *E.coli* | Menge Sequenz in bp | Menge Sequenz in bp ohne *E.coli* |
|---|---|---|---|---|
| 144N24 | 10967 | 10849 | 3646226 | 3591222 |
| 119F13 | 17987 | 17847 | 6033910 | 5957456 |
| 219G11 | 32904 | 32484 | 10553629 | 10381924 |
| 86N21 | 39606 | 39106 | 12991596 | 12750077 |
| 16B06 | 36198 | 35849 | 12523123 | 12357036 |
| 84L18 | 50537 | 34162 | 15991645 | 10776458 |
| 128D02 | 15998 | 15847 | 5138442 | 5064677 |
| 25M23 | 22551 | 22416 | 7864493 | 7786402 |
| 96H10 | 7723 | 7614 | 2569604 | 2525488 |
| 19J24 | 21953 | 21775 | 7327364 | 7234315 |
| 136A01 | 31998 | 31724 | 10298869 | 10158900 |
| 75H06 | 24345 | 24121 | 8021727 | 7920125 |
| 135F07 | 29702 | 29484 | 9721708 | 9604010 |

### B) BAC Sequenz Assembly, -Annotation und Kandidaten-Gen-Selektion:

Erstellung eines Sequenz Scaffolds: Die BAC Klone 144N24, 119F13, 219G11, 86N21, 16B06, 84L18, 128D02, 25M23, 19J24, 96H10, 136A01, 75H06, 137F07 wurden mittels der 454-Technik sequenziert (Margulies et al., 2005).

Das automatische Assembly der Roh-Sequenzen der BAC Klone wurde mit der Software "Newbler" (454 runAssembly Software, Software Release 2.3) durchgeführt. Die so erzeugten Sequenz-Contigs pro BAC wurden in manueller Analyse korrekt angeordnet, wobei folgende Techniken angewandt wurden:
1. Sequenzen von überlappenden BACs konnten grob in überlappende und nicht überlappende Bereiche eingeteilt werden.
2. Sequenz-Contigs von verschiedenen überlappenden BACs wurden in den überlappenden Bereichen verglichen. Etwa 20% der Sequenz-Contigs konnten so angeordnet werden und Lücken zwischen ihnen geschlossen werden (z.B. wenn ein Contig des einen BACs zwei Contigs des anderen BACs abdeckt oder verbindet).
3. Alle Sequenz-Contigs wurden manuell annotiert. Hier wurden zunächst nur Repetitive Elemente (Transposons und Retrotransposons, kurz "TEs") annotiert. Da Sequenzlücken vor allem in TEs auftreten, kann die TE Annotation helfen, Sequenz-Contigs korrekt anzuordnen. Das heißt, wenn das eine Ende eines TEs auf einem Sequenz-Contig liegt und das andere Ende auf einem anderen, können die zwei Contigs entsprechend angeordnet werden. In solchen Fällen wurde jeweils eine Sequenz von 100 Ns eingefügt, um die Lücke zwischen den Sequenz-Contigs zu füllen. Auch die Information von TEs, die verschachtelt sind (d.h. TEs, welche sich in andere TEs inseriert hatten) wurde verwendet, um Sequenz-Contigs anzuordnen.
4. In manchen Bereichen konnten weder Informationen von überlappenden BACs noch TE-Annotationen verwendet werden (dies war v.a. der Fall in Bereichen, die nur von einem BAC abgedeckt wurden). Dort wurden die Sequenz-Contigs willkürlich angeordnet und die Lücken zwischen ihnen mit Sequenzen von 200 Ns gefüllt.
5. Viele der TEs im Maisgenom sind "Long Terminal Repeat" (LTR) Retrotransposons, die von langen (1-2 kb) LTR-Sequenzen flankiert werden. Diese LTRs können bis 100% identisch sein. In einigen Fällen wurden daher Roh-Sequenzen der beiden LTRs in eine Konsensus-Sequenz assembliert (d.h. eine Kopie der LTR is nicht im Assembly vorhanden. In diesen Fällen wurden die Sequenz-Lücken mit der Anzahl N's gefüllt die der Länge des zweiten LTR entsprechen würde.
6. Gene wurden manuell annotiert. Hierzu wurden die kodierenden Sequenzen (CDS) des publizierten B73 Maisgenoms als Referenz benutzt (http://www.maizegdb.org/gene_model.php). Die CDS wurden via DotPlot (http://www.dotplot.org/) mit der RP4HTN-Sequenz aliniert und so die Positionen von Exons und Introns bestimmt. Kandidatengene wurden einerseits durch die Beschreibung Ihrer Funktion (falls öffentlich bekannt) bestimmt. Andererseits wurden die CDS der resistenten RP4HTN mit der den anfälligen B73 AGPv02 verglichen. Falls Unterschiede vorhanden waren, wurde das jeweilige Gen in die Liste der Kandidaten aufgenommen. Die fertige Sequenz hat eine Länge von 1'328'253 bp. Die Liste der Kandidaten-Gene ist in Tabelle 1 wiedergegeben.

### 5. Molekulare Analyse der Kandidaten-Gene:

Expressionanalyse: Die Expression der verschiedenen Kandidatengene wurde auf 21 Tage alten (nach Aussaat), nicht infizierten Pflanzen (Infektionstag = 0 dpi) als auch auf 36 Tage alten mit *H. turcicum* infizierten und nicht infizierten (15 Tage nach der Infektion =15 dpi inf/ni) Pflanzen getestet.

RNA vom zweiten Blatt wurde aus den getesteten Maispflanzen extrahiert, in cDNA revers transkribiert und die Expression mittels qPCR gemessen. Jeweils das zweite Blatt wurde geerntet, eingefroren und die RNA mit dem SV Total RNA Isolation System Kit (Z3100; Promega, Dübendorf, Schweiz), gleich wie beschrieben (Brunner et al., 2011; Risk et al., 2012) extrahiert, quantifiziert und auf die Qualität und Reinheit hin geprüft.

1 µg RNA wurde mit dem iScript RT Supermix (170-8841; Bio-Rad, Cressier, Schweiz) in einem Reaktionsvolumen von 20 µl gemäss Herstellerangaben revers transkribiert. Um eine mögliche Kontamination durch genomische DNA auszuschliessen (RT minus), wurde parallel von jeder Probe eine Reaktion ohne die Zugabe der reversen Transkriptase mit inkubiert.

Quantitative Real Time PCR (RT-qPCR) wurde in technischen Triplikaten oder Duplikaten in einem Reaktionsvolumen von 10 µl und Zugabe von 4 µl 1:10 verdünnter (10mM Tris HCL pH8, 0,1 mM EDTA) cDNA 5 ul SsoFast EvaGreen^{®} Supermix (172-5201; Bio-Rad, Schweiz) und einer Primerkonzentration von 400 nM auf dem C1000 Touch Cycler (Bio-Rad, Schweiz) durchgeführt. Für die Amplifikation wurde folgendes Programm verwendet: 95° C für 30 Sekunden, gefolgt von 40 Zyklen 95°C für 3 Sekunden, dann 60-63 °C (gem Tabelle 2) für 5 Sekunden. Zur Analyse der Schmelzkurve (ausschliessen von Primer Dimeren) wurde das PCR Produkt in 0,5 °C Schritten von 65 °C auf 95 °C aufgeheizt. Amplifikationskurven und Schmelzkurven wurden in Programm CFX Manager V 3.0 (Bio-Rad, Schweiz) überprüft und die Cq-Werte (quantification cycle) in das Programm qbasePLUS V 2.3 (Biogazelle, Zwijnaarde, Belgien) zur Bestimmung der Relativen Expression exportiert.

Primer für die Kandidatengene wurden mithilfe von Primer-BLAST (http://www.ncbi.nlm.nih.gov/tools/primer-blast/) bestimmt, um unspezifische Amplifikation auf bereits bekannten Transkripten möglichst auszuschliessen. Zur Evaluation geeigneter Amplikons wurden die PCR Produkte mittels Agarose Gel Elektrophorese aufgetrennt und auf eine einzelne Bande und ihre Grösse hin überprüft. Weiter wurden Amplikons gem. Tabelle 1 von RP1HtN als auch NILHtN sequenziert. Zur Normalisierung der Expressionsdaten wurden 1-3 Referenzgene (LUG, MEP, FPGS) verwendet (Manoli et al., 2012).

Alle Kandidaten-Gene sind im anfälligen Genotyp RP1 und im resistenten Genotyp RP1HTN exprimiert. Eine differentielle Expression zwischen RP1 und RP1HTN konnte für RLK1 gezeigt werden. RLK1 ist in den anfälligen Pflanzen bis zu 4 mal stärker exprimiert als in den resistenten Pflanzen.

**Tabelle 9: Primerpaare für die Kandidaten-Gene mit ihrer Amplicon-Länge in bp und der geeigneten Annealing Temperatur.**

| Gen Name | Primer Name | SEQ ID NO: | Primer Sequenzen (F = Forward Sequenz; R = Reverse Sequenz) | Länge (in bp) in RP1 | Länge (in bp) in RP1HTN | annealing Temperatur |
|---|---|---|---|---|---|---|
| ZNF1 | GH034 | 229 | F: TGGTTGGTGTCGAAGCTGAG | 130 | 130 | 60 °C |
| | GH033 | 230 | R: ATTTATCCCGGCCTTTGCAT | | | |
| HYD | GH039 | 231 | F: | 74 | 74 | 60 °C |
| | | | GATCTACAGGGAAGCCCACT GA | | | |
| | GH040 | 232 | R: | | | |
| | | | TTTTTCCTTGAGGCAGTTATA TGCT | | | |
| RLK4 | GH220 | 233 | F: TTGTGCAGCGGAGGGAA | 91 | 85 | 63 °C |
| | GH221 | 234 | R: CCAGGGCACCAGCAAGAAT | | | |
| EXT1 | GH168 | 235 | F: CGACTACAAGACGCGTACC | 103 | 103 | 60 °C |
| | GH170 | 236 | R: GGTGTCGATGGTGAGGTTC | | | |
| RLK1 | GH138 | 237 | F: TATTGTTGGTGCTGTTGCCG | 121 | 121 | 60 °C |
| | GH139 | 238 | R: GGACTCAATCCTTGTCCCTG | | | |
| RET1 | GH055 | 239 | F: CGCTCGTTTGCCAGATAGCC | 165 | 165 | 60 °C |
| | GH056 | 240 | R: CACGGTGTGTGCCAGTTTGT | | | |

Tilling-Population Sichtung und Detektion von Mutanten: Für die Kandidaten-Gene (Tabelle 1) kann eine Tilling-Population von 10000 Pflanzen gesichtet werden, die die Introgression aus Pepitilla auf Chromosom 8 im Bereich von 151688552 - 153139596 bp im Vergleich zur B73-Referenz AGPv02 (www.maizesequence.org) trägt (RP3HTN1) und eine Resistenz gegenüber *Helminthosporium turcicum* aufweist. Die Mutationen können entweder stille Nukleotid-Austausche, Aminosäure-Austausche oder Stop-Codons sein und dienen zum Nachweis der Funktion des Kandidaten-Gens.

Transformation: Kandidaten-Gene können beispielsweise in den anfälligen Genotyp A188 durch Agrobacterium tumefanciens-vermittelte Transformation eingebracht werden. Dieser Genotyp zeichnet sich durch AUDPC-Werten von 702 im GWH-Test (n=18 Pflanzen) aus, so dass eine Transformation mit dem Resistenz-Gen eine deutliche Resistenzantwort ergibt.

### 6. Bestimmung der Rassenspezifität; Nachweis, dass HTN1 auch rassenunspezifisch Resistenz vermittelt

Das Screening der Genotypen mit HtN Gen erfolgt an vielen Standorten in allen Befallsregionen Europas. Bisher wurde diese Resistenz noch nicht gebrochen, sodass wir davon ausgehen, dass sie bisher rassenunspezifisch wirkt, solange, bis eine Rasse N gefunden wird. Vorherrschend in Europa ist die Rasse 1, wobei auch in einzelnen Regionen schon die Rassen 2 oder 3 oder eine Kombination dieser nachgewiesen werden konnte (Hanekamp et al., 2013).

### 7. Phänotypischer Test von weiteren rekombinanten Pflanzen

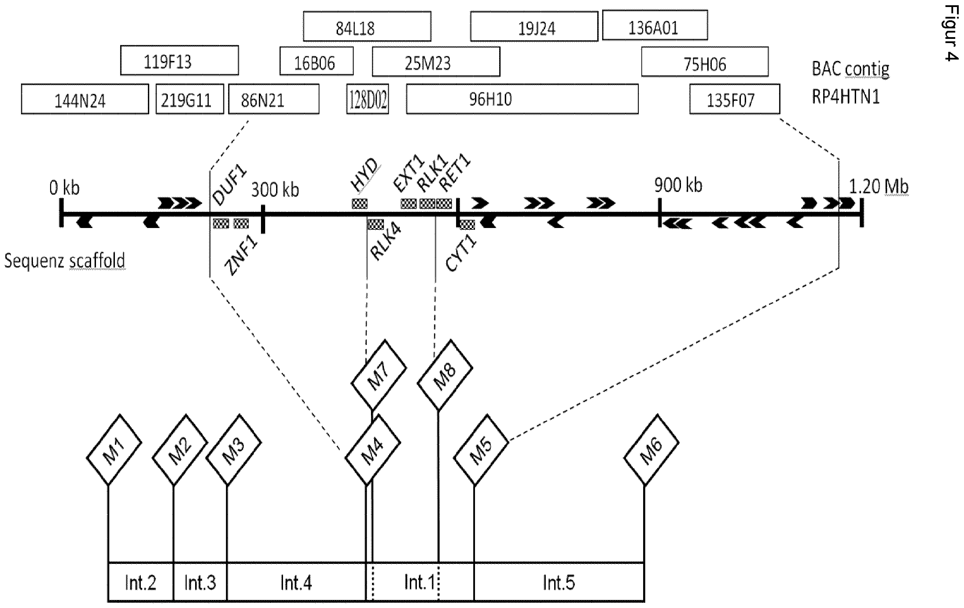

Es wurden neue rekombinante Pflanzen für den QTL-Bereich selektiert und mit den phänotypischen Daten korreliert. Die Selektion umfasste Pflanzen, die unterschiedliche Bereiche der Targetregion abdeckten. Es konnten rekombinante Pflanzen identifiziert werden, die zwischen den Markern MA0005 und MA0021 - Markerregion M7 und den Markern MA0013 und MA0022 - Markerregion M8 eine Introgression des Donors B37HTN1 im genetischen Hintergrund von RP1 aufweisen. In der Figur 4 ist dargestellt, dass dieser Bereich nur noch die drei Gene RLK4, EXT1 und RLK1 umfasst. Diese rekombinanten Pflanzen, die den Bereich M7 - M8 umfassen, zeigen sowohl im Feld unter künstlicher Inokulation, als auch im Gewächshaus-Test den resistenten Phänotyp.

### 8. Identifikation des resistenz-vermittelnden Kandidaten-Gen

Zur Identifikation des resistenz-vermittelnden Gens wurde die Tilling-Population von 10000 Pflanzen gesichtet, die die Introgression aus Pepitilla auf Chromosom 8 im Bereich von 151688552 - 153139596 bp im Vergleich zur B73-Referenz AGPv02 (http://www.genome.arizona.edu) trägt (RP3HTN1) und eine Resistenz gegenüber *Helminthosporium turcicum* aufweist.

Für die Gene RLK4 und RLK1 wurden Amplicons entwickelt (Tabelle 10) und diese nach Amplifikation der Einzelpflanzen-DNA der Tilling-Population mittels Sanger-Sequenzierung sequenziert.

**Tabelle 10: Primersequenzen der Amplicons**

| Gen Name | Primer Name | SEQ ID NO: | Primer Sequenzen (F = Forward Sequenz; R = Reverse Sequenz) | Länge des Amplicons (in bp) | Annealing Temperatur (°C) |
|---|---|---|---|---|---|
| RLK4 | MA04916-6f | 247 | F: | 399 | 60 |
| | | | TGTTTCAG GAATCACG CAACTGGA | | |
| | MA04916-6r | 248 | R: | | |
| | | | GCACCACG CCATGACC AACATC | | |
| RLK1 | TG10013-10.f | 249 | F: | 804 | 60 |
| | | | CTTCCTAC AGAAGAAC GAGAGT | | |
| | TG10013-11.r | 250 | R: | | |
| | | | TTCCTCAC GAGCTCTG TGGTC | | |

Die Amplicon-Sequenzen wurden mit der Software DNASTAR Lasergene ausgewertet und Basen-Mutationen identifiziert. In der Tabelle 11 ist eine Auswahl von gefundenen Mutationen aufgelistet.

**Tabelle 11: Identifizierte Mutationen für die Gene RLK4 und RLK1**

| Gen Name | Mutation Name | Position der Mutation in cDNA des RP3HTN1 (bp) | Basenaustausch | Position der Mutation in Proteinsequenz des RP3HTN1 (bp) | Aminosäure-Wechsel Effekt |
|---|---|---|---|---|---|
| RLK4 | RLK4d | 977 in SEQ ID NO: 3 | G > A | 326 in SEQ ID NO: 4 | G > D |
| | RLK4f | 1169 in SEQ ID NO: 3 | C > T | 390 in SEQ ID NO: 4 | T > I |
| RLK1 | RLK1b | 1365 in SEQ ID NO: 1 | G > A | 455 in SEQ ID NO: 2 | M > I |
| | RLK1d | 1490 in SEQ ID NO: 1 | G > A | 497 in SEQ ID NO: 2 | G > E |

Die identifizierten Mutanten wurden im Gewächshaus geselbstet und Saatgut der homozygoten Pflanzen mit Wildtyp-Allel und Mutations-Allel pro Mutationsereignis für einen phänotypischen Test geerntet.

Jeweils 15 homozygote Einzelpflanzen mit Wildtyp-Allel und Mutations-Allel der Mutanten RLK1b, RLK1d, RLK4d und RLK4f und die Kontrollen RP1 und RP1HTN1 wurden wie oben beschrieben im Gewächshaus mit *H. turcicum* inokuliert. Im Zeitraum von 11 bis 25 Tage nach Inokulation wurde der Befall an jedem Tag bestimmt. Die AUDPC-Werte aller getesteten Pflanzen sind in Tabelle 12 zusammengefasst. Durch die Änderung der Aminosäure im resistenten Elter der Tilling-Population RP3HTN1 wird eine Anfälligkeit der homozygoten Mutanten erwartet.

**Tabelle 12: AUDPC-Werte der homozygoten Pflanzen mit Wildtyp-Allel und Mutations-Allel der Gene RLK1 und RLK4. In der Spalte Phänotyp bedeutet 0 - 100 resistent, 101 - 450 heterozygot, und > 450 anfällig.**

| Mutanten Name | Allele | AUDPC | Phänotyp |
|---|---|---|---|
| RLK4d | Hom. Mutante | 33,3 | resistent |
| | Hom. Wildtyp | 0,0 | resistent |
| RLK4f | Hom. Mutante | 46,7 | resistent |
| | Hom. Wildtyp | 96,7 | resistent |
| RLK1b | Hom. Mutante | 346,7 | heterozygot |
| | Hom. Wildtyp | 46,4 | resistent |
| RLK1d | Hom. Mutante | 406,7 | heterozygot |
| | Hom. Wildtyp | 83,3 | resistent |
| RP1 | | 1030,0 | anfällig |
| RP1HTN1 | | 0,0 | resistent |

### Referenzen

Bar-Zur, A, Tadmor, Y, Juvik, JA, Shimoni, M, & Reuveni, R (1998). "Resistance to northern leaf blight in maize (Zea mays) conditioned by the HtN gene and the association with isoperoxidases." Canadian Journal of Plant Pathology 20(1): 28-34.
Brunner, S., Hurni, S., Herren, G., Kalinina, O., von Burg, S., Zeller, S.L., Schmid, B., Winzeler, M. and Keller, B. (2011) Transgenic Pm3b wheat lines show resistance to powdery mildew in the field. Plant Biotechnology Journal, no-no.
Chevalier, BS, Kortemme, T, Chadsey, MS, Baker, D, Monnat, RJ, Stoddard, BL (2002). "Design, activity, and structure of a highly specific artificial endonuclease". Mol. Cell 10 (4): 895-905. doi:10.1016/S1097-2765(02)00690-1.
Carson, ML (1995) "A new gene in maize conferring the chlorotic halo reaction to infection by Exserohilum turcicum. Plant Disease 79: 717-720.
Chung, C-L, Poland, J, Wisser, R, Kolkman, J, und Nelson, R (2008). "Analysis of qEt8.06, a Major QTL for Resistance to Northern Leaf Blight in Maize" Poster, Annual Research Meeting of Generation Challenge Programme, Bangkok, Thailand. http://www.plantpath.cornell.edu/labs/nelson_r/Docs/01_CLC_08GCP_12Sep08_2.pdf
Chung, C-L, Jamann, T, Longfellow, J und Nelson, R (2010). "Characterization and finemapping of a resistance locus for northern leaf blight in maize bin 8.06" Theoretical and Applied Genetics 121(2): 205-227.
Coates, ST und White DG (1998). "Inheritance of resistance to gray leaf spot in crosses involving selected resistant inbred lines of corn." Phytopathology 88(9): 972-982.
Depicker A, Stachel S, Dhaese P, Zambryski P, Goodman HM (1982) Nopaline synthase: transcript mapping and DNA sequence. J Mol Appl Genet. 1(6): 561-73.
Ferguson, LM und Carson ML (2007). "Temporal Variation in Setosphaeria turcica Between 1974 and 1994 and Origin of Races 1, 23, and 23N in the United States." Phytopathology 97: 1501-1511.
Gevers, HO (1975). "A new major gene for resistance to Helminthosporium turcicum leaf blight of maize." Plant Dis Rep 59: 296-300.
Gaj, T, Gersbach, CA und Barbas III, CF (2013). ZFN, TALEN, and CRISPR/Cas-based methods for genome engineering. Trends in biotechnology.
Ganal MW, Durstewitz G, Polley A, Bérard A, Buckler ES, Charcosset A, Clarke JD, Graner E-M, et al. (2011) "A large maize (Zea mays L.) SNP genotyping array: Development and germplasm genotyping, and genetic mapping to compare with the B73 reference genome." PLoS ONE6 e28334.
Gianasi, L, de Castro, HA und da Silva, HP (1996). "Raçes fisiológocas de Exserohilum turcicum identificadas em regiöes produtoras de milho no Brasil, Safra 93/94." Summa Phytopathol. 22: 214-217.
Griffiths, AJF, Miller, JH, Suzuki, DT, Lewontin, RC und Gelbart, WM (2000) An Introduction to Genetic Analysis, 7th edition, New York: W. H. Freeman; ISBN-10: 0-7167-3520-2.
Gupta, PK and Varshney, R (2013) Cereal Genomics II. Springer Science+Business Media Dordrecht, Niederlande; DOI: 10.1007/978-94-007-6401-9_1.
Hanekamp H., Kessel B., Koopmann B., von Thiedemann A.: Turcicum Blattdürre an Mais: Rassenbestimmung und regionales Auftreten von Exserohilum turcicum in Europa; 28.01.2013, PG Krankheiten an Getreide, Vortrag; Blattdürre.
Jordan, EG, Perkins, JM, Schall, RA und Pedersen, WL (1983). "Occurrence of race 2 of Exserohilum turcicum on corn in the central and eastern United States." Plant Disease 67: 1163-1165.
Li, T., Huang, S., Jiang, W. Z., Wright, D., Spalding, M. H., Weeks, D. P., & Yang, B. (2011). TAL nucleases (TALNs): hybrid proteins composed of TAL effectors and Fokl DNA-cleavage domain. Nucleic acids research, 39(1), 359-372.
Lipps, PE, und Hite, RE (1982). "Exserohilum turcicum virulent on corn with the Ht resistance gene in Ohio." Plant Disease 66: 397-398.
Lipps, PE, Pratt, RA und Hakiza, JJ (1997). "Interaction of Ht and partial resistance to Exserohilum turcicum in maize." Plant Disease 81: 277-282.
Maniatis, T, Fritsch, EF, Sambrook, J (1982) Molecular cloning. Cold Spring Harbor Laboratory, Cold Spring Harbor, New York.
Manoli A., Sturaro A., Trevisan S., Quaggiotti S., Nonis A. (2012) Evaluation of candidate reference genes for qPCR in maize, Journal of Plant Physiology, Volume 169, Issue 8, 15 May 2012, Pages 807-815.
Margulies, M, Egholm, M, Altman, WE, Attiya, S, Bader, JS, Bemben, LA et al. und Volkmer, GA (2005) "Genome sequencing in microfabricated high-density picolitre reactors." Nature, 437(7057), 376-380.
Moghaddam, FP und Pataky, JK (1994). "Reactions for isolates from mating of races 1 and 23N of Exserohilum turcicum." Plant Disease 78: 767-771.
Min, J, Chunyu, Z, Khalid, H, Nan, L., Quan, S, Qing, M, Suwen , W und Feng, L (2012). "Pyramiding resistance genes to Northern Leaf Blight and Head Smut in maize." Int. J. Agric. Biol. 14(3): 430-434.
Odell JT, Nagy F, Chua N-H (1985) Identification of DNA sequences required for activity of the cauliflower mosaic virus 35S promoter. Nature 313, 810-812.
Pataky, JK, Raid, RN, du Toit, LJ und Schueneman, TJ (1998). "Disease severity and yield of sweet corn hybrids with resistance to Northern Leaf Blight." Plant Disease 82(1): 57-63.
Perkins, JM und Pedersen, WL (1987). "Disease development and yield losses associated with northern leaf blight on corn." Plant Disease 71: 940-943.
Pratt, R and Gordon, S (2006). "Breeding for resistance to maize foliar pathogens." Plant Breed Rev. 27: 119-173.
Puchta, H and Hohn, B (2010). Breaking news: Plants mutate right on target. Proceedings of the National Academy of Sciences, 107(26), 11657-11658.
Raymundo, AD und Hooker, AL (1981a). "Measuring the relationship between northern corn leaf blight and yield losses." Plant Disease 65: 325-327.
Raymundo, AD, Hooker, AL und Perkins, JM (1981b). "Effect of Gene HtN on the Development of Northern Corn Leaf Blight Epidemics." Plant Disease 65(4): 327-329.
Risk, J.M., Selter, L.L., Krattinger, S.G., Viccars, L.A., Richardson, T.M., Buesing, G., Herren, G., Lagudah, E.S. and Keller, B. (2012) Functional variability of the Lr34 durable resistance gene in transgenic wheat. Plant Biotechnology Journal, 10, 477-487.
Rozen, S und Skaletsky, HJ (2000) Primer3 on the WWW for general users and for biologist programmers. In: Krawetz S, Misener S (eds) Bioinformatics Methods and Protocols: Methods in Molecular Biology. Humana Press, Totowa, NJ, pp 365-386.
Rushton, PJ, Torres, JT, Parniske, M, Wernert, P, Hahlbrock, K und Somssich, IE (1996) Interaction of elicitor-induced DNA-binding proteins with elicitor response elements in the promoters of parsley PR1 genes. EMBO J. 15(20): 5690-5700.
Sambrook J, Russell DW (2001) Molecular Cloning. A laboratory manual, Cold Spring Harbor Laboratory Press, 3. Aufl. 2001.Sanger, F und Coulson, AR. (1975) J. molec. Biol. 94, 414-418.
Silva, G, Poirot, L, Galetto, R, Smith, J, Montoya, G, Duchateau, P, & Päques, F (2011) Meganucleases and other tools for targeted genome engineering: perspectives and challenges for gene therapy. Current gene therapy, 17(1), 11.
Simcox, KD, und Bennetzen, JL (1993). "The use of molecular markers to study Setospaeria turcica resistance in maize." Phytopathology 83: 1326-1330.
Shimoni, M, Bar-Zur, A, & Reuveni, R (1991). The association of peroxidase activity and resistance of maize to Exserohilum turcicum. Journal of phytopathology, 131(4), 315-321.
Stanković, S, Lević, J, & Ivanović, D (2007). Genetic variability of maize pathogens in Serbia. *Genetika,* 39(2), 227-240.
Thakur, RP, Leonard, KJ und Leath, S (1989). "Effects of temperature and light on virulence of Exserohilum turcicum on corn." Phytopathology 1989, 79: 631-635.
Tzfira, T, Weinthal, D, Marton, I, Zeevi, V, Zuker, A, & Vainstein, A (2012). Genome modifications in plant cells by custom-made restriction enzymes. Plant biotechnology journal, 10(4), 373-389.
Ullstrup, AJ und Miles, SR (1957). "The effects of some leaf blights on corn grain yield." Phytopathology 47:331-336.
Walsh et al. (2008), Posterpräsentation P192, 50th Maize Genetics Conference in Washington D.C.
Welz, HG (1998). "Genetics and epidemiology of the pathosystem Zea mays / Setosphaeria turcica." Habilitationsschrift Institut für Pflanzenzüchtung, Saatgutforschung und Populationsgenetik, Universität Hohenheim*.*
Welz, HG und Geiger, HH (2000). "Genes for resistance to northern corn leaf blight in diverse maize populations." Plant Breeding 119: 1-14.
WO/2000/29592 (Max-Planck-Gesellschaft zur Förderung der Wissenschaften e.V.). Chimeric promoters capable of mediating gene expression in plants upon pathogen infection and uses thereof.
WO/2007/147395 (KWS SAAT AG). Pathogen induzierbarer synthetischer Promotor.
WO/2010/079430 (Bonas et al.) Modular DNA-binding domains and methods of use.
WO/2011/072246 (Regents of the University of Minnesota) TAL effector-mediated DNA modification.
WO/2011/163590 (Du Pont) Compositions and methods for enhancing resistance to Northern Leaf Blight in maize.

## Patentansprüche

1. Verfahren zur Identifizierung einer *Helminthosporium turcicum* resistenten Maispflanze, in deren Genom ein Chromosomfragment aus dem Donor Pepitilla integriert ist, umfassend den Nachweis von mindestens zwei Allelen im Genom der Pflanze, wobei mindestens ein Allel in einem genomischen Abschnitt lokalisiert ist, welcher durch einen Marker in der ersten Markerregion M1, die durch die Marker SYN14136 und PZE-108076510 flankiert ist, der zweiten Markerregion M2, die durch die Marker SYN24931 und PZE-108077560 flankiert ist, der dritten Markerregion M3, welche durch die Marker PZE-108093423 und PZE-108093748 flankiert ist, oder der vierten Markerregion M4, welche durch die Marker MA0004 und MA0005 flankiert ist, und durch das Polynukleotid, das in der Maispflanze Resistenz gegenüber *Helminthosporium turcicum* vermittelt, flankiert ist, und wobei mindestens ein Allel in einem genomischen Abschnitt lokalisiert ist, welcher durch das Polynukleotid und durch einen Marker in der sechsten Markerregion M6, welche durch die Marker PZE-108107671 und SYN4196 flankiert ist, oder der fünften Markerregion M5, welche durch die Marker MA0006 und PZE-108097482 flankiert ist, flankiert ist.

2. Verfahren zur Erhöhung des Ertrags einer *H. turcicum* resistenten Maispflanze, in deren Genom ein Chromosomfragment aus dem Donor Pepitilla integriert ist, wobei das Chromosomfragment ein Intervall des Denars umfasst, welches Donor-Allele gemäß dem Haplotyp nach Tabelle 2 aufzeigt und ein Polynukleotid aufweist, das in der Maispflanze Resistenz gegenüber *Helminthosporium turcicum* vermittelt, umfassend den Schritt der Entfernung
a) eines Intervalls des Donars zwischen einem Marker in einer ersten Markerregion, welche durch die Marker SYN14136 und PZE-108076510 flankiert ist, und einem Marker in einer zweiten Markerregion, welche durch die Marker SYN24931 und PZE- 108077560 flankiert ist, und/oder
b) eines Intervalls des Donors zwischen einem Marker in einer dritten Markerregion, welche durch die Marker PZE-108093423 und PZE-108093748 flankiert ist, und einem Marker in einer vierten Markerregion, welche.durch die Marker MA0004 und MA0005 flankiert ist, und/oder
c) eines Intervalls des Donors zwischen einem Marker in einer fünften Markerregion, welche durch die Marker MA0006 und PZE-108097482 flankiert ist, und einem Marker in einer sechsten Markerregion, welche durch die Marker PZE-108107671 und SYN4196 flankiert ist.

3. isoliertes Oligonukleotid, das ein Nukleinsäuremolekül mit einer Nukleotidsequenz, ausgewählt aus einer der SEQ ID NOs: 41-49, 53-100 oder 229-250, umfasst.

4. Verwendung eines isolierten Oligonukleotids, das ein Nukleinsäuremolekül mit einer Nukleotidsequenz, ausgewählt aus einer der SEQ ID NOs: 17-250, umfasst, zur Identifizierung einer *Helminthosporium turcicum-resistenten* Maispflanze.

5. isoliertes Polynukleotid, das ein Nukleinsäuremolekül umfasst,
(a) das eine Nukleotidsequenz gemäß SEQ ID NO: 1, 3, 5, 7, 9, 11, 13 oder 15 aufweist,
(b) eine Nukleotidsequenz mit einer Identität von mindestens 90% zu einer der Nukleotidsequenzen gemäß SEQ ID NO: 1, 3, 5, 7, 9, 11, 13 oder 15 vorzugsweise über die gesamte Sequenzlänge, aufweist,
(c) das mit dem komplementären Strang eines Nukleinsäuremoleküls nach (a) oder (b) unter stringenten Bedingungen hybridisiert,
(d) das ein Polypeptid mit einer Aminosäuresequenz gemäß SEQ ID NO: 2, 4, 6, 8, 10, 12, 14 oder 16 kodiert, oder
(e) das ein Polypeptid mit einer Aminosäuresequenz, die zu mindestens 90% identisch ist mit einer der Aminosäuresequenzen nach (d), kodiert.

6. Vektor oder mobiles genetisches Element, umfassend ein isoliertes Polynukleotid gemäß Anspruch 5.

7. Vektor gemäß Anspruch 6, wobei der Vektor eine oder mehrere operativ verknüpfte regulatorische Sequenzen, welche die Transkription und optional die Expression in einer prokaryotischen oder eukaryotischen Wirtszelle erlaubt, aufweist.

8. Transgene Pflanzenzelle, umfassend ein isoliertes Polynukleotid gemäß Anspruch 5 als Transgen oder einen Vektor oder mobiles genetisches Element nach Anspruch 6.

9. Transgene Pflanze, umfassend ein isoliertes Polynukleotid gemäß Anspruch 5 als Transgen oder einen Vektor oder mobiles genetisches Element nach Anspruch 6 oder eine Pflanzenzelle nach Anspruch 8.

10. Korn oder Samen von der Pflanze nach Anspruch 9, wobei das Korn oder der Samen ein isoliertes Polynukleotid gemäß Anspruch 5 als Transgen umfasst.
